(19) Europäisches Patentamt
European Patent Office
Office européen des brevets

(11) EP 1 842 926 B1

(12) **EUROPEAN PATENT SPECIFICATION**

(45) Date of publication and mention of the grant of the patent:
**19.08.2015 Bulletin 2015/34**

(51) Int Cl.:
***C12Q 1/68*** *(2006.01)*

(21) Application number: **07090040.2**

(22) Date of filing: **08.03.2007**

# (54) A method of identifying a biological sample for methylation analysis

Verfahren zur Bestimmung einer biologischen Probe zur Methylisierungsanalyse

Procédé d'identification d'un échantillon biologique pour analyse de méthylation

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **10.03.2006 EP 06090032**
**30.05.2006 EP 06090091**

(43) Date of publication of application:
**10.10.2007 Bulletin 2007/41**

(73) Proprietor: **Epigenomics AG**
**10829 Berlin (DE)**

(72) Inventors:
- **Berlin, Kurt, Dr.**
  **14532 Stahnsdorf (DE)**
- **Dietrich, Dimo**
  **10437 Berlin (DE)**
- **Kluth, Antje, Dr.**
  **21465 Wentdorf (DE)**
- **Schatz, Philipp, Dr.**
  **10435 Berlin (DE)**
- **Wandell, Michael, Dr.**
  **10437 Berlin (DE)**
- **Tetzner, Reimo**
  **10439 Berlin (DE)**

(74) Representative: **Zwicker, Jörk**
**ZSP Patentanwälte**
**Partnerschaftsgesellschaft**
**Radlkoferstrasse 2**
**81373 München (DE)**

(56) References cited:

| | |
|---|---|
| **EP-A- 1 647 600** | **EP-A1- 0 799 897** |
| **WO-A-96/19586** | **WO-A-99/43855** |
| **WO-A-02/052030** | **WO-A-03/054551** |
| **WO-A-2004/083819** | **US-A- 6 153 389** |

- **BIBIKOVA MARINA ET AL: "High-throughput DNA methylation profiling using universal bead arrays" GENOME RESEARCH, vol. 16, no. 3, March 2006 (2006-03), pages 383-393, XP002445638 ISSN: 1088-9051 -& BIBIKOVA MARINA ET AL: "High-throughput DNA methylation profiling using universal bead arrays, Supplementary Fig.1" GENOME RESEARCH, [Online] vol. 16, no. 3, March 2006 (2006-03), pages 383-393, XP002445639 ISSN: 1088-9051 Retrieved from the Internet: URL:http: //www.genome.org/cgi/data/gr.4410 706/DC1/1> [retrieved on 2007-08-03]**
- **KARIMI ET AL: "LUMA (LUminometric Methylation Assay)-A high throughput method to the analysis of genomic DNA methylation" EXPERIMENTAL CELL RESEARCH, SAN DIEGO, CA, US, vol. 312, no. 11, 1 July 2006 (2006-07-01), pages 1989-1995, XP005485748 ISSN: 0014-4827**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

FIELD OF THE INVENTION

The invention relates generally to novel and substantially improved methods of identifying a biological sample for methylation analysis comprising an identifier, in particular their application, use and detection.

BACKGROUND OF ASPECTS OF THE INVENTION

*Methylation analysis.* Many diseases, in particular cancer diseases, are accompanied by a modified gene expression. This may be a mutation of the genes themselves, which leads to an expression of modified proteins or to an inhibition or over-expression of the proteins or enzymes. A modulation of the expression may however also occur by epigenetic modifications, in particular DNA methylation. Such epigenetic modifications do not affect the actual DNA coding sequence. It has been found that DNA methylation processes have substantial implications for the health, and it seems to be clear that knowledge about methylation processes and modifications of the methyl metabolism and DNA methylation are essential for understanding diseases, for the prophylaxis, diagnosis and therapy of diseases.

The precise control of genes, which represent a small part only of the complete genome of mammals, is a question of the regulation under consideration of the fact that the main part of the DNA in the genome is not coding. The presence of such trunk DNA containing introns, repetitive elements and potentially actively transposable elements, requires effective mechanisms for their durable suppression (silencing). Apparently, the methylation of cytosine by S-adenosylmethionine (SAM) dependent DNA methyltransferases, which form 5-methylcytosine, represents such a mechanism for the modification of DNA-protein interactions. Genes can be transcribed by methylation-free promoters, even when adjacent transcribed or not-transcribed regions are widely methylated. This permits the use and regulation of promoters of functional genes, whereas the trunk DNA including the transposable elements is suppressed. Methylation also takes place for the long-term suppression of X-linked genes and may lead to either a reduction or an increase of the degree of transcription, depending on where the methylation in the transcription unit occurs.

Nearly the complete natural DNA methylation in mammals is restricted to cytosine-guanosine (CpG) dinucleotide palindrome sequences, which are controlled by DNA methyl transferases. CpG dinucleotides are about 1 to 2% of all dinucleotides and are concentrated in so-called CpG islands. A generally accepted definition of CpG islands means that a 200 bp long DNA region has a CpG content of at least 50%, and that the ratio of the number of observed CG dinucleotides and the number of the expected CG dinucleotides Is larger than 0.6 (Gardiner-Garden, M., Frommer, M. (1987) J. Mol. Biol. 196, 261-282. Typically, CpG islands have at least 4 CG dinucleotides in a sequence having a length of 100 base pairs.

If CpG islands are present in promoter areas, they have often a regulatory function for the expression of the respective gene. If the CpG island is hypomethylated, expression can take place. Hypermethylation often leads to the suppression of the expression. In the normal state, a tumor suppressor gene is hypomethylated. If a hypermethylation takes place, this will lead to a suppression of the expression of the tumor suppressor gene, which is frequently observed in cancer tissues. In contrast thereto, oncogenes are hypermethylated In healthy tissue, whereas in cancer tissue they are frequently hypomethylated.

By the methylation of cytosine, regularly the binding of proteins regulating the transcription is prevented. This leads to a modification of the gene expression. With regard to cancer, for instance the expression of cell division regulating genes is affected thereby, i.e. for instance the expression of apoptosis genes is regulated down, whereas the expression of oncogenes is regulated up. The hypermethylation of the DNA has however also a long-term influence on the regulation. By the methylation of cytosine, histone de-acetylation proteins can bind by their 5-methylcytosine-specific domain to the DNA. This has as a consequence that histones are de-acetylated, which will lead to a tighter compacting of the DNA. Thereby, regulatory proteins do not have the possibility anymore to bind to the DNA.

For the reason of this, the detection of DNA methylation is important with respect to diagnosing a disease, prognosing a disease, predicting a treatment response, diagnosing a predisposition for a disease, diagnosing a progression of a disease, grading a disease, staging a disease, classifying a disease, characterizing a disease, or for identifying a new marker associated with a disease. An overview of method for DNA methylation analysis can be gathered from Laird PW. "The power and the promise of DNA methylation markers" Nat Rev Cancer 2003 Apr;3 (4):253-66. Many methods for methylation analysis are based on treatment of genomic DNA with reagent that differentiates between methylated and unmethylated cytosines. In many cases this reagent is a bisulfite reagent which leads to a conversion of unmethylated cytosines to uracil or after amplification to thymin while methylated cytosines remain unchanged.

*Pronounced need in the art.* Like many modern laboratory workflows methods for methylation analysis are characterized in that a large number of samples has to be processed. Thereby it is irrelevant, if the methods or workflows are applied for diagnosing a disease, prognosing a disease, predicting a treatment response, diagnosing a predisposition for a disease, diagnosing a progression of a disease, grading a disease, staging a disease, classifying a disease,

characterizing a disease, or for identifying a new marker like methylation, RNA, or protein which is associated with a disease. In any case it is import that samples are not interchanged and no sample is contaminated with another sample. As relevant prior art the following is considered:

According to WO994385 endogenous polymorphic sequences are used as unique identifier for biological samples. These identifiers link the sample to its source and other relevant information.

According to US Patent 6,153,389 biological forensic or medical samples are marked by the addition of nucleic acids of known sequence. It further utilizes primers and their use for the detection of the added nucleic acid in an amplification reaction resulting in a nucleic acid molecule of specific length.

Currently the applicant is not aware of any prior art, which addresses the question of detecting sample interchange and/or sample crosscontamination for methylation analysis. The two above cited documents does not teach a method for labelling a biological sample which survives a bisulfite treatment as it is used many times in methylation analysis.

SUMMARY OF ASPECTS OF THE INVENTION

Aspects of the present disclosure relate to compositions and methods of identifying a biological sample in the field of methylation analysis comprising at least one identifier, in particular their application, use and detection.

Particular aspects provide compositions and methods of identifying a biological sample in the field of methylation analysis, wherein biological samples are provided, one or more identifiers are applied to one sample, the applied identifier(s) are detected or quantified, and the DNA methylation of each biological sample is analyzed.

Particular aspects provide compositions and methods of identifying a biological sample in the field of methylation analysis, wherein the DNA of the biological sample including the identifier is brought into contact with a reagent or enzyme which differentiates between methylated or unmethylated cytosine positions.

Particular aspects provide compositions and methods of identifying a biological sample in the field of methylation analysis, wherein the biological sample and the identifier are subjected to experimental procedures. For example, but not limited to, wherein the DNA of the sample Is isolated, bisulfite treated, purified, and desulfonated.

Particular aspects provide compositions and methods of identifying a biological sample in the field of methylation analysis, wherein the detection or quantification is realized simultaneously with the methylation analysis.

Particular aspects provide compositions and methods of identifying a biological sample in the field of methylation analysis, wherein the identifier is at least in parts a nucleic acid. In particular aspects the identifier comprises at least one cytosine, at least one guanin, or both; is a variant of a polymorphism; and/or has a similar base composition as the DNA section of interest. In particular aspects the identifier is part of the endogenous genomic DNA of the biological sample. In other particular aspects the identifier is part of a external DNA molecule which is added to the biological sample shortly after collecting the sample or at the beginning of an experimental procedure.

Particular aspects provide compositions and methods of identifying a biological sample In the field of methylation analysis, wherein the applied identifiers of different samples are assigned to different set of identifiers according to their biological, chemical, or physical properties. In particular aspects the identifiers are assigned to sets of sequence polymorphic variants, to sets of length polymorphic variants, to sets of single nucleotide polymorphic variants, or to sets of deletion polymorphic variants.

Particular aspects provide compositions and methods of identifying a biological sample in the field of methylation analysis, wherein the identifier is detected or quantified by nucleic acids or analogues thereof which comprise at least one cytosine and/or at least one guanin.

Particular aspects provide compositions and methods of identifying a biological sample in the field of methylation analysis, which can be used as a method of detection of sample interchange, crosscontamination, or both; as a method of identifying a sample in a pooled sample set; as a method of an amplification inhibition; as a method of normalization, calibration or both; as a method of identification of a carry-over contamination or both; as a method of determining the rate of DNA conversion.

Particular aspects provide compositions and methods for controlling the correctness of a process or method.

Particular aspects disclose also kits for realizing said particular aspects.

The invention itself is defined by the appended claims.

BRIEF DESCRIPTION OF THE FIGURES

Figure 1 shows an overview over one embodiment of the invention. According to this embodiment two identifiers are assigned to each other, whereby each identifier belongs to a different set of identifiers. One set contains 8 variants of a sequence polymorphism, and the other 8 variants of a length polymorphism. The primers for detection

are the same for every variant of a set.

Figure 2 shows exemplary an overview over a set of 24 samples. A unique combination of identifiers is applied to each sample. Each identifier is detected by means of hybridization of the correspondent amplification products. As can be easily seen, every identifier combination hence every sample has its own characteristic hybridization pattern.

Figure 3 shows at the top exemplary how a sample interchange of sample 2 with sample 17 is detected. Figure 3 shows in the lower part exemplary how a sample cross contamination of sample 2 with sample 17 is detected.

Figure 4 shows schematic drawings of a hybridization. In A the probe orientation at the array is shown. B and C show hybridizations of samples. In B two plasmids are used which were generated by domain-primer 1 + domain-primer 2 and domain-primer 1 + domain-primer 3. In C two plasmids are used which were generated by domain-primer 1 + domain-primer 2 and domain-primer 1 + domain-primer 4.

Figure 5 shows a schematic drawing of a hybridized microarray detecting a contamination of the sample. A combination of two plasmids was used.

Figure 6A shows an agarose gel analysis of amplificates of the linearized bisulfite treated plasmid 23. 100 pg of bisulfite treated linearized plasmid 23 were used for amplification. Figure 6B) shows an agarose gel analysis of amplificates of the linearized bisulfite treated plasmid 195. 100 pg of bisulfite treated linearized plasmid 23 were used for amplification. (m = size standard, bright band is about 200 bp)

Figure 7 shows the image of two hybridized array tubes. A) Amplificates derived from molecular identification plasmid 23 were used for hybridization. B) Amplificates derived from molecular Identification plasmid 195 were used for hybridization. The amplificates of each of the two molecular identification plasmids hybridizes specifically two oligonucleotides of the array tube (dark spots marked by quadrats). Dark spots at the corner of the image show control spots necessary for scanning the array tubes. Light grey spots represent unspecific hybridization.

Figure 8A shows a schematic overview of an identification pattern according to the invention. A first identifier is assigned to samples of the columns 1, 5, 9 of a microtiter plate. A second identifier is assigned to samples of the columns 2, 6, 10. A third identifier is assigned to samples of the column 3, 7, 11 (identifier X = light grey, identifier Y = middle grey, identifier Z = dark grey).

Figure 8B shows a schematic overview of the determined identifier pattern by analysis. Every detected identifier identity is assigned to the position of the sample from which an aliquot was used for determining the identifier identity. It is obvious that a sample interchange occurred in the first run (samples 5 and 6) as well as in the third run (samples 12 and 13).

## DETAILED DESCRIPTION OF ASPECTS OF THE INVENTION

For achieving various technical objects, aspects of the invention teach compositions and methods of identifying a biological sample for methylation analysis comprising identifier, in particular their application, use and detection. Said compositions and methods comprise providing at least one biological sample, applying at least one identifier, detecting or quantifying the applied identifier(s) and performing a methylation analysis.

Particular aspects provide methods comprising at least one identifier and at least one nucleic acid, which enables a detection or quantification of the at least one identifier even after the treatment with bisulfite, sulfide or any other correspondent DNA converting reagent. Particular aspects provide further methods for simultaneous detection or quantification of the applied at least one identifier and the detection or quantification of the methylation of the genomic DNA of the provided biological sample. Particular aspects provide suitable combinations and adjustments of these methods with each other in a manner that actually meets the technical object(s).

### Advantages of aspects of the invention.

In particular aspects, the exemplary inventive method has the advantage that it enables the use of identifiers in conjunction with bisulfite conversion of DNA. In particular, it therefore has the following advantages for methylation analysis:

- It enables a detection of sample interchange.

- It enables a detection of sample crosscontamination.
- It enables a detection of carry over contamination.
- It enables further a normalization and/or calibration of the sample or of the used methylation analysis method.
- It enables an identification of a sample in a pooled set of samples.
- It enables to calculate a conversion rate for bisulfite treatment.
- It enables a detection of inhibition of subsequent processes in particular PCR based analysis.
- It enables an assessment of the success of a hybridization step.

In particular aspects, the exemplary inventive method has the advantage that it controlls the error free and accurate run of a process or method, in particular of a high throughput method. Thereby said method can be a method for diagnosis, prognosis, or for marker discovery.

Method of aspects of the invention.

The method of the invention is a method of identifying at least one biological sample in the field of methylation analysis, comprising steps (a) and (b) in the indicated order:

(a) providing a sample set of at least two biological sample, wherein at least one sample comprises genomic DNA differentially methylated at least at one position;
(b) applying at least one identifier for each sample, wherein the applied at least one identifier does not interfere with subsequent analysis; and wherein the applied at least one identifier is a nucleic acid forming no stable secondary structure and comprises at least one cytosine-free, or cytosine-free and guanine-free oligonucleotide binding site; contacting the DNA of each sample with bisulfite;
(c) subjecting each sample to a detection or quantification reaction specific for the binding site(s) of the one or more applied identifiers; and
(d) subjecting each sample to methylation analysis, whereby methylation is detected or quantified

wherein step (c) is carried out prior, simultaneously with or subsequent to step (d).

In particular aspects, the method of the invention is a method of identifying at least one biological sample in the field of methylation analysis, wherein the identifier is part of the genomic DNA of the biological sample.

In particular aspects, the method of the invention is a method of identifying at least one biological sample in the field of methylation analysis, wherein the identifier is added to the biological sample.

According to an embodiment, the method of the invention is a method of identifying at least one biological sample in the field of methylation analysis, comprising the first two steps in the indicated order:

providing a sample set of at least two biological samples, wherein at least one sample comprises genomic DNA differentially methylated at least at one position;
applying at least one identifier for each sample, wherein the applied at least one identifier does not interfere with subsequent analysis;
subjecting each sample to a detection or quantification reaction specific for the one or more applied identifiers; and
subjecting each sample to methylation analysis.

In a preferred embodiment the identifier is a nucleic acid which is part of a sample endogenous DNA molecule, in particular a genomic DNA molecule. In another preferred embodiment, the identifier is a nucleic acid which is added to a sample. In a preferred embodiment, the identifier is detected or quantified before methylation analysis of the provided DNA. In another preferred embodiment, the identifier is detected or quantified subsequent to methylation analysis of the provided DNA. In a particular preferred embodiment, the detection or quantification of the identifier is carried out simultaneously with the methylation analysis.

A preferred embodiment further comprises at least one of the following

In a preferred embodiment, the identifier can be detected or quantified in between or subsequent to an experimental procedure. For example, but not limited to it, such an experimental procedure can, comprise a procedure for Isolating genomic DNA, a procedure for treating genomic DNA with a reagent which differentiates between methylated and unmethylated DNA, a procedure for purifying DNA, and/or a procedure for detecting or quantifying DNA methylation. Suitable experimental procedures are for example but not limited to described in WO 2006/113770 or in WO 2006/039563.

According to a preferred embodiment, the identifier is at least in parts part of a larger molecule; part of an endogenous molecule of the respective sample; part of an exogenous molecule added to the respective sample; a section of genomic DNA or total genomic DNA derived from a plant; a section of genomic DNA or total genomic DNA derived from a bacteria; a section of genomic DNA or total genomic DNA derived from a non-vertebrate; a section of

genomic DNA or total genomic DNA derived from a vertebrate; a short tandem repeat a variant of a deletion polymorphism; a variant of a single nucleotide polymorphism; a variant of a length polymorphism; an artificial nucleic acid; a circular nucleic acid; a circular DNA; a plasmid; a polynucleotide; an oligonucleotide; a PNA; a PNA-oligomer; a PNA-polymer; an artificial methylation; or combinations thereof.

According to a preferred embodiment, different identifiers are assigned to different sets of identifiers according to their respective biological, chemical, or physical properties.

A preferred embodiment is characterized in that a representative of each of at least two sets of identifiers is comprised in a plasmid. Such a plasmid can be for example, but not limited to, a plasmid as shown in Figure 1. A plasmid according to Figure 1 comprises a variant of a sequence polymorphism out of 8 variants and a variant of a length polymorphism out of 8 variants. Thereby each variant of the sequence polymorphism and each variant of the length polymorphism represents an identifier. Furthermore, all variants of the sequence polymorphism represent a set of identifiers and all variants of the length polymorphism represent second set of identifiers. This combination of different sets of identifiers has the advantage that different samples can be identified according to different methods. A simple identification of corresponding samples is for example possible by detection of the different variants of length polymorphism by means of PCR and gel electrophoresis. For some applications, it might be favourable to identify samples according to the sequence polymorphic variants. For example, but not limited to, in case the methylation analysis of the DNA of biological sample comprises PCR and detection of the PCR products by hybridization. In this case it is easily possible also to perform an amplification of the different variants of the sequence polymorphism and detect them also by hybridization. Preferably the methylation analysis and the detection of the Identifier are performed simultaneously. If desired it is also possible to quantify the identifier. This allows for example, but not limited to, drawbacks onto the amount of crosscontamination of the original sample.

According to a preferred embodiment, the first set of identifiers comprises a sequence polymorphism and wherein the second set of identifiers comprises a length polymorphism.

According to a preferred embodiment the identifier
comprises at least one oligonucleotide binding site covering cytosine positions converted by bisulfite;
is characterized by a similar base composition as the analyzed genomic DNA of the provided sample;
is a polymorphic sequence of about 5, about 10, about 15, about 20, about 25, about 30, about 35, about 40, about 50, about 75, about 100, or about 200 nucleotides;
has a content of cytosin-nucleotides and guanosin-nucleotides of about 15%, about 20%, about 30%, about 40%, about 50%, about 60%, about 70%, or about 80%; or combinations thereof.

According to a preferred embodiment the identifier
is characterized by a similar base composition as the analyzed genomic DNA of the provided sample;
is a polymorphic sequence of about 5, about 10, about 15, about 20, about 25, about 30, about 35, about 40, about 50, about 75, about 100, or about 200 nucleotides;
has a content of cytosin-nucleotides and guanosin-nucleotides of about 15%, about 20%, about 30%, about 40%, about 50%, about 60%, about 70%, or about 80%; or combinations thereof.

According to a preferred embodiment the identifier is a variant of a sequence polymorphism and additionally comprises about 1, about 10, about 20, about 30, about 40, about 50, about 60, about 70, about 80, about 90, or about 100 variable nucleotide sites.

According to a preferred embodiment the identifier is a variant of a sequence polymorphism and additionally comprises about 5, about 10, about 15, about 20, or about 25 variable nucleotide sites;
has a content of cytosin-nucleotides and guanosin-nucleotides of about 20%, about 30%, about 40%, about 50%, about 60%, about 70%, or about 80%; or combinations thereof.

According to a preferred embodiment the identifier is a variant of a length polymorphism and additionally
has a length difference of about 10, about 100, about 200, about 300, about 400, about 500, about 600, about 700, about 800, about 900, or about 1000 nucleotides compared to other used nucleic acid length polymorphic identifiers;
is of about 10, about 100, about 500, about 1.000, about 1.500, about 2.000, about 2.500, about 3.000, about 3.500, about 4.000, about 4.500, about 5.000, about 5.500, about 6.000, about 6.500, about 7.000, about 7.500, about 8.000, about 8.500, about 9.000, about 9.500, or about 10.000 nucleotides in length; is derived from non-human DNA; or combinations thereof.

According to a preferred embodiment the identifier is a variant of a length polymorphism and additionally
is either of about 5, about,25, about 50, about 75, about 100, about 125, about 150, about 175, about200, about225, about250, about275, about300,about325, about350, about375, about 400, about 425, about 450, about 475, or about 500 nucleotides in length;
is derived from non-human DNA;
has a content of cytosin-nucleotides and guanosin-nucleotides of about 20%, about 30%, about 40%, about 50%, about 60%, about 70%, or about 80%; or combinations thereof.

According to a preferred embodiment the identifier comprises a tag selected from the group comprising dye,

fluorescent dye, chemiluminescent dye, Cy5, Cy3, TAMRA, FAM, tag, epitope tag, peptide, polypeptide, protein, saccharide, harmon, lipid, mass label, particle, gold particle, silver particle, platin particle, paraffin embedded code or combinations thereof.

The identifier comprises a cytosine-free region or cytosine-free and guanin-free region for conversion-specific detection. A person skilled in the art knows numerous suitable detection methods for example but not limited to real time based PCR methods comprising the use of conversion-specific primers, probes and/or blockers.

In a preferred embodiment, the identifier is embedded into paraffin as a paraffin embedded code. Preferably, the identifier is embedded into paraffin simultaneously with the biological sample or after the embedment of the biological sample. Preferably, an unembedded identifier is added to embedded biological sample

The reagent that differentiates between a methylated or an unmethylated position is a bisulfite reagent, wherein the methylated or unmethylated position is a cytosine position, or both.

In a preferred embodiment, the experimental procedure comprises one or more of the following sample pooling, DNA isolation, DNA pooling, DNA concentration, DNA purification, desulfonation, amplification.

In a preferred embodiment these steps are essentially carried out as described in WO 2006/113770 or in WO 2006/039563.

According to a preferred embodiment, a bisulfite treatment is essentially carried out as described in WO05/038051. According to this, in one embodiment DNA is reacted with a bisulfite reagent, characterized in that said reaction is carried out in the presence of a compound out of the group of dioxane, one of its derivatives and a similar aliphatic cyclic ether.

In an embodiment DNA is reacted with a bisulfite reagent, characterized in that said reaction is carried out in the presence of a compound of the following formula:

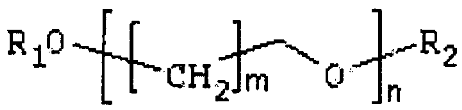

n = 1-35000
m = 1-3
R1 = H, Me, Et, Pr, Bu
R2 = H, Me, Et, Pr, Bu

Preferred are thus n-alkylene glycol compounds, particularly their dialkyl ethers, and especially diethylene glycol dimethyl ether (DME).

The bisulfite conversion may take place both in solution as well as also on DNA bound to a solid phase. Preferably sodium disulfite (= sodium bisulfite/sodium metabisulfite) is used, since it is more soluble in water than sodium sulfite. The disulfite salt disproportionates in aqueous solution to the hydrogen sulfite anions necessary for the cytosine conversion. When bisulfite concentration is discussed below, this refers to the concentration of hydrogen sulfite and sulfite anions in the reaction solution. For the method according to the invention, concentration ranges of 0.1 to 6 mol/l are possible. Particularly preferred is a concentration range of 1 to 6 mol/l, and most particularly preferred, 2-4 mol/l. However, when dioxane is used, the maximal concentration of bisulfite that can be used is smaller (see below). In selecting the bisulfite concentration, one must consider that a high concentration of bisulfite leads to a high conversion, but also leads to a high decomposition rate due to the lower pH.

Dioxane can be utilized in different concentrations. Preferably, the dioxane concentration amounts to 10 to 35% (vol/vol), particularly preferred is 20 to 30%, and most particularly preferred is 22 to 28%, especially 25%. A dioxane concentration higher than 35% is problematic, since this results in a formation of two phases within the reaction solution. In the particularly preferred embodiments with a dioxane concentration of 22-28%, the final preferred bisulfite concentration amounts to 3.3 to 3.6 mol/l, and in the most particularly preferred embodiment with a dioxane concentration of 25%, it amounts to 3.5 mol/l (see Examples).

The n-alkylene glycol compounds according to the invention can be utilized In a different concentration range. DME Is preferably used in concentrations between 1-35% (vol/vol). There is preferably between 5 and 25%, and most preferably 10% DME.

The preferred scavengers utilized according to the invention are chromane derivatives, e.g., 6-hydroxy-2,5,7,8,-tetramethylchromane 2-carboxylic acid (also known as: Trolox-C™) or trihydroxybenzoe acid and derivates thereof, e.g. Gallic acid (see: WO 2005/038051). Further scavengers are listed in the patent application WO 01/98528 (= DE 100 29 915; = US application 10/311,661).

The bisulfite conversion can be conducted in a wide temperature range from 0 to 95 °C. However, as at higher temperatures the rates of both the conversion and decomposition of the DNA increase, in a preferred embodiment the

reaction temperature lies between 0-80°C, preferably between 30-80°C. Particularly preferred is a range between 50-70 °C; most particularly preferred between 57-65°C.

The optimal reaction time of the bisulfite treatment depends on the reaction temperature. The reaction time normally amounts to between 1 and 18 hours (see: Grunau et al. 2001, Nucleic Acids Res. 2001, 29(13), E65-5.

The reaction time is ordinarily 4-6 hours for a reaction temperature of 60°C.

In a particularly preferred embodiment of the method according to the invention, the bisulfite conversion is conducted at mild reaction temperatures, wherein the reaction temperature is then clearly increased for a short time at least once during the course of the conversion. In this way, the effectiveness of the bisulfite conversion can be surprisingly clearly increased. The temperature increases of short duration are named "thermospikes" below. The "standard" reaction temperature outside the thermospikes is denoted as the basic reaction temperature. The basic reaction temperature amounts to between 0 and 80°C, preferably between 30-80°C, more preferably between 50-70 °C, most preferably between 57-65°C , as described above.

The reaction temperature during a thermospike is increased to over 85 °C by at least one thermospike. The optimal number of thermospikes is a function of the basic reaction temperature. The higher the optimal number of thermospikes is, the lower is the basic reaction temperature. At least one thermospike is necessary in each case. And, on the other hand, in principle, any number of thermospikes is conceivable. Of course, it must be considered that with a large number of temperature Increases, the decomposition rate of the DNA also increases, and an optimal conversion is no longer assured. The preferred number of thermospikes is thus between 1 and 10 thermospikes each time, depending on the basic reaction temperature. A number of two to 5 thermospikes is thus particularly preferred. The thermospikes increase the reaction temperature preferably to 85 to 100°C, particularly preferably to 90-100°C, and most preferably to 94°C-100°C.

The duration in time of the thermospikes also depends on the volume of the reaction batch. It must be assured that the temperature is increased uniformly throughout the total reaction solution. For a 20 µl reaction batch when using a thermocycler a duration between 15 seconds and 1.5 minutes, especially a duration between 20 and 50 seconds is preferred. In a particular preferred embodiment the duration is 30 seconds. Operating on a volume of 100 µl the preferred range lies between 30 seconds and 5 minutes, especially between 1 and 3 minutes. Particularly preferred are 1.5-3 minutes. For a volume of 600 µl, a duration of 1 to 6 minutes, is preferred, especially between 2 and 4 minutes. Particularly preferred is a duration of 3 minutes. A person skilled in the art will easily be able to determine suitable durations of thermospikes in relation to a variety of reaction volumes. The above-described use of thermospikes leads to a significantly better conversion rates in the bisulfite conversion reaction, even when the above-described denaturing solvents are not utilized.

According to a preferred embodiment, the method of the invention is a method, wherein bisulfite treated DNA is subjected directly to methods in the field of methylation analysis. This is especially preferred in view of the avoidance of cross-contaminations in PCR based methods. This embodiment is basically carried out as described in US 2006/115835. According to this, decontaminated DNA is provided which is suitable for DNA methylation analysis. This embodiment is characterized in that DNA is incubated with a bisulfite reagent comprising solution as described above. This leads to a sulfonation, a deamination, or both of unmethylated cytosine. Deamination is a spontaneous process in an aqueous solution and leads to sulfonated uracil comprising DNA. No desulfonation occurs yet.

In a separate step, the DNA comprising sulfonated uracil is brought into con,tact and incubated with an enzyme which specifically degrades non-sulfonated uracil containing nucleic acids. Such an enzyme is for example Uracil-DNA-Glycosylase (UNG).

In a preferred embodiment for providing a decontaminated template DNA for polymerase based amplification reactions, the sulfonated and/or deaminated template DNA are mixed with an UNG activity and components required for a polymerase mediated amplification reaction or an amplification based detection assay. After degradation of non-sulfonated uracil containing nucleic acids by use of UNG, the UNG activity is terminated and the template DNA is desulfonated by increased temperature. Subsequently the template DNA is ready to be amplified.

In a preferred embodiment, degradation, termination, desulfonation and amplification occur in a single tube during a polymerase based amplification reaction and/or an amplification based assay. Preferably such an amplification is performed in the presence of dUTP instead of dTTP.

In a preferred embodiment, sulfonated and partially or completely deaminated DNA after bisulfite treatment is subjected directly to a polymerase based amplification reaction and/or an amplification based assay without any prior desulfonation. The desulfonation occurs during the initial temperature increase of the amplification reaction.

These particular embodiments have the advantage in comparison to known methods of bisulfite treatment that the purification step after bisulfite treatment becomes dispensable. This is a simplification which results in reduction of costs and handling effort, minimizes loss of bisulfite treated DNA and is also time saving.

In an embodiment, the method of the invention is a method, wherein treating DNA with a bisulfite reagent allowing differentiation of the methylation status comprises purifying the treated DNA.

According to an embodiment, the treatment that leads to a conversion of unmethylated cytosine to uracil while

methylated cytosines remain unchanged comprises the purification of the bisulfite treated DNA. According to an embodiment, such a purification comprises a desulfonation of the bisulfite treated DNA by bringing the said into contact with an alkaline reagent or solution for example but not limited to a alkaline solution of about 0.1 mol/l sodium hydroxide.

In a preferred embodiment, purifying the treated DNA comprises the use of at least one selected from the group comprising: ultrafiltration, Microcon filter device, filter device, ethanol, propanol, silica surface, silica membrane, magnetic particle, polystyrol particle, positively charged surface, and positively charged membrane, charged membrane, charged surface, charged switch membrane, charged switched surface.

In a particular preferred embodiment the detection or quantification reaction comprises the use of at least one of the following methods or combinations thereof: amplification method, PCR method, isothermal amplification method, NASBA method, LCR method, methylation specific amplification method, MSP (Methylation Specific PCR) method, nested MSP method, HeavyMethyl™ method, detection method, agarose gel, staining of an agarose gel, methylation specific detection method, bisulfite sequencing method, detection by means of DNA-arrays, detection by means of oligonucleotide microarrays, detection by means of CpG-island-microarrays, detection by means of restriction enzymes, simultaneous methylation specific amplification and detection method, COBRA method, real-time PCR, HeavyMethyl™ real time PCR method, MSP MethyLight™ method, MethyLight™ method, MethyLight™ Algo™ method, QM method, Headloop MethyLight™ method, HeavyMethyl™ MethyLight™ method, HeavyMethyl™ Scorpion™ method, MSP Scorpion™ method, Headloop Scorpion™ method, methylation sensitive primer extension, and Ms-SNuPE (Methylation-sensitive Single Nucleotide Primer Extension) method.

According to an embodiment, the amplification method can be any kind of amplification method. A person skilled in the art is in knowledge of suitable amplification methods. According to a preferred embodiment, the amplification method is a PCR method. A person skilled in the art knows suitable PCR methods which can be used according to the invention. According to a preferred embodiment, the amplification method is an isothermal amplification. Suitable amplification methods for use according to the invention are well known in the art. Such a method can be for example but not limited to it the Primer Extension method. According to a preferred embodiment, the amplification method is a NASBA method. NASBA methods are RNA-DNA based amplification methods which comprise the use of a Reverse Transcriptase, a RNA polymerase and a RNase. A person skilled in the art is aware of NASBA methods which can be used according to the invention. According to a preferred embodiment, the amplification method is a Ligase Chain Reaction method. In general, these are amplification methods which are based on the use of a ligase. A person skilled in the art knows suitable LCR which can be used according to the invention.

According to an embodiment, the amplification method is a methylation specific amplification. Suitable methylation specific amplification methods are known to those skilled in the art. According to a preferred embodiment, the methylation specific amplification method is the Methylation Specific PCR (MSP) method. The MSP method allows the assessing of the methylation status of virtually any group of CpG sites within a CpG island, independent of the use of methylation-sensitive restriction enzymes (Herman et al. Proc. Natl. Acad. Sci. USA 93:9821-9826, 1996; US Patent No. 5,786,146. Briefly, DNA is modified by sodium bisulfite converting all unmethylated, but not methylated cytosines to uracil, and subsequently amplified with primers specific for methylated versus unmethylated DNA. MSP primer pairs contain at least one primer, which hybridizes to a bisulfite treated CpG dinucleotide. Therefore, the sequence of said primers comprises at least one CpG dinucleotide. MSP primers specific for non-methylated DNA contain a "T" at the 3' position of the C position in the CpG. Preferably, therefore, the base sequence of said primers is required to comprise a sequence having a length of at least 9 nucleotides which hybridizes to the bisulfite converted nucleic acid sequence, wherein the base sequence of said oligomers comprises at least one CpG dinucleotide. MSP requires only small quantities of DNA and is sensitive to 0.1% methylated alleles of a given CpG island locus. Bisulfite treatments and amplification method described herein may be used in combination with this detection method.

According to a preferred embodiment, the amplification is a nested MSP method. The nested MSP method is essentially carried out as described in WO 02/18649 and US 20040038245. This MSP method considers the apparent conflict of requiring high specificity of the MSP primer to sufficiently differentiate between CG and TG positions and of allowing a mismatch in order to create a unique restriction site.

It comprises the expanding of copy numbers of the genetic region of interest. Therefore a polymerase chain reaction is used to amplify a portion of said region wherein the methylation of interest resides. Thereby an amplification product is generated. An aliquot of said product is then used in a second, methyladon-specific, polymerase chain reaction to detect the presence of methylation. In other words a non-methylation specific PCR is performed prior to the methylation specific PCR.

According to a preferred embodiment, the amplification method is the HeavyMethyl™ method. The HeavyMethyl™ method is essentially carried out as described in WO 02/072880 and Cottrell SE et al. Nucleic Acids Res. 2004 Jan 13;32(1):e10. This method comprises the use of blocking probe oligonucleotides which may be hybridized to the bisulfite treated template nucleic acid concurrently with the PCR primers. Preferably, the blocking oligonucleotides are characterized in that their base sequence comprises a sequence having a length of at least 9 nucleotides which hybridizes to the chemically treated nucleic acid sequence. Thereby the base sequence of said blocker oligonucleotides comprises

at least one CpG, TpG or CpA dinucleotide. The amplification of the template nucleic acid is suppressed in case the complementary sequence of the blocking probe is present in the template. In such a case the amplification is terminated at the 5' position of the blocking probe. The blocking probe may be designed to hybridize to the bisulfite treated nucleic acid in a methylation status specific manner. For example, methylated nucleic acids within a population of unmethylated nucleic acids can be detected by suppressing the amplification of nucleic acids which are unmethylated at a position in question. Therefore a blocking probe would comprise a 'CpA' or 'TpA' at the position in question, as opposed to a 'CpG' if the suppression of amplification of methylated nucleic acids is desired. The use of blocker oligonucleotides requires for a efficient disruption of polymerase-mediated amplification that the blocker oligonucleotides cannot be elongated by the polymerase. According to the HeavyMethyl™ method, this is achieved through the use of blockers that are 3'-deoxyoligonucleotides, or oligonucleotides derivatized at the 3' position with other than a "free" hydroxyl group. For example, but not limited to it, 3'-O-acetyl oligonucleotides are representative of a preferred class of blocker molecules.

Additionally, polymerase-mediated degradation of the blocker oligonucleotides should be precluded. Preferably, such preclusion comprises either i) the use of a polymerase lacking 5'-3' exonuclease activity, or ii) the use of modified blocker oligonucleotides. These modified blocker oligonucleotides are characterized in having, for example, thiolate bridges at the 5'-termini. This renders the blocker molecule nuclease-resistant. Particular applications may not require such 5' modifications of the blocker oligonucleotide. For example, degradation of the blocker oligonucleotide will be substantially precluded if the blocker- and primer-binding sites overlap. Thereby the binding of the primer is precluded (e.g., in case of excess blocker oligonucleotide). Therefore the polymerase cannot bind on the primer and elongated it. Because no polymerase is extending the primer, the blocking oligonucleotide will not be degraded. A particularly preferred embodiment of the HeavyMethyl™ method, for purposes of the present invention and as implemented herein, comprises the use of peptide nucleic acid (PNA) oligomers as blocking oligonucleotides. Such PNA blocker oligomers are ideally suited because they are neither degraded nor extended by the polymerase.

According to an embodiment, the detection method can be any kind of detection method. A person skilled in the art is in knowledge of suitable detection methods. Preferably, a detection method can be any kind of detection method which comprises the use of a fluorescent dye, a non-fluorescent dye, a mass label, a separation by size, or a separation by weight. For example, but not limited to it, the detection method is a separation by size in an agarose gel followed by a staining of DNA by means of a fluorescent dye. According to a preferred embodiment, the detection method is a methylation specific detection. A person skilled in the art knows suitable methylation specific detection methods. According to a preferred embodiment, the methylation specific detection method Is a bisulfite sequencing method. The bisulfite sequencing method is essentially carried out as described in Frommer et al. Proc. Natl. Acad. Sci. USA 89:1827-1831, 1992. The bisulfite sequencing method is a method wherein the sequencing of a previously amplified fragment of the bisulfite treated genomic DNA is carried out. As the bisulfite treated DNA is amplified before sequencing, an amplification method as described herein may be used in combination with this detection method. !t is further especially preferred that the results of a bisulfite sequencing are essentially analyzed as described in EP 1369493. In brief, according to this method the degree of methylation of a cytosine is determined by means of an electropherogram of one or more bases. Thereby the area underneath the electropherogram of a detected base is calculated. The degree of methylation is then deduced by comparison this value for a cytosine position to be analyzed with the value obtained for an unmethylated cytosine. For better results, the determination and the consideration of the conversion rate of cytosine to uracil of the bisulfite treatment and/or a standardization of electropherogram signals is favorable.

According to a preferred embodiment, the detection method is a method of detection by means of a DNA-array. A person skilled in the art knows a lot of suitable DNA-arrays. Preferably, a DNA array comprises DNA molecules which are bound to or elsewise associated with a solid phase. The array can be characterized, for example but not limited to it, in that the DNA molecules are arranged on the solid phase in the form of a rectangular or hexagonal lattice. Thereby the solid phase is at least one phase selected from the group comprising: silicon, glass, polystyrene, aluminum, steel, iron, copper, nickel, silver, gold, nitrocellulose, or plastics such as but not limited to it nylon. But also combinations of the said materials are thinkable. For detection, the DNA hybridized on the array is labeled, preferably with a fluorescent dye. Such labelling is for example, but not limited to it, the simple attachment of Cy3 and Cy5 dyes to the 5'-OH of the DNA fragment. The detection of the fluorescence of the hybridized DNA may be carried out, for example, but not limited to it, via a confocal microscope.

According to a particular preferred embodiment, the detection method is a method of detection by means of an oligonucleotide microarray. An overview of the prior art in oligomer array manufacturing can be gathered from a special edition of Nature Genetics (Nature Genetics Supplement, Volume 21, January 1999, and from the literature cited therein.

According to a particular preferred embodiment, the detection method is a method of detection by means of a CpG-island-microarray. Thereby the immobilized or associated DNA of the array comprises sequences which were derived from CpG islands.

According to a particular preferred embodiment, the detection method is a method of detection by means of a DNA-array as essentially described in WO 99/28498, WO 01/38565, or in WO 02/18632.

According to a preferred embodiment, the detection method is a method of detection by means of restriction enzymes. A person skilled in the art is in knowledge of suitable methods.

According to a preferred embodiment, the methylation specific amplification and the detection are carried out simultaneously. Suitable methods are known to those skilled in the art. According to a particular preferred embodiment, the method for simultaneous methylation specific amplification and detection is the COBRA method. The COBRA method is a quantitative methylation method useful for determining DNA methylation levels at specific gene loci in small amounts of genomic DNA (Xiong & Laird, Nucleic Acids Res. 25:2532-2534, 1997; this reference is incorporated by reference to its entirety). According to the COBRA method, restriction enzyme digestion is used to reveal methylation-dependent sequence differences in PCR products of bisulfite-treated DNA. Methylation-dependent sequence differences are first introduced into the genomic DNA by bisulfite treatment. PCR amplification of the bisulfite converted DNA is then performed using methylation unspecific primers followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. Additionally, restriction enzyme digestion of PCR products amplified from bisulfite-converted DNA is also used, in the method described by Sadri & Homsby (Nucl. Acids Res. 24:5058-5059, 1996. Bisulfite treatments and amplification methods described herein may be used in combination with this detection method.

According to a particular preferred embodiment, the method for simultaneous methylation specific amplification and detection is a real-time PCR method. A person skilled in the art knows suitable real-time PCR methods. According to a particular preferred embodiment, the real-time PCR method is a HeavyMethyl™ method. The HeavyMethyl™ method is thereby performed as described above by means of a real-time PCR machine.

According to a particular preferred embodiment, the real-time PCR method is a MethyLight™ method. The MethyLight™ method is a high-throughput quantitative methylation method that utilizes fluorescence-based real-time PCR (TaqMan™) technology that requires no further manipulations after the PCR step (Eads et al., Cancer Res. 59:2302-2306, 1999). Briefly, the MethyLight™ process begins with a mixed sample of genomic DNA that is converted, in a bisulfite reaction, to a mixed pool of methylation-dependent sequence differences according to standard procedures. Fluorescence-based PCR is then performed either in an "unbiased" (with primers that do not overlap known CpG methylation sites) PCR reaction, or in a "biased" (with PCR primers that overlap known CpG dinucleotides) reaction. Sequence discrimination can occur either at the level of the amplification process or at the level of the fluorescence detection process, or both.

The MethyLight™ method may be used as a quantitative test for methylation patterns in the genomic DNA sample, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinudeotides. Alternatively, a qualitative test for genomic methylation is achieved by probing of the biased PCR pool with either control oligonucleotides that do not "cover" known methylation sites (a fluorescence-based version of the "MSP" technique also named MSP MethyLight™ method), or with oligonucleotides covering potential methylation sites.

The MethyLight™ process can be used with a "TaqMan®" probe in the amplification process. For example, double-stranded genomic DNA is treated with bisulfite and subjected to one of two sets of PCR reactions using TaqMan® probes; e.g., with either biased primers and Tasman® probe, or unbiased primers and TaqMan® probe. The TaqMan® probe is dual-labeled with fluorescent "reporter" and "quencher" molecules, and is designed to be specific for a relatively high GC content region so that it melts out at about 10°C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Variations on the TaqMan® detection technology that are also suitable include the use of dual-probe technology (LightCycler™), fluorescent amplification primers (Sunrise™ technology), Molecular Beacon Probes (Tyagi S., and Kramer F.R., Nature Biotechnology 14, 303-308, 1996), Scorpion primers (Whitcombe et al., Nature and Biotechnology, 17, 804-807, 1999), or LNA (Locked Nucleid Acid) Double-Dye Oligonucleotide probes (Exiqon A/S). All of these techniques may be adapted in a manner suitable for use with bisulfite treated DNA, and moreover in the field of methylation analysis within CpG dinudeotides.

Bisulfite treatments and amplification methods described herein may be used in combination with the MethyLight™ method or its variants.

According to a particular preferred embodiment, the real-time PCR method is the MethyLight™ ALGO™ method. The MethyLight™ ALGO™ method is an improved method of the MethyLight™ method as essentially described in EP04090255.3. According to this improved method, the degree of methylation is calculated from the signal intensities

of probes using different algorithms.

According to a particular preferred embodiment, the real-time PCR method is the QM (quantitative methylation) assay. This assay Is a methylation unspecific and therefore unbiased real-time PCR amplification. It is accompanied by the use of two methylation specific probes (MethyLight™) one for the methylated amplificate and a second for the unmethylated amplificate. In this way, two signals are generated which can be used a) to determine the ratio of methylated (CG) to unmethylatad (TG) nucleic acids, and at the same time b) to determine the absolute amount of methylated nucleic acids. For the later, a calibration of the assay is necessary with a known amount of control DNA.

According to preferred embodiment, the method for simultaneous methylation specific amplification and detection is a Headloop PCR method. The Headloop PCR method is a suppression PCR method. It essentially carried out as described in Rand K.N., et al., Nucleic Acid Research, 33(14), e127. It is a PCR method for distinguishing related sequences in which the selectivity of amplification is dependent from the amplicon's sequence. A 5' extension is included in one (or both) primer(s) that corresponds to sequences within one of the related amplicons. After copying and incorporation into the amplificate this sequence is then able to loop back, anneal to the internal sequences and prime to form a hairpin structure. This structure prevents then further amplification. Thus, amplification of sequences containing a perfect match to the 5' extension is suppressed while amplification of sequences containing mismatches or lacking the sequence is unaffected.

According to a particular preferred embodiment, the method for simultaneous methylation specific amplification and detection is a combination of the Headloop PCR method and the MethyLight™ method, also named Headloop MethyLight™ method.

According to preferred embodiment, the method for simultaneous methylation specific amplification and detection is a Scorpion™ method. This method was first described by Whitcombe et al.: Detection of PCR products using self-probing amplicons and fluorescence. Nat Biotechnol. 1999; 17(8):804-7; Thelwell et al.: Mode of action and application of Scorpion™ primers to mutation detection. Nucleic Acids Res. 2000 Oct 1; 28(19):3752-61; US 6,326,145; US 6,365,729; US 20030087240 A1. Several embodiments of this method are known to those skilled in the art. All of these methods have the intramolecular probing in common. According to the so-called Hairloop variant, Scorpion™ primers possess a specific probe sequence at their 5' end. This sequence is present in a hairloop like configuration. A fluorescent dye and a quencher are located in spatial proximity at the end of the probing sequence. After denaturation subsequent to an amplification cycle, the probe hybridizes intramolecularly onto the elongated primer sequence of the same strand. Thereby the hairloop is opened, the dye and the quencher are separated and thus the dye's signal can be detected.

Other Scorplon™ method variants are for example the Duplex variant (Solinas et al.: Duplex Scorpion™ primers in SNP analysis and FRET applications. Nucleic Acids Res. 2001 Oct 15; 29(20):E96), or the variants as described in US 6,326,145 and US 20030087240.

According to a particular preferred embodiment, the Scorpion™ method is a method as essentially described in WO 05/024056.

According to a particular preferred embodiment, the method for simultaneous methylation specific amplification and detection is a combination of the HeavyMethyl™ method and the Scorpion™ method, also named HeavyMethyl™ Scorpion™ method.

According to a particular preferred embodiment, the method for simultaneous methylation specific amplification and detection is a combination of the HeavyMethyl™ method and the MethyLight™ method, also named HeavyMethyl™ MethyLight™ method.

According to a particular preferred embodiment, the method for simultaneous methylation specific amplification and detection Is a combination of the MSP method and the Scorpion™ method, also named MSP Scorpion™ method.

According to a particular preferred embodiment, the method for simultaneous methylation specific amplification and detection is a combination of the Headloop method and the Scorpion™ method, also named Headloop Scorpion™ method.

According to a preferred embodiment, the method for simultaneous methylation specific amplification and detection is a method of methylation specific primer extension. A person skilled in the art knows several methods which can be used according to the invention.

According to a particular preferred embodiment, the method of methylation specific primer extension is the Ms-SNuPE (methylation-sensitive Single Nucleotide Primer Extension) method. The Ms-SNuPE method is a method as essentially carried out as described in Gonzalgo et al., Nucleic Acids Research 25(12), 2529-2531, 1997 and US Patent 6,251,594. According to the Ms-SNuPE method, regions of interest are amplified by PCR from bisulfite treated DNA. After purification of the PCR products, primers are proximately hybridized in front of the position to be analyzed. The primer is then elongated by a single nucleotide either with labeled dCTP or with differently labeled dTTP. In case the cytosine in the original DNA was methylated, then dCTP will be incorporated because methylated cytosines remain unchanged during bisulfite treatment. In the other case, the cytosine in the original DNA was unmethylated, then dTTP will be incorporated because unmethylated cytosine is converted to uracil by bisulfite treatment and subsequent PCR will substitute uracil by thymine. By detection of the different labels, it can be distinguished if a cytosine of a CpG position

was methylated or unmethylated. The MS-SNuPE method can also be performed in a quantitative manner.

According to a particular preferred embodiment, the method of methylation specific primer extension is a method as essentially described in WO 01/062960, WO 01/062064, or WO 01/62961. All of these methods can be performed in a quantitative manner. According to WO 01/062960, the primer to be extended hybridizes with its 3' terminus complete or only partially onto the positions of interest. An extension of at least one nucleotide occurs only if the primer hybridizes completely. WO 01/062064 discloses a method in which the primer to be extended hybridizes proximately adjacent or at a distance of up to ten bases to the position to be analyzed. The primer is then extended by at least a single nucleotide. The third method is described in WO 01/62961. According to this method, two sets of oligonucleotides are hybridized to the amplified DNA after bisulfite treatment. The first type of oligonucleotide hybridizes 5' proximately adjacent or at a distance of up to 10 bases to the position to be analyzed. The second type of oligonucleotide hybridizes on the amplified DNA so that its 5' terminus hybridizes 3' proximately adjacent to said position to be analyzed. Through this, the two oligonucleotides are separated from each other by a gap of in the range of 1 to 10 nucleotides. The first type of oligonucleotide is then extended by means of a polymerase, wherein not more than the number of nucleotides lying between the two oligonucleotides are added. Thereby nucleotides are used which comprise differentially labeled dCTP and/or dTTP. The two oligonucleotides are then linked to each other by means of a ligase enzyme. In case the cytosine in the original DNA was methylated, then dCTP will be incorporated. In case the cytosine in the original DNA was unmethylated, then dTTP will be incorporated.

Of course other similar methods, which are further developed methods of the named methods or combinations thereof are also useable according to the invention.

In a preferred embodiment the detection or quantification reaction comprises a nucleic acid, DNA, PNA, oligonucleotide, or PNA oligomer which
is at least of about 10, about 15, about 20, about 25, about 30, about 35, about 40, about 50, about 60, about 70, about 80, about 90, about 100, about 150 or about 200 nucleotides in length,
has a content of cytosin-nucleotides and guanosin-nucleotides of about 15%, about 20%, about 30%, about 40%, about 50%, about 60%, about 70%, about 80%, or about 85%;
has a melting temperature of about 37°C, about 45°C, about 50°C, about 55°C, about 60°C, about 65°C, about 70°C, about 75°C, about 80°C, about 85°C, about 90°C, about 95°C, or about 99°C; or
combinations thereof.

In a preferred embodiment the detection or quantification reaction comprises a oligonucleotide which
is at least of about 5, about 10, about 15, about 20, about 25, about 30, about 35, about 40, about 50, about 60, about 70, about 80, or about 90 nucleotides in length;
has a content of cytosin-nucleotides and guanosin-nucleotides of about 5%, of about 10%, about 20%, about 30%, about 40%, about 50%, about 60%, about 70%, about 80%, about 90% or about 95%;
has a melting temperature of about 37°C, about 45°C, about 50°C, about 55°C, about 60°C, about 65°C, about 70°C, about 75°C, about 80°C, about 85°C, about 90°C, about 95°C, or about 99°C; or
combinations thereof.

In a preferred embodiment the detection or quantification reaction comprises a oligonucleotide which
is at least of about 16, about 20, about 25, about 30, about 35, or about 40 nucleotides in length;
has a content of cytosin-nucleotides and guanosin-nuelcotides of about 20%, about 30%, about 40%, about 50%, about 60%, about 70%, or about 80%; and
has a melting temperature of about 50°C, about 53°C, about 56°C, about 59°C, or about 62°C.

In a preferred embodiment the detection or quantification reaction comprises an oligonucleotide which comprises a gene-specific priming sequence and a sequence which hybridizes on a variant of a sequence polymorphism.

In a preferred embodiment the detection or quantification reaction comprises an oligonucleotide which comprises two domains,
wherein one domain comprises a target-specific priming sequence of about 10, about 15, about 20, about 25, about 30, about 35, or about 40 nucleotides, has a content of cytosin-nucleotides and guanosin-nucleotides of about 20%, about 30%, about 40%, about 50%, about 60%, about 70%, or about 80%, and has a domain melting temperature of about 50°C, about 52°C, about 54°C, about 56°C, about 58°C, about 60°C, or about 62°; and herein the other domain comprises a unique sequence of about 5, about 10, about 15, about 20, about 25, about 30, about 35, about 40, about 45, or about 50 nucleotides, is free of cytosines, guanin, or both, and has a content of cytosin-nucleotides and guanosin-nucleotides of about 20%, about 30%, about 40%, about 50%, about 60%, about 70%, or about 80%.

The detection or quantification reaction may comprise an oligonucleotide which comprises a gene-specific priming sequence and a sequence which hybridizes on a variant of a sequence polymorphism. According to a preferred embodiment, the gene-specific priming sequence is located in the 5' terminal region and the sequence hybridizing onto the variant of the sequence polymorphism is located in the 3' terminal region of the said oligonucleotide. According to a particular preferred embodiment, the sequence hybridizing onto the variant of the sequence polymorphism is located in the 5' terminal region and gene-specific priming sequence is located in the 3' terminal region of the said oligonucleotide.

The detection or quantification reaction may comprise an oligonucleotide which comprises two domains, wherein one domain comprises a target-specific priming sequence of about 10, about 15, about 20, about 25, about 30, about 35, or about 40 nucleotides, has a content of cytosin-nucleotides and guanosine-nucleotides of about 20%, about 30%, about 40%, about 50%, about 60%, about 70%, or about 80%, and has a domain melting temperature of about 50°C, about 52°C, about 54°C, about 56°C, about 58°C, about 60°C, or about 62°C; and
wherein the other domain comprises a unique sequence of about 5, about 10, about 15, about 20, about 25, about 30, about 35, about 40, about 45, or about 50 nucleotides, and has a content of cytosine-nucleotides and guanosine-nucleotides of about 20%, about 30%, about 40%, about 50%, about 60%, about 70%, or about 80%.

According to the herein specified embodiments for detection or quantification, a said oligonucleotide is a DNA oligonucleotide or comprises at least in parts DNA analogues like PNA (peptide nucleic acid),

In a preferred embodiment, methylation analysis comprises at least one selected from the group comprising detection of methylation status, detection of methylation level, detection of methylation pattern, detection of methylation pattern level, amplification method, PCR method, isothermal amplification method, NASBA method, LCR method, methylation specific amplification method, MSP (Methylation Specific PCR) method, nested MSP method, HeavyMethyl™ method, detection method, methylation specific detection method, bisulfite sequencing method, detection by means of DNA-arrays, detection by means of oligonucleotide microarrays, detection by means of CpG-island-microarrays, detection by means of restriction enzymes, simultaneous methylation specific amplification and detection method, COBRA method, real-time PCR, HeavyMethyl™ real time PCR method, MSP MethyLight™ method, MethyLight™ method, MethyLight™ Algo™ method, QM method, Headloop MethyLight™ method, HeavyMethyl™ MethyLight™ method, HeavyMethyl™ Scorpion™ method, MSP Scorpion™ method, Headloop Scorpion™ method, methylation sensitive primer extension, and Ms-SNuPE (Methylation-sensitive Single Nucleotide Primer Extension) method.

According to an embodiment, the determining of a methylation status of at least one CpG position, determining of at least one methylation pattern, or both comprises the use of at least once of the following methods or combinations thereof: amplification method, PCR method, isothermal amplification method, NASBA method, LCR method, methylation specific amplification method, MSP (Methylation Specific PCR) method, nested MSP method, HeavyMethyl™ method, detection method, agarose gel, staining of an agarose gel, methylation specific detection method, bisulfite sequencing method, detection by means of DNA-arrays, detection by means of oligonucleotide microarrays, detection by means of CpG-island-microarrays, detection by means of restriction enzymes, simultaneous methylation specific amplification and detection method, COBRA method, real-time PCR, HeavyMethyl™ real time PCR method, MSP MethyLight™ method, MethyLight™ method, MethyLight™ Algo™ method, QM method, Headloop MethyLight™ method, HeavyMethyl™ MethyLight™ method, HeavyMethyl™ Scorpion™ method, MSP Scorpion™ method, Headloop Scorpion™ method, methylation sensitive primer extension, and Ms-SNuPE (Methylation-sensitive Single Nucleotide Primer Extension) method. A person skilled in the art knows how to perform such methods. Preferably such methods are performed as described above.

According to a particular preferred embodiment, the method of the invention is a method of detection of sample interchange, crosscontamination, or both in the field of methylation analysis, comprising the first two steps in the indicated order:

providing a sample set of at least two biological samples, wherein at least one sample comprises genomic DNA differentially methylated at least at one position,
applying at least one identifier for each sample,
subjecting each sample with at least one identifier to a detection or quantification reaction that is specific for the at least one identifier, and
deducing the presence or absence of a sample interchange, of a crosscontamination, or both from the presence or absence of at least one identifier in a single sample.

In a particular preferred embodiment, the step of deducing the presence or absence of a sample interchange, of a crosscontamination, or both further comprises
deducing the extent of a crosscontamination for a single sample from the absolute or relative amount of at least one identifier present in said single sample.

Said particular preferred embodiments of detection of sample interchange, crosscontamination, or both in the field of methylation analysis are carried out essentially as the herein described embodiments of identifying at least one biological sample in the field of methylation analysis.

According to a particular preferred embodiment, identifiers are applied to samples of a sample set as indicated by Figure 2. Each sample of the set is represented by two different identifiers, for example, but not limited to, by two different identifiers belonging to a set of variants of sequence polymorphism. Preferably, thereby each identifier is encoded by a different plasmid. A respective sample can be identified in between steps of an experimental procedure or subsequent to it by amplifying the variants of the sequence polymorphism and hybridizing the amplicon onto a chip. Each combination of identifiers results In a unique hybridization pattern. A sample interchange is detected if two hybridization signals are

detected whereby one or both are not characteristic for the applied identifiers for said sample. A sample cross contamination is detected if at least three instead of two hybridization signals are detected. Thereby the non-expected hybridizations signals for said sample indicate with which one or more samples the said sample is contaminated. By quantifying the hybridization signals it is possible to deduce the amount of contamination(s). According to this embodiment, the use of two different identifiers for each sample has the advantage that the assignment of the identifiers to the different samples is unambiguous. For example, in the case that two identical identifiers are used and two different hybridization signals are detected, it can not be distinguished between a cross contamination and a sample to which two different identifiers are applied.

According to a particular preferred embodiment a maximum of 28 samples are encoded by means of the above described system of two different identifiers. According to other particular preferred embodiments, the use of various different numbers of identifiers are used for coding samples. These embodiments are summarized in Table 1.

Table 1

| number of plasmids per sample →<br>number of identifier per plasmid ↓ | 1 | 2 | 3 | 4 | 5 | 6 | 7 | 8 |
|---|---|---|---|---|---|---|---|---|
| 1 | 1 | - | - | - | - | - | - | - |
| 2 | 2 | 1 | - | - | - | - | - | - |
| 3 | 3 | 3 | 1 | - | - | - | - | - |
| 4 | 4 | 6 | 4 | 1 | - | - | - | - |
| 5 | 5 | 10 | 10 | 5 | 1 | - | - | - |
| 6 | 6 | 15 | 20 | 15 | 6 | 1 | - | - |
| 7 | 7 | 21 | 35 | 35 | 21 | 7 | 1 | - |
| 8 | 8 | 28 | 56 | 70 | 56 | 28 | 8 | 1 |
| 9 | 9 | 36 | 84 | 126 | 126 | 84 | 36 | 9 |
| 10 | 10 | 45 | 120 | 210 | 252 | 210 | 120 | 45 |
| 11 | 11 | 55 | 165 | 330 | 462 | 462 | 330 | 165 |
| 12 | 12 | 66 | 220 | 495 | 792 | 924 | 792 | 495 |
| 13 | 13 | 78 | 286 | 715 | 1287 | 1716 | 1716 | 1287 |
| 14 | 14 | 91 | 364 | 1001 | 2002 | 3003 | 3432 | 3003 |
| 15 | 15 | 105 | 455 | 1365 | 3003 | 5005 | 6435 | 6435 |
| 16 | 16 | 120 | 560 | 1820 | 4368 | 8008 | 11440 | 12870 |
| 17 | 17 | 136 | 680 | 2380 | 6188 | 12376 | 19448 | 24310 |
| 18 | 18 | 153 | 816 | 3060 | 8568 | 18564 | 31824 | 43758 |
| 19 | 19 | 171 | 969 | 3876 | 11628 | 27132 | 50388 | 75582 |
| 20 | 20 | 190 | 1140 | 4845 | 15504 | 38760 | 77520 | 125970 |
| 21 | 21 | 210 | 1330 | 5985 | 20349 | 54264 | 116280 | 203490 |
| 22 | 22 | 231 | 1540 | 7315 | 26334 | 74613 | 170544 | 319770 |
| 23 | 23 | 253 | 1771 | 8855 | 33649 | 100947 | 245157 | 490314 |
| 24 | 24 | 276 | 2024 | 10626 | 42504 | 134596 | 346104 | 735471 |
| 25 | 25 | 300 | 2300 | 12650 | 53130 | 177100 | 480700 | 1081575 |
| 26 | 26 | 325 | 2600 | 14950 | 65780 | 230230 | 657800 | 1562275 |
| 27 | 27 | 351 | 2925 | 17550 | 80730 | 296010 | 888030 | 2220075 |
| 28 | 28 | 378 | 3276 | 20475 | 98280 | 376740 | 1184040 | 3108105 |
| 29 | 29 | 406 | 3654 | 23751 | 118755 | 475020 | 1560780 | 4292145 |

| | | | | | | | | |
|---|---|---|---|---|---|---|---|---|
| 30 | 30 | 435 | 4060 | 27405 | 142506 | 593775 | 2035800 | 5852925 |
| 31 | 31 | 465 | 4495 | 31465 | 169911 | 736281 | 2629575 | 7888725 |
| 32 | 32 | 496 | 4960 | 35960 | 201376 | 906192 | 3365856 | 10518300 |
| 33 | 33 | 528 | 5456 | 40920 | 237336 | 1107568 | 4272048 | 13884156 |
| 34 | 34 | 561 | 5984 | 46376 | 278256 | 1344904 | 5379616 | 18156204 |
| 35 | 35 | 595 | 6545 | 52360 | 324632 | 1623160 | 6724520 | 23535820 |
| 36 | 36 | 630 | 7140 | 58905 | 376992 | 1947792 | 8347680 | 30260340 |
| 37 | 37 | 666 | 7770 | 66045 | 435897 | 2324784 | 10295472 | 38608020 |
| 38 | 38 | 703 | 8436 | 73815 | 501942 | 2760681 | 12620256 | 48903492 |
| 39 | 39 | 741 | 9139 | 82251 | 575757 | 3262623 | 15380937 | 61523748 |
| 40 | 40 | 780 | 9880 | 91390 | 658008 | 3838380 | 18643560 | 76904685 |
| 41 | 41 | 820 | 10660 | 101270 | 749398 | 4496388 | 22481940 | 95548245 |
| 42 | 42 | 861 | 11480 | 111930 | 850668 | 5245786 | 26978328 | 118030185 |
| 43 | 43 | 903 | 12341 | 123410 | 962598 | 6096454 | 32224114 | 145008513 |
| 44 | 44 | 946 | 13244 | 135751 | 1086008 | 7059052 | 38320568 | 177232627 |
| 45 | 45 | 990 | 14190 | 148995 | 1221759 | 8145060 | 45379620 | 215553195 |
| 46 | 46 | 1035 | 15180 | 163185 | 1370754 | 9366819 | 53524680 | 260932815 |
| 47 | 47 | 1081 | 16215 | 178365 | 1533939 | 10737573 | 62891499 | 314457495 |
| 48 | 48 | 1128 | 17296 | 194580 | 1712304 | 12271512 | 73629072 | 377348994 |
| 49 | 49 | 1176 | 18424 | 211876 | 1906884 | 13983816 | 85900584 | 450978066 |
| 50 | 50 | 1225 | 19600 | 230300 | 2118760 | 15890700 | 99884400 | 536878650 |
| 51 | 51 | 1275 | 20825 | 249900 | 2349060 | 18009460 | 115775100 | 636763050 |
| 52 | 52 | 1326 | 22100 | 270725 | 2598960 | 20358520 | 133784560 | 752538150 |
| 53 | 53 | 1378 | 23426 | 292825 | 2869685 | 22957480 | 154143080 | 886322710 |
| 54 | 54 | 1431 | 24804 | 316251 | 3162510 | 25827165 | 177100560 | 1040465790 |
| 55 | 55 | 1485 | 26235 | 341055 | 3478761 | 28989675 | 202927725 | 1217566350 |
| 56 | 56 | 1540 | 27720 | 367290 | 3819816 | 32468436 | 231917400 | 1420494075 |
| 57 | 57 | 1596 | 29260 | 395010 | 4187106 | 36288252 | 264385836 | 1652411475 |
| 58 | 58 | 1653 | 30856 | 424270 | 4582116 | 40475358 | 300674088 | 1916797311 |
| 59 | 59 | 1711 | 32509 | 455126 | 5006386 | 45057474 | 341149446 | 2217471399 |
| 60 | 60 | 1770 | 34220 | 487635 | 5461512 | 50063860 | 386206920 | 2558620845 |
| 61 | 61 | 1830 | 35990 | 521855 | 5949147 | 55525372 | 436270780 | 2944827765 |
| 62 | 62 | 1891 | 37820 | 557845 | 6471002 | 61474519 | 491796152 | 3381098545 |
| 63 | 63 | 1953 | 39711 | 595665 | 7028847 | 67945521 | 553270671 | 3872894697 |
| 64 | 64 | 2016 | 41664 | 635376 | 7624512 | 74974368 | 621216192 | 4426165368 |
| 65 | 65 | 2080 | 43680 | 677040 | 8259888 | 82598880 | 696190560 | 5047381560 |
| 66 | 66 | 2145 | 45760 | 720720 | 8936928 | 90858768 | 778789440 | 5743572120 |
| 67 | 67 | 2211 | 47905 | 766480 | 9657648 | 99795696 | 869648208 | 6522361560 |
| 68 | 68 | 2278 | 50116 | 814385 | 10424128 | 109453344 | 969443904 | 7392009768 |
| 69 | 69 | 2346 | 52394 | 864501 | 11238513 | 119877472 | 1078897248 | 8361453672 |
| 70 | 70 | 2415 | 54740 | 916895 | 12103014 | 131115985 | 1198774720 | 9440350920 |
| 71 | 71 | 2485 | 57155 | 971635 | 13019909 | 143218999 | 1329890705 | 1,0639E+10 |
| 72 | 72 | 2556 | 59640 | 1028790 | 13991544 | 156238908 | 1473109704 | 1,1969E+10 |
| 73 | 73 | 2628 | 62196 | 1088430 | 15020334 | 170230452 | 1629348612 | 1,3442E+10 |
| 74 | 74 | 2701 | 64824 | 1150626 | 16108764 | 185250786 | 1799579064 | 1,5071E+10 |
| 75 | 75 | 2775 | 67525 | 1215450 | 17259390 | 201359550 | 1984829850 | 1,6871E+10 |
| 76 | 76 | 2850 | 70300 | 1282975 | 18474840 | 218618940 | 2186189400 | 1,8856E+10 |
| 77 | 77 | 2926 | 73150 | 1353275 | 19757815 | 237093780 | 2404808340 | 2,1042E+10 |
| 78 | 78 | 3003 | 76076 | 1426425 | 21111090 | 256851595 | 2641902120 | 2,3447E+10 |
| 79 | 79 | 3081 | 79079 | 1502501 | 22537515 | 277962685 | 2898753715 | 2,6089E+10 |
| 80 | 80 | 3160 | 82160 | 1581580 | 24040016 | 300500200 | 3176716400 | 2,8988E+10 |

| | | | | | | | | |
|---|---|---|---|---|---|---|---|---|
| 81 | 81 | 3240 | 85320 | 1663740 | 25621596 | 324540216 | 3477216600 | 3,2164E+10 |
| 82 | 82 | 3321 | 88560 | 1749060 | 27285336 | 350161812 | 3801756816 | 3,5641E+10 |
| 83 | 83 | 3403 | 91881 | 1837620 | 29034396 | 377447148 | 4151918628 | 3,9443E+10 |
| 84 | 84 | 3486 | 95284 | 1929501 | 30872016 | 406481544 | 4529365776 | 4,3595E+10 |
| 85 | 85 | 3570 | 98770 | 2024785 | 32801517 | 437353560 | 4935847320 | 4,8125E+10 |
| 86 | 86 | 3655 | 102340 | 2123555 | 34826302 | 470155077 | 5373200880 | 5,306E+10 |
| 87 | 87 | 3741 | 105995 | 2225895 | 36949857 | 504981379 | 5843355957 | 5,8434E+10 |
| 88 | 88 | 3828 | 109736 | 2331890 | 39175752 | 541931236 | 6348337336 | 6,4277E+10 |
| 89 | 89 | 3916 | 113564 | 2441626 | 41507642 | 581106988 | 6890268572 | 7,0625E+10 |
| 90 | 90 | 4005 | 117480 | 2555190 | 43949268 | 622614630 | 7471375560 | 7,7516E+10 |
| 91 | 91 | 4095 | 121485 | 2672670 | 46504458 | 666563898 | 8093990190 | 8,4987E+10 |
| 92 | 92 | 4186 | 125580 | 2794155 | 49177128 | 713068356 | 8760554088 | 9,3081E+10 |
| 93 | 93 | 4278 | 129766 | 2919735 | 51971283 | 762245484 | 9473622444 | 1,0184E+11 |
| 94 | 94 | 4371 | 134044 | 3049501 | 54891018 | 814216767 | 1,0236E+10 | 1,1132E+11 |
| 95 | 95 | 4465 | 138415 | 3183545 | 57940519 | 869107785 | 1,105E+10 | 1,2155E+11 |
| 96 | 96 | 4560 | 142880 | 3321960 | 61124064 | 927048304 | 1,1919E+10 | 1,326E+11 |

Table 1 gives an overview over various particular preferred embodiments. It shows the maximum amount of samples which can be encoded by means of 1, 2, 3, 4, 5, 6, 7, or 8 plasmids and by means of 1 to 96 identifiers, whereby each identifier can be comprised by each plasmid. In general, the following formula expresses the maximum amount of samples which can be encoded by means of a certain number of plasmids and a certain number of identifiers:

$$K\ (n,k)=\binom{n}{k}$$

Thereby K represents the maximum number of samples which can be encoded, k represents the number of identifiers (e.g. polymorphism variants), and n represents the number of plasmids. Of course further correspondent embodiments are possible and herewith preferred.

According to a particular preferred embodiment, the method of the invention is a method of identifying a sample in a pooled sample set in the field of methylation analysis, comprising the first two steps in the indicated order:

providing a pooled sample set of at least two biological samples, wherein at least one sample comprises genomic DNA differentially methylated at least at one position;
applying at least one identifier for each sample;
subjecting the sample set to a detection or quantification reaction that is specific for the at least one identifier of each sample; and
identifying a sample in the pooled sample set by detecting the respective applied at least one identifier.

The method comprises contacting the DNA of each sample and the applied at least one identifier with a bisulfite reagent which differentiates between a methylated or an unmethylated position. Said particular preferred embodiments of identifying a sample in a pooled sample set in the field of methylation analysis are carried out essentially as the herein described embodiments of identifying at least one biological sample in the field of methylation analysis.

According to a particular preferred embodiment, samples taken from the same individual are encoded by identifiers of the same set of identifiers. This allows an organ specific encoding of the samples, a tissue specific encoding of the samples, a body specific encoding of the samples, or combinations thereof. According to a particular embodiment, samples taken from different individuals are encoded by identifiers of different sets of identifiers. It is further particular preferred, to combine these two embodiments, so that it is possible to pool the samples derived from the same individual and to analyze them simultaneously with pooled samples of the other individuals. The samples can be assigned to the corresponding individuals by means of the individual specific identifiers. It is possible to identify each sample and to assign it to analysis results by means of the identifiers specific for each sample obtained from the same individual. Therefore it is necessary that each identifier specific for each sample is associated with the genomic DNA or has similar properties as the genomic DNA, in case exogenous identifiers are used. But of course also sample DNA endogenous identifiers are used and also preferred. Such exogenous or endogenous identifiers can, for example but not limited to, be all kinds of polymorphisms (sequence, length, deletion, SNP).

According to a particular preferred embodiment, the method of the invention is a method of detection of an amplification inhibition in the field of methylation analysis, comprising the first two steps in the indicated order:

providing a sample set of at least two biological samples, wherein at least one sample comprises genomic DNA differentially methylated at least at one position;
applying at least one identifier for each sample;
subjecting each sample with at least one identifier to an amplification reaction that is specific for the at least one identifier; and
deducing a presence, absence or partial amplification inhibition from the presence, absence, or amount of the product of the Identifier specific amplification reaction.

This embodiments comprises contacting the DNA of each sample and the applied at least one Identifier with a bisulfite reagent which differentiates between a methylated or an unmethylated position. Said particular preferred embodiments of detection of an amplification inhibition in the field of methylation analysis are carried out essentially as the herein described embodiments of identifying at least one biological sample in the field of methylation analysis.

According to this embodiment, a person skilled in the art can easily detect an amplification inhibition because at least one identifier is applied which can be detected by amplification. He deduces a presence of an amplification inhibition in case the applied identifier(s) is not amplifiable any more.

According to a particular preferred embodiment, the method of the invention is a method of normalization, calibration, or both in the field of methylation analysis, comprising the first two steps in the indicated order:

providing a sample set of at least two biological samples, wherein at least one sample comprises genomic DNA differentially methylated at least at one position;
applying at least one identifier for each sample by adding at least one identifier to each provided sample;
subjecting each sample with at least one identifier to a detection or quantification reaction; and
normalizing at least one sample, calibrating an experimental procedure, or both according to the detected or quantified one or more identifiers compared to the added total amount of the one or more identifiers. Thereby normalization means a correction according to standards, in particular according to the used one or more identifiers. Furthermore, calibration means an adjustment of an experimental procedure or method, so that procedure or method characteristic values lie within certain pre-defined ranges. According to the invention, such characteristic values can be those derived from one or more identifiers.

According to a preferred embodiment, the one or more identifier applied for at least one biological sample are detected or quantified at least twice during an experimental procedure. Preferably, they are detected or quantified after one or more steps of the experimental procedure and subsequent to the experimental procedure.

This embodiment comprises contacting the DNA of each sample and the applied at least one identifier with a bisulfite reagent which differentiates between a methylated or an unmethylated position. The particular preferred embodiments of normalization, calibration, or both in the field of methylation analysis are carried out essentially as the herein described embodiments of identifying at least one biological sample in the field of methylation analysis.

According to this embodiment, the one or more identifiers are applied in a defined amount. It is possible to calibrate an experimental procedure or to normalize samples processed by an experimental procedure by using these identifiers as an internal standard and taking the quantitative changes into consideration. Such changes might be determined, for example but not limited to, by pipetting steps or amplifications.

According to a particular preferred embodiment, the method of the invention is a method of identification of a carry-over contamination in the field of methylation analysis, comprising the first two steps in the indicated order:

providing a sample set of at least two biological samples, wherein at least one sample comprises genomic DNA differentially methylated at least at one position;
applying at least one identifier for each sample;
subjecting each sample with at least one identifier to a detection or quantification reaction that is specific for at least one identifier; and
deducing the presence of a sample carry over contamination from the presence of at least one identifier not applied for said sample, or deducing the absence of a sample contamination from the absence of identifiers not applied for said sample.

This embodiment comprises contacting the DNA of each sample and the applied at least one identifier with a bisulfite reagent which differentiates between a methylated or an unmethylated position. Said particular preferred embodiments of identification of a carry-over contamination in the field of methylation analysis are carried out essentially

as the herein described embodiments of identifying at least one biological sample in the field of methylation analysis.

According to this embodiment, each set of samples is provided with an identifier when it is processed or stored at a certain location. A carry over contamination is existent, in case a previous for another sample set used identifier is detected for a sample set.

According to a particular preferred embodiment, the method of the invention is a method of assessing the success of a hybridization step in the field of methylation analysis, comprising the first two steps in the indicated order:

providing a sample set of at least two biological samples, wherein at least one sample comprises genomic DNA differentially methylated at least at one position; applying at least one identifier;
subjecting each sample Including the applied at least one identifier to a detection or quantification reaction that is specific for the said at least one identifier; wherein the detection or quantification reaction comprises a hybrization step,
assessing the success of the hybridization step wherein (a) the presence of a signal derived for the applied at least one identifier Indicates the presence of a successful hybrization step, and wherein (b) the absence of signal derived for the applied at least one identifier indicates the presence of an unsuccessful hybrization step.

This embodiment comprises contacting the DNA of each sample and the applied at least one identifier with a bisulfite reagent which differentiates between a methylated or an unmethylated position. Said particular preferred embodiments of assessing the success of a hybridization step in the field of methylation analysis are carried out essentially as the herein described embodiments of identifying at least one biological sample in the field of methylation analysis.

According to this embodiment, it is in principle sufficient to apply only one identifier per experimental batch. Preferably, two or more identifiers are applied for a single experimental batch. This is in particular the case wherein the processed samples are treated differently or independently from one another. Most preferably, at least one identifier is applied for every sample to be processed per experimental batch.

According to a particular preferred embodiment, the method of the invention is a method of determining the rate of DNA conversion in the field of methylation analysis, comprising the first two steps in the indicated order:

providing a sample set of at least two biological samples, wherein at least one sample comprises genomic DNA differentially methylated at least at one position;
applying at least one identifier for each sample, at least one of the applied identifiers comprises a cytosine that is not part of a CpG dinucleotide;
subjecting each sample with at least one identifier to at least one reaction that converts unmethylated cytosines to a base with a different base pairing behaviour than cytosine, in particular to uracil, while methylated cytosines remain unchanged;
subjecting the at least one identifier of each sample to at least one quantification reaction, wherein the total amount of identifier and the amount of converted identifier are detected; and
determining the rate of DNA conversion according to the amount of converted identifier compared to the total amount of identifier.

According to this embodiment, the conversion rate of a bisulfite treatment is determined by quantifying the amount of an applied identifier before a step and thereafter. Thereby the step comprises contacting the DNA and at least one identifier with a bisulfite reagent. Usually the amount after treatment is divided by the amount before treatment. But further possibilities are possible and also included herewith.

According to other embodiments, the efficiency of an experimental procedure or only steps of it are determined correspondingly as the conversion rate of bisulfite treatment.

Further disclosed is a method for testing an experimental procedure, comprising
applying at least one identifier instead of a biological sample to an experimental procedure;
subjecting the one or more identifiers to a detection or quantification reaction that is specific for the said one or more identifiers, and that is carried out before or after individual steps of the experimental procedure or subsequent to it.

Further disclosed is a method for testing an experimental procedure in the field of methylation analysis, comprising
applying at least one identifier instead of a biological sample to an experimental procedure, the experimental procedure comprising contacting the applied at least one identifier with a reagent or enzyme which differentiates between a methylated or an unmethylated position; subjecting the one or more identifiers to a detection or quantification reaction that is specific for the said one or more identifiers, and that is carried out before or after individual steps of he experimental procedure or subsequent to it.

Accordingly, one or more identifiers substituting a biological sample are referred herein as identifier sample.

In these embodiments, one or more identifiers may substitute a biological sample. Preferably, one or more

identifiers substitute a biological sample within a sample set or one or more identifiers substitute a sample set resulting in an identifier sample set. According to these embodiments, the one or more identifiers are provided in comparable, similar or identical manners as a biological sample. For example, the one or more identifiers are provided in the same container, in the same buffer or both as a biological sample. But, according to these embodiments, the one or more identifiers can also be provided as dried substance or in solution with water or any suitable buffer. According to these embodiments the identifiers are detected or quantified in between individual steps of the experimental procedure or at its end. The detection or quantification reaction is carried out as specified by other embodiments described herein. A person skilled In the art knows to adjust them if necessary. According to this embodiment, the identifier samples or identifier samples sets are subjected to other embodiments described herein.

It is disclosed that the experimental procedure is for example but not limited to it any combination of chemical, biological or physical reactions.

It is disclosed that the testing of an experimental procedure comprises at least one of the following

- determining the probability for a sample interchange, crosscontamination, or both;
- determining the extent of a possible crosscontamination;
- determining the probability of Identifying a sample in a pooled sample set;
- determining the probability of an amplification inhibition;
- calibrating the experimental procedure;
- determining the necessity of normalization;
- determining the probability of carry over contamination;
- determining the efficiency of a reaction, of a step of said experimental procedure, or of the complete experimental procedure;
- optimizing the experimental procedure; and
- determining the presence of a successful hybridization step or the presence of an unsuccessful hybridization step.

It is disclosed that the testing of an experimental procedure comprises determining the probability for a sample interchange, crosscontamination, or both. Accordingly, at least one or more identifiers substitute a biological sample (identifier sample) and at least two identifier samples are applied to an experimental procedure. A sample interchange or crosscontamination is determined by detecting the presence or absence of applied identifiers. A interchange is determined wherein a) at least one of the applied identifiers of an identifier sample is not detected, and wherein b) at least one identifier Is detected that was applied to a different identifier sample, said identifiers samples being processed at the same time. A crosscontamination of an identifier sample is determined wherein a) at least one identifier is detected that was applied to a different identifier sample and both identifier samples were processed at the same time, and wherein b) all of the applied identifiers of said identifier sample are detected. A simultaneous interchange and crosscontamination is detected wherein a interchange and a cross contamination are determined for a single identifier sample for the same experimental procedure run. The probability for a sample interchange is determined by multiplying the quotient of the halved number of interchanged identifier samples and the total number of analyzed identifier samples with the factor 100. The probability for a crosscontamination is analogically determined by multiplying the quotient of the number of crosscontaminated identifier samples and the total number of analyzed identifier samples by the factor 100. The probability for simultaneous sample interchange and crosscontamination is also determined analogically by multiplying the quotient of the number of interchanged and crosscontaminated identifier samples and the total number of analyzed identifiers samples by the factor 100. Of course and also preferred, a person skilled in the art is aware of other suitable algorithms.

It is disclosed that the testing of an experimental procedure comprises determining the extent of a crosscontamination. Accordingly, at least one or more identifiers substitute a biological sample (identifier sample) and at least two identifier samples are applied to an experimental procedure. The extent of a crosscontamination is determined by determining the ratio of the amount of contaminating identifier and the total amount of identifier detected in the quantification reaction for an identifier sample. According to a particular preferred embodiment, a probability distribution for the extent of crosscontaminations of an experimental procedure is determined by considering many crosscontaminated identifier samples. Of course, and also preferred, a person skilled in the art is aware of other suitable algorithms.

It is disclosed that the testing of an experimental procedure In the field of methylation analysis comprises determining the probability for a sample interchange, crosscontamination, or both as specified above. Accordingly the determining of the probability for a sample interchange, crosscontamination, or both comprises contacting of at least one identifier with a bisulfite reagent.

It is disclosed that the testing of an experimental procedure comprises determining the probability of identifying a sample, in particular in a pooled sample set. Accordingly, the one or more identifiers are applied instead of a biological sample (identifier sample). Preferably, an arbitrarily number of identifier samples is combined to a pooled sample set. The identifier sample or the preferred identifier sample set is then applied to an experimental procedure. The probability of identifying a sample before or after an individual step of the experimental procedure or at the end of it is determined

by performing many times the detection or quantification reaction specific for an identifier, the identifier being part of an identifier sample. The probability is then determined by multiplying the ratio of successful attempts in detecting said identifier and the total attempt in detecting said identifier by the factor 100. Of course, also preferred are other suitable algorithms a person skilled in the art is aware of. These particular embodiments are particular of use for determining the minimum amount of identifier or biological sample which has to be applied to an experimental procedure to obtain reliable results. Preferably, these embodiments are of particular use for determining the amount of identifier or biological sample which allow reliable results with a likelihood of about 60%, about 75%, of about 85%, of about 95%, of about 98%, of about 99%, or about 100%.

It is disclosed that, the testing of an experimental procedure in the field of methylation analysis comprises determining the probability of identifying a sample, in particular in a pooled sample set as specified above. Accordingly, the determining of the probability of identifying a sample, in particular in a pooled sample set comprises contacting of at least one identifier with a bisulfite reagent.

It is disclosed that the testing of an experimental procedure comprises determining the probability of an amplification inhibition. Accordingly, the one or more identifiers are applied to an experimental procedure instead of a biological sample (identifier sample). An amplification inhibition for an identifier sample is present wherein at least one applied identifier is not detected during a previously established amplification based detection or quantification. The probability of an amplification inhibition is determined by multiplying the ratio of the number of unsuccessful detection attempts and the total number of detection attempts by the factor 100. Of course and also preferred, a person skilled in the art is aware of other suitable algorithms. Preferably, for determining the probability of amplification inhibition, one or more identifier samples are applied to the experimental procedure and at least two identifiers are subjected to correspondent previously established amplification based detections or quantifications. The probability of an amplification inhibition is then determined by averaging the probabilities of an amplification inhibition determined for each considered identifier. Of course, also preferred are other suitable algorithms a person skilled in the art is aware of.

It is disclosed that the testing of an experimental procedure in the field of methylation analysis comprises determining the probability of an amplification inhibition as specified above. Accordingly, the determining of the probability of an amplification inhibition comprises contacting of at least one identifier with a bisulfite reagent.

It is disclosed that the testing of an experimental procedure comprises calibrating the experimental procedure. Accordingly, at least one or more identifiers substitute a biological sample (identifier sample) and at least one identifier sample is applied to an experimental procedure. The calibration of the experimental procedure is realized in amending the different single steps of the experimental procedure so that the applied one or more identifiers are detected with a certain predefined likelihood, that the amount of the applied one or more identifiers determined in the quantification reaction lies within a certain predefined range, or both. The predefined likelihood and the predefined quantification range are determined by at least one run of the optimized experimental procedure. This embodiment is particularly preferred whenever an experimental procedure is established or reestablished, for example, but not limited to, after a location change or after a time period not performing the procedure.

It is disclosed that the testing of an experimental procedure in the field of methylation analysis comprises calibrating the experimental procedure as specified above. Accordingly, the calibrating of an experimental procedure comprises contacting of at least one identifier with a bisulfite reagent.

It is disclosed that the testing of an experimental procedure comprises determining the necessity of normalization for said experimental procedure. Accordingly, at least one or more identifiers substitute a biological sample (identifier sample) and at least one identifier sample is applied to an experimental procedure. The applied identifiers are quantified before or after individual steps of the experimental procedure or at its end. The necessity of normalization is determined wherein the amounts of one or more quantified identifiers is not within a certain predefined range. In many cases, but not limited to them, this range is determined by subsequent data processing, analysis or interpretations and not by the experimental procedure itself. Wherein the necessity is determined, also suitable algorithms for normalization are preferred a person skilled in the art is aware of or is able to adjust

It is disclosed that the testing of an experimental procedure in the field of methylation analysis comprises determining the necessity of normalization for said experimental procedure as specified above. For example but not limited to it, the quantification of the effect of a methylation sensitive digestion can be normalized by one or more applied identifiers containing a recognition site for the used enzyme or enzymes. According to a particular preferred embodiment, the determining the necessity of normalization for an experimental procedure comprises contacting of at least one identifier with a bisulfite reagent.

It is disclosed that the testing of an experimental procedure comprises determining the probability of carry over contamination. Accordingly, at least one or more identifiers substitute a biological sample (identifier sample), and at least one identifier sample is applied sequentially to at least two experimental procedure runs. Thereby said experimental procedure runs can comprise the same or similar reaction(s) or not. A carry over contamination for an identifier sample is determined wherein an identifier is detected that was applied to a previous run. A sample is considered as free from a carry-over contamination wherein no identifier is detected that is indicative for a previous run. The probability of carry

over contamination for an experimental procedure is determined by multiplying the ratio of the number of carry over contaminated sample and the total number of samples by the factor 100. Of course and also preferred, a person skilled in the art is aware of other suitable algorithms.

It is disclosed that the testing of an experimental procedure in the field of methylation analysis comprises determining the probability of carry over contamination as specified above. Accordingly, the determining the probability of carry over contamination comprises contacting of at least one identifier with a bisulfite reagent.

It is disclosed that the testing of an experimental procedure comprises determining the efficiency of a reaction, of a step of said experimental procedure, of the complete experimental procedure, or combinations thereof. Accordingly, at least one or more identifiers substitute a biological sample (identifier sample). At least one identifier sample is subjected to the experimental procedure comprising at least one reaction or step, said step comprising at least one reaction. The efficiency is determined by considering the amount of at least one identifier before and after a reaction, before and after a step, or at the beginning and the end of the experimental procedure. The efficiency of a reaction is determined by the ratio of the amount of an identifier before the reaction and the amount of the said identifier or its derivative after the reaction. The efficiency of a procedure step is determined by the ratio of the amount of an identifier before the step and the amount of the said identifier or its derivative after the said step. The efficiency of a experimental procedure is determined by the ratio of the amount of an identifier at the beginning of the experimental procedure and the amount of the said identifier or its derivative at the end of the experimental procedure. Alternatively, the efficiency of a step of a experimental procedure is determined by multiplying the efficiencies of all reactions that are part of the said step with each other. Analogically, the efficiency of a complete experimental procedure is determined by either a) multiplying the efficiencies of all steps that are part of the experimental procedure with each other, or b) by multiplying the efficiencies of all reactions that are part of the experimental procedure with each other. A person skilled in the art is aware of further suitable algorithms. Those are herewith also preferred.

It is disclosed that the testing of an experimental procedure in the field of methylation analysis comprises determining the efficiency of a reaction, of a step of said experimental procedure, of the complete experimental procedure, or combinations thereof. Accordingly, the efficiency of a reaction, procedure step, or experimental procedure is determined wherein the said comprises contacting of at least one identifier with a bisulfite reagent.

It is disclosed that the testing of an experimental procedure comprises optimizing the experimental procedure. According to this embodiment, at least one or more identifiers substitute a biological sample (identifier sample), and at least one identifier sample is applied to an experimental procedure. The experimental procedure is optimized by amending at least one step or reaction of the experimental procedure. This amendment is carried out according to different aims, for example, but not limited to, for a maximum amount of an identifier or its derivative at the end of the experimental procedure. Of course and also preferred, a person skilled in the art is aware of further aims. He is also aware of how to amend one or more reaction or one or more step to achieve a certain aim.

It is disclosed that the testing of an experimental procedure in the field of methylation anaylsis comprises optimizing the experimental procedure as specified above. Accordingly, the optimizing of the experimental procedure comprises contacting of at least one identifier with a bisulfite reagent.

It is disclosed that the testing of an experimental procedure comprises assessing the success of a hybridization step. Accordingly at least one or more identifiers substitute a biological sample (identifier sample), and at least one identifier sample is applied to an experimental procedure. This experimental procedure comprises an hybridization step which by itself may be characterized by different substeps like DNA preparation, prehybridization, hybridization, washing steps, or detection. Correspondingly any material, solution or substances may be used for this hybridization step as long as the hybridization is enabled of the processed applied identifier and the respective processed DNA derived from the biological sample(s), respectively.

It is disclosed that the testing of an experimental procedure in the field of methylation anaylsis comprises assessing the success of a hybridization step of a experimental procedure as specified above. Accordingly, the assessing the success of a hybridization step comprises contacting of at least one identifier with a bisulfite reagent.

In addition, particular aspects of the invention refer to controlling a process or method.

The following technical problems underlie the inventive embodiments for controlling the correctness of a process or method. Many laboratory routines require the parallel processing of samples, in particular a large number of samples. This is in particular the case for diagnostic, prognostic or screening purposes. Because of the large number of samples said processes or methods are highly accessible for errors. Such errors are for example but not limited to sample interchange or crosscontaminations. This error can occur on different levels of the analysis, for example but not limited to sample collection, sample preparation, DNA/RNA extraction, DNA/RNA modification, DNA/RNA amplification, or DNA/RNA characterization like PCR, Hybridization or sequencing. In addition, because of the large number of samples and a maybe limited number of applicable molecular identifiers, it might not be possible to assign a unique identifier to each sample. This is for example, but not limited to, the case, wherein the process or method comprises a real time PCR step. Only a limited number of dyes is detectable in real time PCR analysis, but up to 384 different sample can be measured in a single run.

The solution of the said problems is to use a small number of molecular identifiers e.g. 1-10 for spiking it/them into the samples in a defined order. Even if the number of samples is higher than the number of indentifiers an specific pattern of identifiers is expected at the end of the process. Every disorder of the samples is than easily recognizable by an unexpected order of the identifiers which are themselves identified according to a suitable method.

The particular advantage of this embodiment is that a lower number of molecular identifiers is needed to controll a process or method i.e. to exclude errors from a process or method. This results amongst others in lower handling efforts, costs, processing steps and a shorter analysis time.

Disclosed herein is a method for controlling the correctness of a process or method. Preferably said process or method is a high-throughput process or method. Preferably said process or method is a process or method for analysing DNA, genomic DNA or RNA. Preferably said process or method is a process or method for methylation analysis. The method of the invention comprises the following in arbitrary order:

A) The providing of a sample set of at least two or more biological samples which comprises DNA or RNA.
B) The assignment of one or more identifiers to the sample set, wherein preferably the identifiers are nucleic acids or at least in parts nucleic acids. Thereby the samples of the set become characterized by a pattern.
C) The execution of an experimental procedure, wherein all provided samples are analyzed. This analysis comprises for every sample the analysis of the provided sample DNA or RNA, the assigned at least one identifier, or both. Preferably this workflow enables detection or quantification of the methylation, the identifier, or both. Preferably the analysis of the sample DNA or RNA and the detection or quantification of the identifier are realized simultaneously.

A method of the invention is a method for controlling the correctness of a process or method, comprising

providing a sample set of at least 2, 3, 4, 100, 200, 400, or 800 biological samples, wherein each sample comprises a nucleic acid;
applying at least one identifier to the sample set, wherein the applied at least one identifier does not interfere with subsequent analysis, and wherein the applied identifiers generate an identification pattern across the samples;
subjecting each sample to a detection or quantification reaction specific for the one or more applied identifiers;
subjecting each sample to analysis;
deducing the correctness of said process or method from the signals of the detected or quantified identifiers of the samples.

According to a preferred embodiment, at least one identifier is applied to each sample of the sample set.

According to a preferred embodiment, the method of the invention is a method for controlling the correctness of a process or method. Said method comprises the following:

(A) The providing of a sample set of 2, 3, 4, 10, 30, 60, 100, 200, 400, 800, 1000, 1500, 4000, 10000 or more samples. Thereby each samples comprises a nucleic acid to be analyzed. Preferably, said nucleic acid is genomic DNA or RNA. Of course and it is obvious to those skilled in the art said embodiment can also be applied to processes or methods, wherein proteins, peptides, metabolic compounds, hormones, lipids, cells, or combinations thereof are analyzed.

(B) The applying of at least one identifier to the sample set. Preferably only one identifier is applied to the sample set. Preferably only a single identifier is applied to each sample of the set. Preferably the total number of applied different identifiers is smaller than the total number of samples. Most preferably the total number of different identifiers is only 2, 3, 4, 5, 6, 7, 8, 9, 10.
The applied identifier or identifiers are characterized in that they enable subsequent analysis and they give rise to an identification pattern of the samples. Preferably, said pattern is a spatial or geometrical pattern. Preferably said pattern is any abstract order for example but not limited to numerical or alphabetical order. Preferably, every 2$^{nd}$, 3$^{rd}$, 4$^{th}$, 5$^{th}$, 6$^{th}$, 7$^{th}$, 8$^{th}$, 9$^{th}$, 10$^{th}$, etc. sample is encoded by the same identifier or group of identifiers. Preferably, the samples are placed in an hexagonal order and the same identifier or identifiers are applied to all samples of a row or a column. Preferably, the samples are placed in an radial order and the same identifier or identifiers are applied to all samples which have the same distance to a reference point for example the center. In a particular embodiment, the samples are encoded by two or more groups of identifiers. For example but not limited to, hexagonally orientated samples are encoded by a two groups of identifiers, wherein the first group encodes each row and the second groups encodes each column. Preferably each row, column or both are encoded by identifiers, wherein subgroups of the sample set may be formed, each sample of a subgroup be encoded by a specific combination of identifiers. Preferably one or more identifiers are applied to the sample set, whereby only every 2$^{nd}$, 3$^{rd}$, 4$^{th}$, 5$^{th}$, 6$^{th}$, 7$^{tn}$, 8$^{th}$, 9$^{th}$, 10$^{th}$, etc. sample is encoded by one or more identifiers and all other samples of the set obtain no Identifier.

Thereby, correspondingly, a identification pattern is generated.
According to the invention for the method of controlling the correctness of a process or method, the applied one or more identifiers are identifier(s) as described above for the method of identifying at least one biological sample in the field of methylation analysis.

(C) The subjecting of each sample to a detection or quantification reaction specific for the one or more applied identifiers. Thereby the detection or quantification reaction is any kind of detection or quantification reaction. Preferably the detection or quantification reaction is a detection or quantification reaction as described above for the method of identifying at least one biological sample in the field of methylation analysis.

(D) The subjecting of each sample to analysis. Thereby each sample is analyzed by any kind of analysis, process or method. Preferably said analysis is carried out as described above for the method of identifying at least one biological sample in the field of methylation analysis. In particular each sample is analyzed with respect to the methylation of one or more CpG dinucleotides.

(E) The deducing the correctness of the process or method from the signals of the detected or quantified identifiers of the samples.

According to a preferred embodiment, the deducing the correctness of said process or method from the signals of the detected or quantified identifiers of the samples, comprises
determining the presence of an error-free process or method, wherein the said signals generate a pattern that is corresponds to the identification pattern as initially generated by applying the identifiers to the samples; or
determining the absence of an error-free process or method, wherein the said signals generate a pattern that does not correspond to the identification pattern as initially generated by applying the identifiers to the samples.
In a preferred embodiment, the correctness of said process or method is deduced from the signals of the detected or quantified identifiers of the samples. This assessment is achieved by comparing the pattern of the derived signals with the identification pattern generated by the applied identifier or indentifiers. In case the order or geometrical position of the samples relative to each other is not alternated during the process or method run, the pattern of the signals can be directly compared with the original identification pattern. In case the order or geometrical position of the samples relative to each other has be altered, the alterations have to be considered in the comparison with the original identification pattern. The presence of an accurate i.e. error-free process or method run is deduced, wherein the pattern of the signals is identical - no alteration of sample order or positions -, or resembles - alteration of sample order or positions - the originally identification pattern. On the other hand, the absence of an accurate i.e. error-free process or method run is deduced, wherein the pattern of the signals is not identical - no alteration of sample order or positions -, or resembles not - alteration of sample order or positions - the originally identification pattern.
According to a preferred embodiment, the process or method is a high-throughput process or method. Preferably it is a process or method in the field of methylation analysis and most preferably it is a high-throughput process or method in the field of methylation analysis.
According to the invention, the said embodiments for controlling the correctness of a process or method is applicable for quality ensurance or assessing the correctness of process runs for diagnostic, prognostic or screening purposes. Amongst others, it is in particular suitable for the application in reference laboratories.

<u>Kit.</u>

Disclosed is also a kit, comprising a container and one or more of the following:

at least one nucleic acid comprising at least one sequence polymorphic section;
at least one nucleic acid comprising at least one length polymorphic section;
at least one plasmid comprising at least one sequence polymorphic section;
at least one plasmid comprising at least one length polymorphic section;
at least one nucleic acid comprising at least one sequence polymorphic section and one length polymorphic section;
at least one oligonucleotide containing target-specific priming site and at least one sequence polymorphic section;
at least one oligonucleotide for amplifying at least one sequence polymorphic nucleic acid section, said oligonucleotide comprising DNA and/or DNA analogs like for example, but not limited to PNA, LNA, HNA, phosphothioate DNA, methylphosphonate DNA;
at least one oligonucleotide for amplifying at least one length polymorphic nucleic acid section, said oligonucleotide comprising DNA and/or DNA analogs like for example, but not limited to PNA, LNA, HNA, phosphothioate DNA, methylphosphonate DNA;

at least one nucleic acid for hybridization on at least one sequence polymorphic nucleic acid section;
at least one nucleic acid for hybridization on at least one length polymorphic nucleic acid section;
at least one antibody specific for one selected from the group comprising a protein, a peptide, a tag, a dye, a saccharide, a hormon, a lipid, a particle or combinations thereof;
at least one nucleic acid further comprising a protein, peptide, tag, dye, saccharide, hormon, lipid, nucleic acid, mass label, particle or combinations thereof; and
a description for carrying out the method of the invention. Preferably, a said kit further comprises a description for interpretation of results obtained by means of embodiments described herein.

A disclosed kit comprises
at least one nucleic acid comprising at least one variant of a sequence polymorphism, at least one variant of a length polymorphism, or both, and
at least one oligonucleotide for amplifying at least one variant of a sequence polymorphism, at least one oligonucleotide for amplifying at least one variant of a length polymorphism, or both. A particular kit comprises further at least one nucleic acid for hybridization on at least one variant of a sequence polymorphism, at least one nucleic acid for hybridization on at least one variant of a length polymorphism, or both. Preferably, said oligonucleotides and/or nucleic acids comprises DNA and/or DNA analogs like for example, but not limited to PNA, LNA, HNA, phosphothioate DNA, methylphosphonate DNA.

Another disclosed kit comprises
at least one nucleic acid comprising at least one variant of a sequence polymorphism, at least one variant of a length polymorphism, or both, and
at least one nucleic acid for hybridization on at least one variant of a sequence polymorphism, at least one nucleic acid for hybridization on at least one variant of a length polymorphism, or both. A particular preferred kit comprises further at least one oligonucleotide for amplifying at least one variant of a sequence polymorphism, at least one oligonucleotide for amplifying at least one variant of a length polymorphism, or both. Preferably, said oligonucleotides and/or nucleic acids comprises DNA and/or DNA analogs like for example, but not limited to PNA, LNA, HNA, phosphothioate DNA, methylphosphonate DNA.

For the said disclosed kits, it is further preferred that the said at least one nucleic acid is one or more plasmids or is derived from one or more plasmids. Accordingly, preferably, the said oligonucleotides are able to amplify the at least one nucleic acid. Also preferably, the said nucleic acids are variants of a polymorphism, whereby each variant is a identifier.

Another disclosed kit comprises a set of particles,
whereby each part of the set comprises at least one particle of at least one size, and
whereby the one or more particles of different parts are of different sizes.

Another disclosed kit comprises a set of dyes,
whereby each part of the set comprises at least one dye of at least one color, and
whereby the one or more dyes of different parts are of different colors.

Another disclosed kit comprises a set of antibodies and corresponding epitopes,
whereby each part of the set comprises at least one epitope which is detected by at least one defined antibody, and
whereby the one or more epitopes of different parts are detected by different antibodies.

A disclosed kit is a kit for identification of a biological sample,
wherein the sample comprises genomic DNA differentially methylated at least at one position. Preferably such a kit is used in the field of methylation analysis.

In particular such a kit is used for detection of sample interchange, crosscontamination, or both.

In particular such a kit is used for identifying a sample in a pooled sample set.

In particular such a kit is used for detection of an amplification inhibition.

In particular such a kit is used for determining the rate of DNA conversion.

In particular such a kit is used for normalization of a sample, calibration of a sample, or both.

In particular such a kit is used for identification of a carry over contamination.

In particular such a kit is used assessing the success of a hybridization step.

Use of a method or a kit of the invention.

The methods disclosed herein are preferably used for the analysis of at least one DNA methylation status, at least one DNA methylation level, or of at least one DNA methylation pattern. Of course also combinations of the said are preferred.

Preferably, the embodiments described herein are used for at least one selected from the group comprising detection of sample interchange; detection of crosscontamination; identifying a sample in a pooled sample set; detection

of amplification inhibition; determining the rate of DNA conversion; normalization of a sample; calibration of a sample; identification of carry over contamination, assessing the success of a hybridization step or combinations thereof.

A method according of the invention may be used for at least one of the following with regard to a patient or individual diagnosing a condition, prognosing a condition, predicting a treatment response, diagnosing a predisposition for a condition, diagnosing a progression of a condition, grading a condition, staging a condition, classification of a condition, characterization of a condition, or combinations thereof, wherein the condition is a healthy condition or an adverse event, the adverse event comprises at least one category selected from the group comprising: undesired drug Interactions; cancer diseases, proliferative diseases or therewith associated diseases; CNS malfunctions; damage or disease; symptoms of aggression or behavioral disturbances; clinical; psychological and social consequences of brain damages; psychotic disturbances and personality disorders; dementia and/or associated syndromes; cardiovascular disease of the gastrointestinal tract; malfunction, damage or disease of the respiratory system; lesion, inflammation, infection, immunity and/or convalescence; malfunction, damage or disease of the body as an abnormality in the development process; malfunction, damage or disease of the skin, of the muscles, of the connective tissue or of the bones; endocrine and/or metabolic malfunction, damage or disease; and headaches or sexual malfunction.

Moreover, a method is disclosed which is preferably used for distinguishing cell types or tissue, or for investigating cell differentiation.

DEFINITIONS.

In particular aspects, the term "identifier" refers to, but is not limited to, a molecule which has unique chemical, physical or biological properties when compared with other molecules. It further refers to a molecule which is unambiguously assigned to a sample. According to the invention the term "identifier" refers to a molecule which is at least in parts a nucleic acid or a nucleic acid analog.

In particular aspects, the term "variants of polymorphism" or "polymorphism" refers to, but is not limited to, one of two or more alternate forms or alleles of nucleic acids or parts of nucleic acids that differ in nucleotide sequence or have variable numbers of nucleotides, in particular repeated nucleotides. According to the invention this refers preferably to the sequence (single nucleotide polymorphism, sequence polymorphism) or to the length (length polymorphism, deletion polymorphism).

In particular aspects, the term "methylation status" refers to, but is not limited to, the presence or absence of methylation of a single nucleotide in a single DNA molecule, said nucleotide being capable of being methylated.

In particular aspects, the term "methylation level" refers to, but is not limited to, the average methylation occupancy at a single nucleotide in a plurality of DNA molecules, said nucleotide being capable of being methylated.

In particular aspects, the term "methylation pattern" refers to, but is not limited to, the methylation status of a series of nucleotides located in cis on a single DNA molecule, said nucleotides being capable of being methylated.

In particular aspects, the term "remote sample" includes, but is not limited to, a sample having genomic DNA, wherein the sample is taken from a site (e.g., organ, tissue, body fluid, group of cells, cell, etc.) that is remote with respect to or that is distinct from the site of the cell, group of cells, tissue, or organ from which said genomic DNA originated.

In particular aspects, the term "crosscontamination" refers to, but is not limited to, an unintended addition of one or more components of a sample to another sample. Thereby the two samples are collected, processed, and/or analyzed at least in parts in parallel for example, but not limited to, as samples of the same sample set.

In particular aspects, the term "carry-over contamination" refers to, but is not limited to, an unintended addition of one or more components of a sample to another sample. Thereby the two samples are collected, processed, and/or analyzed at least in parts one after the other, for example, but not limited to, as samples of different sample sets.

In particular aspects, the term "experimental procedure" refers to, but is not limited to, any combination of chemical, biological or physical reactions.

In particular aspects, the term "differentials methylated" refers to, but is not limited to, a state of a base of a nucleotide, the nucleotide being part of a nucleic acid, preferably of a DNA, in particular of a genomic DNA. Thereby said state is characterized in that the corresponding base Is either methylated or unmethylated. Preferably the methylation or unmethylation is characteristic for an individual the corresponding sample is taken from, his state of health, the time at which the sample is taken, or combinations thereof.

EXAMPLES.

Example 1: Plant-specific fragments for the identification of sample contamination and sample confusion during DNA methylation analysis

The *Arabidopsis thaliana* cellulose syntethase gene (SEQ ID NO: 1) At1g55850 has been checked for sequence homologies with the human genome using a BLAST search (hftp:/Avww.ncbi.nlm.nih.gov/BLAST/Blast.cgi), Primers

were designed which allow the amplification of fragments of different lengths by combining primer 1 with primer 2; primer 1 with primer 3; primer 1 with primer 4; primer 1 with primer 5; primer 1 with primer 6; primer 1 with primer 7; primer 1 with primer 8 and primer 1 with primer 9. The Combinations are summarized in Table 2.

Table 2 Designed plant-specific primer

| Name | sequence | resulting fragment size |
|---|---|---|
| primer 1<br>SEQ ID NO: 2 | 5'ccgctgcttacttgtcttcc3' | |
| primer 2<br>SEQ ID NO: 3 | 5'acagcttagccacctcctca3' | 66 bp |
| primer 3<br>SEQ ID NO: 4 | 5'ctccggtattcgtcccagt3' | 122 bp |
| primer 4<br>SEQ ID NO: 5 | 5'agcatcccactgtgaaaacc3' | 167 bp |
| primer 5<br>SEQ ID NO: 6 | 5'atggttccatggtttcttcg3' | 196 bp |
| primer 6<br>SEQ ID NO: 7 | 5'ttcccttctcttccatctacca3' | 229 bp |
| primer 7<br>SEQ ID NO: 8 | 5'ttttctcttgataaatacaccaacg3' | 271 bp |
| primer 8<br>SEQ ID NO: 9 | 5'cattgctccagccttgaagt3' | 311 bp |
| primer 9<br>SEQ ID NO: 10 | 5'ccaagtttagtatgattttcccaca3' | 369 bp |

In addition, 8 different domains were designed, which contain a cytosine-free oligonucleotide binding site (underlined in Table 3), a recognition site for *Sca I* (underlined and bold in Table 3), a cutting site for *Swa I* (bold in Table 3) and a unique identification site (twofold underlined in Table 3). pGem has a unique cutting site for *Sca I*. In that way the successful cloning of a fragment containing a single *Sca I* site can be recognized by gel analysis after a *Sca I* treatment. The Swa *I* cutting site is stable during bisulfite treatment and can be used to destroy bisulfite PCR contaminations.

Table 3 Domain primer

| Name | Sequence | fragment size |
|---|---|---|
| domain-primer 1 SEQ ID NO: 11 | 5'GTGATGTGAGTTAATGATGGGccgctgcttacttgtcttcc3' | |
| domain-primer 2 SEQ ID NO: 12 | 5'CCCTAACCTTAACATCTTCCAAGTACTATTTAAATAACCATAC TATACCAAAATAATCACAGCTTAGCCACCTCCTCA3' | 66 bp |
| domain-primer 3 SEQ ID NO: 13 | 5'AACCTTACTTTACCATACTCTAGTACTATTTAAATAACCATAC TATACCAAAATAATCctccggtattcgtcccagt3' | 122 bp |
| domain-primer 4 SEQ ID NO: 14 | 5'ATATAATCCAATAACCCCCAAGTACTATTTAAATAACCATACT ATACCAAAATAATCagcatcccactgtgaaaacc3' | 167 bp |
| domain-primer 5 SEQ ID NO: 15 | 5'CACACCACCCAAAAACTAGTACTATTTAAATAACCATACTATA CCAAAATAATCatggttccatggtttcttcg3' | 196 bp |
| domain-primer 6 SEQ ID NO: 16 | 5'CACAATTACACATCCCAATAAACTTAGTACTATTTAAATAACC ATACTATACCAAAATAATCttcccttctcttccatctacca3' | 229 bp |
| domain-primer 7 SEQ ID NO: 17 | 5'CCCCACAATCAAACATACCATAGTACTATTTAAATAACCATAC TATACCAAAATAATCttttctcttgataaatacaccaacg3' | 271 bp |
| domain-primer 8 SEQ ID NO: 18 | 5'TACAATCCAACTTAAAACCACTCAGTACTATTTAAATAACCAT ACTATACCAAAATAATCcattgctccagccttgaagt3' | 311 bp |
| domain-primer 9 SEQ ID NO: 19 | 5'CTCAACTCAATAAACCTTTACACAGTACTATTTAAATAACCAT ACTATACCAAAATAATCccaagtttagtatgattttcccaca3' | 369 bp |

Plant-specific fragments will be generated by using the following primer combinations: domain-primer 1 + domain-primer 2; domain-primer 1 + domain-primer 3; domain-primer 1 + domain-primer 4; domain-primer 1 + domain-primer 5; domain-primer 1 + domain-primer 6; domain-primer 1 + domain-primer 7; domain-primer 1 + domain-primer 8 and domain-primer 1 + domain-primer 9. Polymerase Chain Reaction will be performed in a total volume of 25 µl containing 10 ng *Arabidopsis thaliana* DNA, 1 U Hotstart Taq polymerase (Qiagen), 10 pmol of each forward and reverse primer, 1x PCR buffer (Qiagen) and 0.2 mmol/l of each dNTP (MBI Fermentas). Cycling will be done using a Mastercycler (Eppendorf) under the following conditions: 15 min at 95°C and 15 cycles at 95°C for 1 min, 60°C for 45 s and 72°C for 1:30 min and 30 cycles at 95°C for 1 min, 72°C for 1:30 min. 10 µl of the PCR mix will be loaded on a 2.5% agarose gel and fragments of the expected size will be cut out of the gel, purified using a gel-extraction kit (Qiagen) and used for TA-cloning (Promega). Successful cloning in a pGem vector will be verified by Sca I treatment and sequencing. In that way 8 different identifier plasmids will be created containing a sequence polymorphism and a length polymorphism.

2000 copies of two different plasmids will be added to each sample and deparafination, DNA extraction and bisulfite treatment will be performed together with the analyzed specimen. After bisulfite treatment bisulfit-independent primer will be used to analyze the identity of the samples by using the following primers: CF-primer 1 (SEQ ID NO: 20) GTGATGTGAGTTAATGATGGG and CF-primer 2 (SEQ ID NO: 21) AACCATACTATACCAAAATAATC. Polymerase Chain Reaction will be performed in a total volume of 25 µl containing 5 µl of bisulfite eluate, 1 U Hotstart Taq polymerase (Qiagen), 10 pmol of each forward and reverse primer, 1x PCR buffer (Qiagen) and 0.2 mmol/l of each dNTP (MBI Fermentas). Cycling will be done using a Mastercycler (Eppendorf) under the following conditions: 15 min at 95°C and 45 cycles at 95°C for 1 min, 55°C for 45 s and 72°C for 1:30 min. 5 µl PCR mix will be loaded on a 2.5% agarose gel and sample identity will be analyzed by fragment size (e.g. 66 bp and 311 bp) after gel electrophoresis. Sample contamination with PCR products can be identified by additional bands at the agarose gel (e.g. 66 bp; 122 bp and 311 bp). After bisulfite treatment the methylation of the sample will be analyzed in a duplex reaction using the following primers
APC-F (SEQ ID NO: 22) Cy5-GGAGAGAGAAGTAGTTGTGTAATT and
APC-R (SEQ ID NO: 23) ACTACACCAATACAACCACATATC;
ID-primer 1 (SEQ ID NO: 24) Cy5-tgtTttgattTtgTggTtga and
ID-primer 2 (SEQ ID NO: 25) ccaacAcAttAAAaActctcc.

Each capital letter represents a converted C; only complete converted DNA will be amplified. Polymerase Chain Reaction will be performed in a total volume of 25 µl containing 5 µl of bisulfite eluate, 1 U Hotstart Taq polymerase (Qiagen), 5 pmol of each primer, 1x PCR buffer (Qiagen) and 0.3 mmol/l of each dNTP (MBI Fermentas). Cycling will be done using a Mastercycler (Eppendorf) under the following conditions: 15 min at 95°C and 45 cycles at 95°C for 1 min, 55°C for 45 s and 72°C for 1:30 min.

Bisulfite conversion and sample identification will be analyzed at a microarray using probes 1 to 8:

probe 1 (SEQ ID NO: 26) NH2-TGGAAGATGTTAAGGTTAGGG;
probe 2 (SEQ ID NO: 27) NH2-AGAGTATGGTAAAGTAAGGTT;
probe 3 (SEQ ID NO: 28) NH2-TGGGGGTTATTGGATTATAT;
probe 4 (SEQ ID NO: 29) NH2-AGTTTTTGGGTGGTGTG;
probe 5 (SEQ ID NO: 30) NH2-AAGTTTATTGGGATGTGTAATTGTG;
probe 6 (SEQ ID NO: 31) NH2-ATGGTATGTTTGATTGTGGGG;
probe 7 (SEQ ID NO: 32) NH2-GAGTGGTTTTAAGTTGGATTGTA;
probe 8 (SEQ ID NO: 33) NH2-GAGTGGTTTTAAGTTGGATTGTA;
probe 9 (SEQ ID NO: 34) NH2-GTGTAAAGGTTTATTGAGTTGAG.

Hybridizations are carried out according to standard procedures. Figure 4 shows schematic drawings of such a hybridization. In A the probe orientation at the array is shown. B and C show hybridizations of samples. In B two plasmids are used which are generated by domain-primer 1 + domain-primer 2 and domain-primer 1 + domain-primer 3. In C two plasmids are used which are generated by domain-primer 1 + domain-primer 2 and domain-primer 1 + domain-primer 4. Probes 1 to 8 hybridize with only one molecular identification plasmid. Probe 9 hybridizes with all plasmids.

In Figure 5 the detection of a contamination is visualized. Figure 5 shows a schematic drawing of a hybridized microarray detecting a contamination of the sample. A combination of two plasmids was used.

The system is able to generate 28 different combinations of two different plasmids. In that way allowing both identification of sample and contamination.

*Arabidopsis thaliana* cellulose syntethase gene (SEQ ID NO: 1) At1g55850

**At1g55850**

5'-ctcttatccctctcaccttctcacttggcaccgttgcagagagagaaccaaacatggtaaacaaagacgaccggattagaccggttcatg
aagccgacggtgaaccgctttttgagactaggagaagaaccggtagagtgattgcgtaccggtttttctcagcctcggttttcgtgtgtatctgttt
gatttggttctacagaattggtgagattggtgataaccgtaccgttttagatcgattaatctggtttgttatgtttattgtggagatttggttcggtttatatt
gggtagtcacacaatcttcccggtggaatccggtttggcgatttcccttctccgatagactctctcggagatacggaagcgaccttccgaggct
cgacgtcttcgtttgcacggcggatccggtgattgagccgccgttgttggtggtaaacacagtcttatctgtgacggctcttgactacccaccgg
agaaactcgccgtttatctctcagatgacggtggttctgagctgacgttctatgctctcacggaggcagctgagtttgctaaaacttgggttccctt
ctgcaagaagttcaacgttgagccaacatctcccgctgcttacttgtcttccaaggcaaactgtcttgattctgcggctgaggaggtggctaagc
tgtatagagaaatggcggcgaggattgaaacggcggcgagactgggacgaataccggaggaggcgcgggtgaagtacggtgacgggt
tttcacagtgggatgctgacgctactcgaagaaaccatggaaccattcttcaagttttggtagatggaagagaagggaatacaatagcaata
ccaacgttggtgtatttatcaagagaaaagagacctcaacatcatcataacttcaaggctggagcaatgaacgcattgctgagggtttcttcga
aaattacttgtgggaaaatcatactaaacttggactgtgatatgtacgcaaacaactcaaagtcaacacgcgacgcgctctgcatcctcctcg

atgagaaagagggaaaagagattgctttcgtgcagtttccgcagtgttttgacaatgttacaagaaatgatttgtatggaagcatgatgcgagt
aggaattgatgtggaatttcttggattggatggaaatggtggtccgttatacattggaactggatgctttcacagaagagatgtgatctgtggaag
aaagtatggagaggaagaagaagaagaagaatctgagagaattcacgaaaatttagagcctgagatgattaaggctctcgcgagctgca
cttatgaggaaaacactcaatggggaaaggagatgggtgtgaaatatggttgcccggtagaggatgtaataactggtttgacgattcagtgtc
gcggatggaaatcagcctacctgaacccggaaaagcaagcatttctcggggtagcgccgaccaatttgcatcaaatgctagtgcagcaga
ggagatggtcagagggagactttcagattatgctttcgaagtatagtccggtttggtatggaaaaggaaagatcagtttaggactgatacttggt
tactgttgctattgtctttgggctccatcttcactacctgtgctcatttactctgttttgacttctctctgtctcttcaaaggcattcctctgtttccaaaggtct
cgagctcgtggtttattccgtttggatacgtcactgttgcagctaccgcatatagcctagccgagttcttgtggtgcggagggacgttccgtggat
ggtggaacgagcaaaggatgtggctttatagaagaacaagctcgtttcttttcggatttatggacacgattaagaagctacttggagtttctgag
tctgcgtttgtgatcacagcaaaagtagcagaagaagaagcagcagagagatacaaggaagaggtaatggagtttggagtggagtctcc
catgtttctcgtcctcggaacactcggtatgctcaatctcttctgcttcgccgcagcggttgcgagacttgtttccggagacggtggagatttgaaa
acaatggggatgcaatttgtgataacaggagtactagttgtcataaactggcctctgtataaaggtatgttgttgaggcaagacaaaggaaag
atgccaatgagcgttacagttaaatcagttgttttagctttatctgcctgtacctgtttagcgtttttgtaagattgattaacaacagtcaaaaaagta
atcaaaataatgaccagcagttataatatgtaattttct-3'

Example 2: Multiplex DNA methylation analysis by usage of domain primers with molecular identifiers.

Two samples are mixed with cytosine-free primers (200 pmol each) containing a molecular identification domain. Each sample is mixed with a different set of primers.
primer set 1:

set1F (SEQ ID NO: 35) 5'TGATGGGAGAGTGAGTAGGA3';
set1R (SEQ ID NO: 36) 5'TGGAAGATGTTAAGGTTAGGGTCACTTCTAACTCTACCACTTA3';

primer set 2:

set2F (SEQ ID NO: 37) 5'TGATGGGAGAGTGAGTAGGA3';
set2R (SEQ ID NO: 38) 5'AGAGTATGGTAAAGTAAGGTTTCACTTCTAACTCTACCACTTA3':

After bisulfite treatment with the EpiTect kit (Qiagen) samples are amplified as follows: Polymerase Chain Reaction is performed in a total volume of 25 μl containing 5 μl of bisulfite eluate, 1 U Hotstart Taq polymerase (Qiagen), 1x PCR buffer (Qiagen) and 0.2 mmol/l each dNTP (MBI Fermentas). Cycling is done using a Mastercycler (Eppendorf) under the following conditions: 15 min at 95°C and 45 cycles at 95°C for 1 min, 55°C for 45 s and 72°C for 1:30 min.

Amplification mixtures are pooled and simultaneously hybridized on a microarray with the following capture probes and the following detection probes:

capture probe 1 (SEQ ID NO: 39) NH2-CCCTAACCTTAACATCTTCCA;
capture probe 2 (SEQ ID NO: 40) NH2-AACCTTACTTTACCATACTCT;

(capture probe 1 and capture probe 2 are able to hybridize onto the underlined part of the primers set1R and set2R, respectively.)
detection probe 1 (SEQ ID NO: 41)
Cy5-TAGAAAGTTTACGGTATTTTAAT (detection in case of methylation);
detection probe 2 (SEQ ID NO: 42)
Cy3-TAGAAAGTTTATGGTATTTTAAT (detection in case of unmethylation).

The methylation of each sample can be calculated by the Cy5/Cy3 signal ratio at the specific capture spot. In that way multiple samples can be analyzed in parallel.

Example 3: Performing a methylation detection workflow using a molecular identification plasmid as hybridization control

Two molecular identification plasmids (named 23 and 195) were generated. Therefore the two oligonucleotide pairs 23sens (SEQ ID NO: 43) AGTACTTGTATTTGAATTGTTTTTTTTGA / 23anti (SEQ ID NO: 44) CAAAAAAAACAATTCAAATACAAGTACTA and 195sens (SEQ ID NO: 45) AGTACTGTATTTGGTTGGAGTGGGGA / 195anti (SEQ ID NO: 46) CCCCACTCCAACCAAATACAGTACTA were cloned into pGem®-T vector, respectively. The said two oligonucleotide pairs are specific for oligonucleotides of an array tube (see below). Plasmids were isolated from transformed bacteria using a QIAprep Spin Miniprep kit (Qiagen). 500 ng plasmid DNA was linearized at 37°C in 20 μl water containing 5 U of the restriction enzyme Bfu I and 1x NEB 4 buffer (both New England Biolabs). Reaction was stopped at 80°C for 20 min. Clones were purified using the PCR-Puriflcation kit (Qiagen). 100 fg plasmid DNA in 5 ng/μl poly-A solution (Roche) in a total volume of 27 μl were bisulfite treated using the EpiTect kit (Qiagen). Bisulfite DNA was amplified using the following primers MIDBis-1F (SEQ ID NO: 47) TGTGGAATTGTGAGtGGATA and MIDBis-1R (SEQ ID NO: 48) aCATaCTCCCaaCCaCCATa which are specific for sequences of the pGem-T vector. Therefore the following conditions were applied: total volume 25 μl; 1x QIA mPCR master mix MM (Qiagen) 0.2 μmol/l of each primer; Activation 95°C for 15 min; Denaturation at 95°C for 30 sec; Annealing at 61°C for 45 sec; Extension at 72°C for 1 min. Steps 2 -4 were repeated 40 times. Finally an extension at 72° for 10 min was performed. PCRs were analyzed in an agarose gel and subsequently used for hybridization. Figure 6 shows amplificates of the linearized bisulfite treated plasmid 23 and the linearized bisulfite treated plasmid 195, respectively.

Hybridization was performed in array tubes provided by Clondiag Chip Technologies GmbH. These tubes contain a low density microarray with capture probes for methylation specific hybridization. The array tubes were prehybridized with 200 μl hybridization buffer (2xSSPE; 0.005% Triton) at 30°C for 5 min. Each PCR product was diluted 1 to 100 in hybridization buffer and denatured for 10 min at 99°C before hybridization. Hybridizations were performed for 1h 10 min in a total volume of 100 μl at 35°C. The array tubes were washed three times with washing buffer (2xSSC) at 20°C for 5 min. Subsequently, the array tubes were blocked with blocking solution (Pierce) and incubated with conjugate solution (Poly-Horseradish Peroxidase Streptavidin Conjugate in 2xSSPE/0.005% Triton buffer) for 30 min at 30°C. Afterwards, the array tubes were washed three times with washing buffer at 20°C for 5 min. 100 μl True Blue substrate were added (Pierce) into each tube before the tubes were scanned in an ATS scanner (Clondiag Chip Technologies). The results are shown in Figure 7. The amplificates of each of the two molecular identification plasmids hybridizes specifically two oligonucleotides of the array tube (dark spots marked by quadrats). Dark spots at the corner of the image show control spots necessary for scanning the array tubes. Light grey spots represent unspecific hybridization.

Example 4: Controlling the accurateness of a real time PCR analysis

16 different samples are measured in triplicates in a real time PCR.
(A) The samples are numbered from 1 to 16. Four different identifiers are used to encode the samples. Thereby every 4$^{th}$ sample encloses the same identifier:

| sample number: | spiked identifier |
|---|---|
| 1, 5, 9, 13 | identifier W |
| 2, 6, 10, 14 | identifier X |
| 3, 7, 11, 15 | identifier Y |
| 4, 8, 12, 16 | identifier Z |

The samples with spiked identifiers are subjected to real time PCR analysis, wherein a polymorphism of a sample DNA intrinsic site as well as the identity of the identifier is determined. According to the real time PCR analysis the 1st, 2nd, 3rd, .... 16th samples comprise the following identifier:

1st replicate

| sample | identified identifier |
|---|---|
| 2nd 5th, 9th, 13th | identifier W |
| 1st, 6th, 10th, 14th | identifier X |
| 3rd, 7th, 11th, 15th | identifier Y |
| 4th, 8th, 12th, 16th | identifier Z |

2nd replicate

| sample | identified identifier |
|---|---|
| 2nd, 5th, 9th, 13th | identifier W |
| 1st, 6th, 10th, 14th | identifier X |
| 3rd, 7th, 11th, 15th | identifier Y |
| 4th, 8th, 12th, 16th | identifier Z |

3rd replicate

| sample | identified identifier |
|---|---|
| 2nd, 5th, 9th, 13th | identifier W |
| 1st, 6th, 10th, 14th | identifier X |
| 3rd, 7th, 11th, 15th, 16th | identifier Y |
| 4th, 8th, 12th, 16th | identifier Z |

From this it becomes obviousness that two errors occurred because of unexpected changes in the order of identified identifiers. A sample interchange between sample 1 and 2 took place before the samples were divided up into triplicates. In addition, it is obvious that sample 16 was crosscontaminated with either sample 3, 7, 11, 15 or combinations thereof during the 3rd replicate run.

(B) Alternatively, the samples (numbered 1-16) were arranged in a certain order in a microtiter plate. Three different identifiers are used to encode the samples. Thereby every 3rd sample of the set encloses the same identifier

| sample number: | spiked identifier |
|---|---|
| 1, 4, 7,10,13,16 | identifier X |
| 2, 5, 8, 11, 14 | identifier Y |
| 3, 6, 9, 12, 15 | identifier Z |

Figure 8 (a) gives a schematic overview over the so generated identifier pattern.

The samples with spiked identifiers are subjected to real time PCR analysis, wherein a polymorphism of a sample DNA intrinsic site as well as the identity of the identifier is determined. Figure 8 (b) shows a schematic overview of the results of the determination of the identifier identity, each result assigned to the position of the sample in the microtiter plate. Because the identifier pattern is in an unexpected order, an error is easily detected. It is obvious that samples 5 and 6 are switched in the first replicate (positions B2 and B3), and samples 12 and 13 in the third replicate (positions D11 and E9). Even if the identification of an individual sample is not 100% save (e.g. a switch of sample 1 and 3 would not be detected in this example) almost every process will be recognized.

## Claims

**1.** A method of identifying at least one biological sample in the field of methylation analysis, comprising steps (a) and (b) in the indicated order:

(a) providing a sample set of at least two biological sample, wherein at least one sample comprises genomic DNA differentially methylated at least at one position;
(b) applying at least one identifier for each sample, wherein the applied at least one identifier does not interfere with subsequent analysis; and wherein the applied at least one identifier is a nucleic acid forming no stable secondary structure and comprises at least one cytosine-free, or cytosine-free and guanine-free oligonucleotide binding site; contacting the DNA of each sample with bisulfite;
(c) subjecting each sample to a detection or quantification reaction specific for the binding site(s) of the one or more applied identifiers; and
(d) subjecting each sample to methylation analysis, whereby methylation is detected or quantified

wherein step (c) is carried out prior, simultaneously with or subsequent to step (d).

**2.** A method of claim 1, wherein the detecting or quantifying the applied one or more identifiers for each sample and the methylation analysis of each sample are realized simultaneously.

**3.** A method of claim 1, wherein the identifier is at least in parts part of a larger molecule; part of an endogeneous molecule of the respective sample; part of an exogenous molecule added to the respective sample; a section of genomic DNA or total genomic DNA derived from a plant; a section of genomic DNA or total genomic DNA derived from a bacteria; a section of genomic DNA or total genomic DNA derived from a non-vertebrate; a section of genomic DNA or total genomic DNA derived from a vertebrate; a short tandem repeat; a variant of a deletion polymorphism; a variant of a single nucleotide polymorphism; a variant of a length polymorphism; an artificial nucleic acid; a circular nucleic acid; a circular DNA; a plasmid; a polynucleotide; an oligonucleotide; a PNA; a PNA-oligomer; a PNA-polymer; an artificial methylation; or combinations thereof.

**4.** A method of claim 1, wherein different identifiers are assigned to different sets of identifiers according to their respective biological, chemical, or physical properties.

**5.** A method of claim 4, **characterized in that** a representative of each of at least two sets of identifiers is comprised in a plasmid.

**6.** A method of claim 5, wherein the first set of identifiers comprises a sequence polymorphism and wherein the second set of identifiers comprises a length polymorphism.

**7.** A method of claim I, wherein the identifier;
comprises at least one oligonucleotide binding site covering cytosine positions converted by bisulfite;
is **characterized by** a similar base composition as the analyzed genomic DNA of the provided sample;
is a polymorphic sequence of about 5, about 10, about 15, about 20, about 25, about 30, about 35, about 40, about 50, about 75, about 100, or about 200 nucleotides;
has a content of cytosin-nucleotides and guanosin-nucleotides of about 15%, about 20%, about 30%, about 40%, about 50%, about 60%, about 70%, or about 80%; or
combinations thereof.

**8.** A method of claim 1, wherein the identifier;
is **characterized by** a similar base composition as the analyzed genomic DNA of the provided sample;
is a polymorphic sequence of about 5, about 10, about 15, about 20, about 25, about 30, about 35, about 40, about 50, about 75, about 100, or about 200 nucleotides;
has a content of cytosin-nucleotides and guanosin-nucleotides of about 15%, about 20%, about 30%, about 40%, about 50%, about 60%, about 70%, or about 80%; or
combinations thereof.

**9.** A method of claim 1, wherein the identifier is a variant of a sequence polymorphism and additionally
comprises about 1, about 10, about 20, about 30, about 40, about 50, about 60, about 70, about 80, about 90, or about 100 variable nucleotide sites.

**10.** A method of claim 1, wherein the identifier is a variant of a sequence polymorphism and additionally
comprises about 5, about 10, about 15, about 20, or about 25 variable nucleotide sites;
has a content of cytosin-nucleotides and guanosin-nucleotides of about 20%, about 30%, about 40%, about 50%, about 60%, about 70%, or about 80%; or both.

**11.** A method of claim 1, wherein the identifier is a variant of a length polymorphism and additionally
has a length difference of about 10, about 100, about 200, about 300, about 400, about 500, about 600, about 700, about 800, about 900, or about 1000 nucleotides compared to other used nucleic acid length polymorphic identifiers;
is of about 10, about 100, about 500, about 1.000, about 1.500, about 2.000, about 2.500, about 3.000, about 3.500, about 4.000, about 4.500, about 5.000, about 5.500, about 6.000, about 6.500, about 7.000, about 7.500, about 8.000, about 8.500, about 9.000, about 9.500, or about 10.000 nucleotides in length; is derived from non-human DNA; or combinations thereof.

**12.** A method of claim 1, wherein the identifier is a variant of a length polymorphism and additionally
is either of about 5, about 25, about 50, about 75, about 100, about 125, about 150, about 175, about200, about225, about250, about275, about300,about325, about350, about375, about 400, about 425, about 450, about 475, or

about 500 nucleotides in length;
is derived from non-human DNA;
has a content of cytosin-nucleotides and guanosin-nucleotides of about 20%, about 30%, about 40%, about 50%, about 60%, about 70%, or about 80%; or
combinations thereof.

**13.** A method of claim 1, wherein the identifier comprises a tag selected from the group comprising dye, fluorescent dye, chemiluminescent dye, Cy5, Cy3, TAMRA, FAM, tag, epitope tag, peptide, polypeptide, protein, saccharide, harmon, lipid, mass label, particle, gold particle, silver particle, platin particle, paraffin embedded code or combinations thereof.

**14.** A method of claim 1, wherein the detection or quantification reaction is carried out by one or more means selected from the group comprising: antibody, western blot analysis, chromatography, immunoassay, ELISA immunoassay, radioimmunoassay, FPLC, HPLC, UV light, light, spectrometer, MALDI-TOF, nucleic acid, DNA, PNA, oligonucleotide, PNA oligomer, amplification method, PCR method, isothermal amplification method, NASBA method, LCR method, methylation specific amplification method, MSP (Methylation Specific PCR) method, nested MSP method, HeavyMethyl™ method, detection method, methylation specific detection method, bisulfite sequencing method, detection by means of DNA-arrays, detection by means of oligonucleotide microarrays, detection by means of CpG-island-microarrays, detection by means of restriction enzymes, simultaneous methylation specific amplification and detection method, COBRA method, real-time PCR, HeavyMethyl™ real time PCR method, MSP Methylight™ method, Methylight™ method, Methylight™ AlgoTM method, OM method, Headloop Methylight™ method, HeavyMethyl™ Methylight™ method, HeavyMethyl™ ScorpionTM method, MSP ScorpionTM method, Headloop ScorpionTM method, methylation sensitive primer extension, and Ms-SNuPE (Methylation-sensitive Single Nucleotide Primer Extension) method.

**15.** A method of claim 1, wherein the detection or quantification reaction comprises a nucleic acid, DNA, PNA, oligonucleotide, or PNA oligomer which
is at least of about 10, about 15, about 20, about 25, about 30, about 35, about 40, about 50, about 60, about 70, about 80, about 90, about 100, about 150 or about 200 nucleotides in length,
has a content of cytosin-nucleotides and guanosin-nucleotides of about 15%, about 20%, about 30%, about 40%, about 50%, about 60%, about 70%, about 80%, or about 85%;
has a melting temperature of about 37°C, about 45°C, about 50°C, about 55°C, about 60°C, about 65°C, about 70°C, about 75°C, about 80°C, about 85°C, about 90°C, about 95°C, or about 99°C; or
combinations thereof.

**16.** A method of claim 1, wherein the detection or quantification reaction comprises a oligonucleotide which
is at least of about 5, about 10, about 15, about 20, about 25, about 30, about 35, about 40, about 50, about 60, about 70, about 80, or about 90 nucleotides in length;
has a content of cytosin-nucleotides and guanosin-nucleotides of about 5%, of about 10%,
about 20%, about 30%, about 40%, about 50%, about 60%, about 70%, about 80%, about 90% or about 95%;
has a melting temperature of about 37°C, about 45°C, about 50°C, about 55°C, about 60°C, about 65°C, about 70°C, about 75°C, about 80°C, about 85°C, about 90°C, about 95°C, or about 99°C; or
combinations thereof.

**17.** A method of claim 1, wherein the detection or quantification reaction comprises a oligonucleotide which
is at least of about 16, about 20, about 25, about 30, about 35, or about 40 nucleotides in length;
has a content of cytosin-nucleotides and guanosin-nucleotides of about 20%, about 30%, about 40%, about 50%, about 60%, about 70%, or about 80%; and
has a melting temperature of about 50°C, about 53°C, about 56°C, about 59°C, or about 62°C.

**18.** A method of claim 1, wherein the detection or quantification reaction comprises an oligonucleotide which comprises a gene-specific priming sequence and a sequence which hybridizes on a variant of a sequence polymorphism.

**19.** A method of claim 1, wherein the detection or quantification reaction comprises an oligonucleotide which comprises two domains,
wherein one domain comprises a target-specific priming sequence of about 10, about 15, about 20, about 25, about 30, about 35, or about 40 nucleotides, has a content of cytosin-nucleotides and guanosin-nucleotides of about 20%, about 30%, about 40%, about 50%, about 60%, about 70%, or about 80%, and has a domain melting temperature

of about 50°C, about 52C, about 54°C, about 56°C, about 58°C, about 60°C, or about 62°; and herein the other domain comprises a unique sequence of about 5, about 10, about 15, about 20, about 25, about 30, about 35, about 40, about 45, or about 50 nucleotides, is free of cytosines, guanin, or both, and has a content of cytosin-nucleotides and guanosin-nucleotides of about 20%, about 30%, about 40%, about 50%, about 60%, about 70%, or about 80%.

**20.** A method of claim 1, wherein methylation analysis comprises at least one selected from the group comprising detection of methylation status, detection of methylation level, detection of methylation pattern, detection of methylation pattern level, amplification method, PCR method, isothermal amplification method, NASBA method, LCR method, methylation specific amplification method, MSP (Methylation Specific PCR) method, nested MSP method, HeavyMethyl™ method, detection method, methylation specific detection method, bisulfite sequencing method, detection by means of DNA-arrays, detection by means of oligonucleotide microarrays, detection by means of CpG-island-microarrays, detection by means of restriction enzymes, simultaneous methylation specific amplification and detection method, COBRA method, real-time PCR, HeavyMethyl™ real time PCR method, MSP Methylight™ method, Methylight™ method, Methylight™ AlgoTM method, OM method, Headloop Methylight™ method, HeavyMethyl™ Methylight™ method, HeavyMethyl™ ScorpionTM method, MSP ScorpionTM method, Headloop ScorpionTM method, methylation sensitive primer extension, and Ms-SNuPE (Methylation-sensitive Single Nucleotide Primer Extension) method.

**21.** A method suitable for the detection of sample interchange, crosscontamination, or both in the field of methylation analysis, comprising the steps of the method of claim 1 and further comprising the steps of
deducing the presence of a sample interchange or of a crosscontamination from the presence of at least one identifier not applied to a sample or the absence of a sample interchange or a crosscontamination from the absence of at least one identifier not applied to a sample.

**22.** A method of claim 21, wherein the step of deducing the presence or absence of a sample interchange, of a crosscontamination, or both further comprises
deducing the extent of a crosscontamination for a single sample from the absolute or relative amount of at least one identifier present in said single sample.

**23.** A method suitable for identifying a sample in a pooled sample set in the field of methylation analysis, comprising the steps of the method of claim 1 and further comprising the steps of: identifying a sample in the pooled sample set by detecting the respective applied at least one identifier.

**24.** A method suitable for the detection of an amplification inhibition in the field of methylation analysis, comprising the steps of the method of claim 1 and further comprising the steps of deducing a presence, absence or partial amplification inhibition from the presence, absence, or amount of the product of a identifier specific amplification reaction.

**25.** A method suitable for the normalization, calibration, or both in the field of methylation analysis, comprising the steps of the method of claim 1 and further comprising the steps of normalizing at least one sample, calibrating an experimental procedure, or both according to the detected or quantified one or more identifiers compared to the added total amount of one or more identifiers.

**26.** A method suitable for the identification of a carry over contamination in the field of methylation analysis, comprising the steps of the method of claim 1 and further comprising the steps of
deducing the presence of a sample carry over contamination from the presence of at least one identifier not applied for said sample, or deducing the absence of a sample contamination from the absence of identifiers not applied for said sample.

**27.** A method suitable for assessing the success of a hybridization step in the field of methylation analysis, wherein the detection or quantification reaction comprises a hybrization step, and the method additionally comprising the steps of the method of claim 1 and further comprising the steps of
assessing the success of a hybridization step wherein (a) the presence of a signal derived for the applied at least one identifier indicates the presence of a successful hybrization step, and wherein (b) the absence of signal derived for the applied at least one identifier indicates the presence of an unsuccessful hybrization step.

**28.** A method of claims 21, 23 to 27, further comprising contacting the DNA of each sample and the applied at least one identifier with a reagent or enzyme which differentiates between a methylated or an unmethylated position.

**29.** A method suitable for controlling the correctness of a process or method, comprising the steps of the method of claim 1, wherein

(a) a sample set of at least 2, 3,4, 100,200, 400, or 800 biological samples is provided;
(b) wherein the applied identifiers generate an identification pattern across the sample; and wherein
(c) at least one identifier is applied to a sample of the set; and the method additionally comprising

deducing the correctness of said process or method from the signals of the detected or quantified identifiers of the samples.

**30.** A method of claim 29, wherein only a single identifier is applied to each sample of the sample set.

**31.** A method of claim 29, wherein deducing the correctness of said process or method from the signals of the detected or quantified identifiers of the samples, comprises
determining the presence of an error-free process or method, wherein the said signals generate a pattern that is corresponds to the identification pattern as initially generated by applying the identifiers to the samples; or
determining the absence of an error-free process or method, wherein the said signals generate a pattern that does not correspond to the identification pattern as initially generated by applying the identifiers to the samples.

**32.** A method of claim 31, wherein the process or method is a high-throughput process or method.

**33.** Use of a method according to one of the preceding claims for at least one selected from the group comprising detection of sample interchange; detection of crosscontamination; identifying a sample in a pooled sample set; detection of amplification inhibition; determining the rate of DNA conversion; normalization of a sample; calibration of a sample; identification of carry over contamination; controlling the success of a hybridization step or combinations thereof.

## Patentansprüche

**1.** Verfahren zur Identifizierung mindestens einer biologischen Probe in dem Bereich der Methylierungsanalyse, welches die Schritte a) und b) in der angegebenen Reihenfolge umfasst:

a) Bereitstellen eines Probensets von mindestens zwei biologischen Proben, wobei mindestens eine Probe genomische DNA umfasst, welche an mindestens einer Position unterschiedlich methyliert ist;
b) Verwenden mindestens eines Identifizierers für jede Probe, wobei der mindestens eine verwendete Identifizierer mit der nachfolgenden Analyse nicht interferiert; und wobei mindestens ein verwendeter Identifizierer eine Nukleinsäure ist, welche keine stabile Sekundärstruktur ausbildet und mindestens eine Cytosin-freie, oder Cytosin-freie und Guanin-freie Oligonukleotidbindungsstelle umfasst; Inkontaktbringen der DNA jeder Probe mit Bisulfit;
c) Unterziehen jeder Probe einer Detektions- oder Quantifizierungs-Reaktion, welche spezifisch für die Bindungsstelle(n) des einen oder der mehreren verwendeten Identifizierern ist; und
d) Unterziehen jeder Probe einer Methylierungsanalyse, wobei die Methylierung detektiert oder quantifiziert wird,

wobei Schritt c) vor, gleichzeitig mit oder nach Schritt d) ausgeführt wird.

**2.** Verfahren gemäß Anspruch 1, wobei Detektieren oder Quantifizieren des verwendeten einen oder mehreren Identifizierern für jede Probe und die Methylierungsanalyse jeder Probe gleichzeitig realisiert werden.

**3.** Verfahren gemäß Anspruch 1, wobei der Identifizierer mindestenes teilweise Teil eines größeren Moleküls ; Teil von einem endogenen Molekül der jeweiligen Probe; Teil eines exogenen Moleküls, welches zu der jeweiligen Probe hinzugefügt wurde; ein Abschnitt genomischer DNA oder die gesamte genomische DNA, welche von einer Pflanze abstammt; ein Abschnitt genomischer DNA oder die gesamte genomische DNA, welche von einem Bakterium abstammt; ein Abschnitt genomischer DNA oder die gesamte genomische DNA, welche von einem Nicht-Vertebraten abstammt; ein Abschnitt genomischer DNA oder die gesamte genomische DNA, welche von einem Vertebraten abstammt; ein kurzes Tandemrepeat; eine Variante eines Deletionspolymorphismus; eine Variante eines Einzel-Nukleotid-Polymorphismus; eine Variante eines Längenpolymorphismus; eine künstliche Nukleinsäure; eine zirkuläre Nukleinsäure; eine zirkuläre DNA; ein Plasmid; ein Polynukleotid, ein Oligonukleotid, eine PNA, ein PNA-Oligomer; ein PNA-Polymer; eine künstliche Methylierung; oder Kombinationen dieser ist.

4. Verfahren gemäß 1, wobei die verschiedenen Identifizierer zu verschiedenen Sets von Identifizierern gemäß ihrer jeweiligen biologischen, chemischen oder physikalischen Eigenschaften zugeordnet sind.

5. Verfahren gemäß Anspruch 4, dadurch charakterisiert, dass ein Vertreter von jedem von mindestens zwei Sets von Identifizierern in einem Plasmid umfasst ist.

6. Verfahren gemäß Anspruch 5, wobei das erste Set von Identifizierern einen Sequenzpolymorphismus umfasst und wobei das zweite Set von Identifizierern einen Längenpolymorphismus umfasst.

7. Verfahren gemäß Anspruch 1, wobei der Identifizerer
mindestens eine Oligonukleotidbindungsstelle umfasst, die durch Bisulfit umgewandelte Cytosin-Positionen abdeckt;
durch eine ähnliche Basenzusammensetzung wie die analysierte genomische DNA der bereitgestellten Probe charakterisiert ist;
eine polymorphe Sequenz von ungefähr 5, ungefähr 10, ungefähr 15, ungefähr 20, ungefähr 25, ungefähr 30, ungefähr 35, ungefähr 40, ungefähr 50, ungefähr 75, ungefähr 100 oder ungefähr 200 Nukleotiden ist;
einen Gehalt von Cytosin-Nukleotiden und Guanosin-Nukleotiden von ungefähr 15%, von ungefähr 20%, von ungefähr 30%, von ungefähr 40%, von ungefähr 50%, von ungefähr 60%, von ungefähr 70% oder von ungefähr 80% hat, oder Kombinationen dieser.

8. Verfahren gemäß Anspruch 1, wobei der Identifizierer
durch eine ähnliche Basenzusammensetzung wie die analysierte genomische DNA der bereitgestellten Probe charakterisiert ist;
eine polymorphe Sequenz von ungefähr 5, ungefähr 10, ungefähr 15, ungefähr 20, ungefähr 25, ungefähr 30, ungefähr 35, ungefähr 40, ungefähr 50, ungefähr 75, ungefähr 100 oder ungefähr 200 Nukleotiden ist;
einen Gehalt von Cytosin-Nukleotiden und Guanosin-Nukleotiden von ungefähr 15%, von ungefähr 20%, von ungefähr 30%, von ungefähr 40%, von ungefähr 50%, von ungefähr 60%, von ungefähr 70% oder von ungefähr 80% hat, oder Kombinationen dieser.

9. Das Verfahren gemäß Anspruch 1, wobei der Identifizierer eine Variante eines Sequenzpolymorphismus ist und zusätzlich ungefähr 1, ungefähr 10, ungefähr 20, ungefähr 30, ungefähr 40, ungefähr 50, ungefähr 60, ungefähr 70, ungefähr 80, ungefähr 90 oder ungefähr 100 variable Nukleotidstellen umfasst.

10. Das Verfahren gemäß Anspruch 1, wobei der Identifizierer eine Variante eines Sequenzpolymorphismus ist und zusätzlich ungefähr 5, ungefähr 10, ungefähr 15, ungefähr 20 oder ungefähr 25 variable Nukleotidstellen umfasst; einen Gehalt von Cytosin-Nukleotiden und Guanosin-Nukleotiden von ungefähr 20%, von ungefähr 30%, von ungefähr 40%, von ungefähr 50%, von ungefähr 60%, von ungefähr 70% oder von ungefähr 80% hat, oder beides.

11. Das Verfahren gemäß Anspruch 1, wobei der Identifizierer
eine Variante eines Längenpolymorphismus ist und zusätzlich einen Längenunterschied von ungefähr 10, ungefähr 100, ungefähr 200, ungefähr 300, ungefähr 400, ungefähr 500, ungefähr 600, ungefähr 700, ungefähr 800, ungefähr 900, oder ungefähr 1000 Nukleotiden im Vergleich zu anderen, verwendeten Nukleinsäuren-Längenpolymorphismus-Identifizierern aufweist;
eine Länge von ungefähr 10, ungefähr 100, ungefähr 500, ungefähr 1000, ungefähr 1500, ungefähr 2000, ungefähr 2500, ungefähr 3000, ungefähr3500, ungefähr 4000, ungefähr 4500, ungefähr 5000, ungefähr 5500, ungefähr 6000, ungefähr 6500, ungefähr 7000, ungefähr 7500, ungefähr 8000, ungefähr8500, ungefähr 9000, ungefähr 9500 oder ungefähr 10000 Nukleotiden aufweist;
von nicht-humaner DNA abstammt; oder Kombinationen dieser.

12. Das Verfahren gemäß Anspruch 1, wobei der Identifizierer
eine Variante eines Längenpolymorphismus ist und zusätzlich eine Länge von einem von ungefähr 5, ungefähr 25, ungefähr 50, ungefähr 75, ungefähr 100, ungefähr 125, ungefähr 150, ungefähr 175, ungefähr 200, ungefähr 225, ungefähr 250, ungefähr 275, ungefähr 300, ungefähr 325, ungefähr 350, ungefähr 375, ungefähr 400, ungefähr 425, ungefähr 450, ungefähr 475 oder ungefähr 500 Nukleotiden aufweist;
von nicht-humaner DNA abstammt;
einen Gehalt von Cytosin-Nukleotiden und Guanosin-Nukleotiden von ungefähr 20%, von ungefähr 30%, von ungefähr 40%, von ungefähr 50%, von ungefähr 60%, von ungefähr 70% oder von ungefähr 80% hat, oder Kombinationen dieser.

**13.** Verfahren gemäß Anspruch 1, wobei der Identifizierer ein Tag umfasst, ausgewählt aus der Gruppe umfassend Farbstoff, Fluoreszenzfarbstoff, Chemilumineszenzfarbstoff, Cy5, Cy3, TAMRA, FAM, Tag, Epitop-Tag, Peptid, Polypeptid, Protein, Saccharid, Hormon, Lipid, Massenmarker, Partikel, Goldpartikel, Silberpartikel, Platinpartikel, Paraffineingebetteter Code oder Kombinationen dieser.

**14.** Das Verfahren gemäß Anspruch 1, wobei die Detektions-oder Quantifizierungsreaktion durch ein oder mehrere Mittel ausgeführt wird, welche ausgewählt sind aus der Gruppe umfassend: Antikörper, Western Blot Analyse, Chromatographie, Immunoassay, ELISA immunoassay, Radioimmunoassay, FPLC; HPLC, UV-Licht, Licht, Spektrometer, MALDI-TOF, Nukleinsäure, DNA, PNA, Oligonukleotid, PNA-Oligomer, Amplifikationsverfahren, PCR Verfahren, isotherme Amplifikationsverfahren, NASBA Verfahren, LCR Verfahren, Methylierungsspezifische Amplifikationsverfahren, MSP (Methylierungsspezifische PCR) Verfahren, Nested MSP Verfahren, HeavyMethyl™ Verfahren, Detektionsverfahren, Methylierungsspezifische Detektionsverfahren, Bisulfit-Sequenzierungsverfahren, Detektion durch Mittel von DNA-Arrays, Detektion durch Mittel von Oligonukleotid Microarrays, Detektion durch Mittel von CpG-Insel-Microarrays, Detektion durch Mittel von Restriktionsenzymen, simultanes Methylierungsspezifische Amplifikation- und Detektionsverfahren, COBRA Verfahren, Real-time PCR, HeavyMethyl™ Real-time PCR Verfahren, MSP Methylihgt™ Verfahren, Methylight™ Verfahren, Methylight™ Algo™ Verfahren, OM Verfahren, Headloop Methylight™ Verfahren, HeavyMethyl™ Methylihgt™ Verfahren, HeavyMethyl™ Scorpion™ Verfahren, MSP Scorpion™ Verfahren, Headloop Scorpion™ Verfahren, Methylierungssensitive Primer Verlängerung und Ms-SNuPE (Methylierungssensitive Einzelnukleotid Primer VerlängerungsVerfahren).

**15.** Verfahren gemäß Anspruch 1, wobei die Detektions- oder Quantifizierungsreaktion eine Nukleinsäure, DNA, PNA, Oligonukleotide oder ein PNA-Oligomer umfasst, welches eine Länge von mindestens ungefähr 5, ungefähr 10, ungefähr 15, ungefähr 20, ungefähr 25, ungefähr 30, ungefähr 35, ungefähr 40, ungefähr 50, ungefähr 60, ungefähr 70, ungefähr 80, ungefähr 90, ungefähr 100, ungefähr 150 oder ungefähr 200 Nukleotide hat, einen Gehalt von Cytosin-Nukleotiden und Guanosin-Nukleotiden von ungefähr 15%, ungefähr 20%, von ungefähr 30%, von ungefähr 40%, von ungefähr 50%, von ungefähr 60%, von ungefähr 70%, ungefähr 80% oder ungefähr 85% aufweist;
eine Schmelztemperatur von ungefähr 37°C, ungefähr 45°C, ungefähr 50°C, ungefähr 55°C, ungefähr 60°C, ungefähr 65°C, ungefähr 70°C, ungefähr 75°C, ungefähr 80°C, ungefähr 85°C, ungefähr 90°C, ungefähr 95°C oder ungefähr 99°C hat; oder Kombinationen dieser.

**16.** Verfahren gemäß Anspruch 1, wobei die Detektions- oder Quantifizierungsreaktion ein Oligonukleotid umfasst, welches mindestens eine Länge von ungefähr 5, ungefähr 10, ungefähr 15, ungefähr 20, ungefähr 25, ungefähr 30, ungefähr 35, ungefähr 40, ungefähr 50, ungefähr 60, ungefähr 70, ungefähr 80 oder ungefähr 90 Nukleotiden aufweist,
einen Gehalt von Cytosin-Nukleotiden und Guanosin-Nukleotiden von ungefähr 5%, ungefähr 10%, von ungefähr 20%, von ungefähr 30%, von ungefähr 40%, von ungefähr 50%, von ungefähr 60%, von ungefähr 70%, ungefähr 80%, ungefähr 90 oder ungefähr 95% aufweist;
eine Schmelztemperatur von ungefähr 37°C, ungefähr 45°C, ungefähr 50°C, ungefähr 55°C, ungefähr 60°C, ungefähr 65°C, ungefähr 70°C, ungefähr 75°C, ungefähr 80°C, ungefähr 85°C, ungefähr 90°C, ungefähr 95°C oder ungefähr 99°C aufweist;
oder Kombinationen dieser.

**17.** Verfahren gemäß Anspruch 1, wobei die Detektions- oder Quantifizierungsreaktion ein Oligonukleotid umfasst, welches mindestens eine Länge von ungefähr 16, ungefähr 20, ungefähr 25, ungefähr 30, ungefähr 35 oder ungefähr 40 Nukleotiden aufweist;
einen Gehalt von Cytosin-Nukleotiden und Guanosin-Nukleotiden von ungefähr 20%, von ungefähr 30%, von ungefähr 40%, von ungefähr 50%, von ungefähr 60%, von ungefähr 70% oder ungefähr 80% aufweist; und
eine Schmelztemperatur von ungefähr 50°C, ungefähr 53°C, ungefähr 56°C, ungefähr 59°C oder ungefähr 62°C aufweist.

**18.** Verfahren gemäß Anspruch 1, wobei die Detektions- oder Quantifizierungsreaktion ein Oligonukleotid umfasst, welches eine genspezifische Priming-Sequenz und eine Sequenz, die mit einer Variante eines Sequenzpolymorphismus hybridisiert, umfasst.

**19.** Verfahren gemäß Anspruch 1, wobei die Detektions- oder Quantifizierungsreaktion ein Oligonukleotid umfasst,

welches zwei Domänen umfasst, wobei eine Domäne eine zielspezifische Priming-Sequenz von ungefähr 10, ungefähr 15, ungefähr 20, ungefähr 25, ungefähr 30, ungefähr 35 oder ungefähr 40 Nukleotiden umfasst,
einen Gehalt von Cytosin-Nukleotiden und Guanosin-Nukleotiden von ungefähr 20%, von ungefähr 30%, von ungefähr 40%, von ungefähr 50%, von ungefähr 60%, von ungefähr 70% oder ungefähr 80% aufweist;
eine Domänen-Schmelztemperatur von ungefähr 50°C, ungefähr 52°C, ungefähr 54°C, ungefähr 56°C, ungefähr 58°C, ungefähr 60°C oder ungefähr 62°C aufweist und wobei die andere Domäne eine einzigartige Sequenz von ungefähr 5, ungefähr 10, ungefähr 15, ungefähr 20, ungefähr 25, ungefähr 30, ungefähr 35, ungefähr 40, ungefähr 45 oder ungefähr 50 Nukleotiden umfasst, frei von Cytosin, Guanin oder beiden ist und
einen Gehalt von Cytosin-Nukleotiden und Guanosin-Nukleotiden von ungefähr 20%, von ungefähr 30%, von ungefähr 40%, von ungefähr 50%, von ungefähr 60%, von ungefähr 70% oder ungefähr 80% aufweist.

**20.** Verfahren gemäß Anspruch 1, wobei die Methylierungsanalyse mindestens eine Analyse umfasst, welche aus der Gruppe ausgewählt ist, umfassend: Detektion des Methylierungsstatus, Detektion des Methylierungslevels, Detektion des Methylierungsmusters, Detektion des Methylierungsmusterniveaus, Amplifikationsverfahren, PCR Verfahren, isotherme Amplifikationsverfahren, NASBA Verfahren, LCR Verfahren, Methylierungsspezifische Amplifikationsverfahren, MSP (Methylierungsspezifische PCR) Verfahren, Nested MSP Verfahren, HeavyMethyl™ Verfahren, Detektionsverfahren, Methylierungsspezifische Detektionsverfahren, Bisulfit-Sequenzierungsverfahren, Detektion durch Mittel von DNA-Arrays, Detektion durch Mittel von Oligonukleotid Microarrays, Detektion durch Mittel von CpG-Insel-Microarrays, Detektion durch Mittel von Restriktionsenzymen, simultanes Methylierungsspezifische Amplifikation- und Detektionsverfahren, COBRA Verfahren, Real-time PCR, HeavyMethyl™ Real-time PCR Verfahren, MSP Methylihgt™ Verfahren, Methylight™ Verfahren, Methylight™ Algo™ Verfahren, OM Verfahren, Headloop Methylight™ Verfahren, HeavyMethyl™ Methylihgt™ Verfahren, HeavyMethyl™ Scorpion™ Verfahren, MSP Scorpion™ Verfahren, Headloop Scorpion™ Verfahren, Methylierungssensitive Primer Verlängerung und Ms-SNuPE (Methylierungssensitive Einzelnukleotid Primer VerlängerungsVerfahren).

**21.** Verfahren, welches zur Detektion von Austausch, Kreuzkontamination oder beiden im Bereich der Methylierungsanalyse geeignet ist, das die Schritte des Verfahrens gemäß Anspruch 1 umfasst, und weiterhin die Schritte umfasst:

Ableiten der Anwesenheit eines Probenaustauschs oder einer Kreuzkontamination von der Anwesenheit mindestens eines Identifizierers, welcher nicht für eine Probe verwendet wurde oder Ableiten der Abwesenheit eines Probenaustauschs oder einer Kreuzkontamination von der Abwesenheit von mindestens einem Identifizierer, welcher nicht für eine Probe verwendet wurde.

**22.** Verfahren gemäß Anspruch 21, wobei der Schritt des Ableitens der Anwesenheit oder Abwesenheit eines Probenaustauschs, einer Kreuzkontamination oder beiden weiterhin umfasst:

Ableiten des Ausmaßes einer Kreuzkontamination für eine einzelne Probe von der absoluten oder relativen Menge von mindestens einem Identifizierer, der in besagter einzelner Probe anwesend ist.

**23.** Verfahren geeignet zur Identifizierung einer Probe in einem gepoolten Probenset im Bereich der Methylierungsanalyse, das die Schritte des Verfahrens gemäß Anspruch 1 umfasst und weiterhin die Schritte umfasst:

Identifizieren einer Probe in dem gepoolten Probenset durch Detektieren des jeweiligen verwendeten mindestens einen Identifizierers.

**24.** Verfahren geeignet zur Detektion einer Amplifikationshemmung im Bereich der Methylierungsanalyse, das die Schritte des Verfahrens gemäß Anspruch 1 umfasst und weiterhin die Schritte umfasst
Ableiten der Anwesenheit, Abwesenheit oder partiellen Amplifikationshemmung von der Anwesenheit, Abwesenheit oder der Produktmenge einer Identifizierer-spezifischen Amplifikationsreaktion.

**25.** Verfahren geeignet zur Normalisierung, Kalibrierung oder beidem im Bereich der Methylierungsanalyse, das die Schritte des Verfahrens gemäß Anspruch 1 umfasst und weiterhin die Schritte umfasst
Normalisieren von mindestens einer Probe, Kalibrieren eines experimentellen Verfahrens oder beides, gemäß des detektierten oder quantifizierten einen oder mehreren Identifizierers verglichen mit der hinzugefügten Gesamtmenge eines oder mehrerer Identifizierer.

**26.** Verfahren geeignet zur Detektion einer Übertragungskontamination im Bereich der Methylierungsanalyse, das die Schritte des Verfahrens gemäß Anspruch 1 umfasst und weiterhin die Schritte umfasst

Ableiten der Anwesenheit einer Probenübertragunskontamination von der Anwesenheit von mindestens einem Identifizierer, welcher nicht für besagte Probe verwendet wurde, oder Ableiten der Abwesenheit einer Probenkontamination von der Abwesenheit von einem Identifizierer, welcher nicht für besagte Probe verwendet wurde.

**27.** Verfahren geeignet zur Untersuchung des Erfolgs eines Hybridisierungsschrittes im Bereich der Methylierungsanalyse, wobei die Detektions- oder Quantifizierungsreaktion einen Hybridisierungsschritt umfasst, und das Verfahren zusätzlich die Schritte des Verfahrens gemäß Anspruch 1 umfasst und weiterhin die Schritte umfasst Untersuchen des Erfolgs eines Hyblidisierungsschrittes, wobei a) die Anwesenheit von einem Signal, welches von dem verwendeten mindestens einen Identifizierer abstammt, die Anwesenheit eines erfolgreichen Hybridisierungsschlittes anzeigt, und wobei b) die Abwesenheit eines Signals, welches von dem mindestens einen Identifizierer abstammt, die Anwesenheit eines nichterfolgreichen Hyblidisierungsschrittes anzeigt.

**28.** Verfahren gemäß der Ansprüche 21, 23-27, weiterhin umfassend das Inkontaktbringen der DNA jeder Probe und dem verwendeten mindestens einen Identifizierer mit einem Reagenz oder Enzym, welches zwischen einer methylierten oder einer unmethylierten Position unterscheidet.

**29.** Verfahren geeignet zur Kontrolle der Richtigkeit eines Prozesses oder Verfahrens, das die Schritte des Verfahrens gemäß Anspruch 1 umfasst, wobei

a) ein Probenset von mindestens 2, 3, 4, 100, 200, 400 oder 800 biologischen Proben bereitgestellt wird;
b) wobei die verwendeten Identifizierer ein Identifikationsmuster über die Probe erzeugen; und wobei
c) mindestens ein Identifizierer für eine Probe aus dem Set verwendet wird; und das Verfahren weiterhin umfassend

Ableiten der Richtigkeit von besagtem Prozess oder Verfahren von den Signalen der detektierten oder quantifizierten Identifizierern der Proben.

**30.** Verfahren gemäß Anspruch 29, wobei nur ein einzelner Identifizierer für jede Probe des Probensets verwendet wird.

**31.** Verfahren gemäß Anspruch 29, wobei das Ableiten der Richtigkeit von besagtem Prozess oder Verfahren von den Signalen der detektierten oder quantifizierten Identifizierern der Proben umfasst:

Bestimmen der Anwesenheit eines fehlerfreien Prozesses oder Verfahrens, wobei besagte Signale ein Muster erzeugen, welches mit dem Identifizierungsmuster wie initial erzeugt durch Verwenden der Identifizierer für die Proben, korrespondiert; oder
Bestimmen der Abwesenheit eines fehlerfreien Prozesses oder Verfahrens, wobei besagte Signale ein Muster erzeugen, welches nicht korrespondiert mit dem Identifizierungsmuster wie initial erzeugt durch Verwenden der Identifizierer für die Proben.

**32.** Verfahren gemäß Anspruch 31, wobei der Prozess oder das Verfahren ein Hochdurchsatzprozess oder -verfahren ist.

**33.** Verwendung eines Verfahrens gemäß einem der vorhergehenden Ansprüche für mindestens eines ausgewählt aus der Gruppe umfassend: Detektion von Probenaustausch; Detektion von Kreuzkontamination; Identifizieren einer Probe in einem gepoolten Probenset; Detektion von Amplifikationshemmung: Bestimmen der DNA-Konversionsrate; Normalisierung einer Probe; Kalibrieren einer Probe; Identifizieren einer Übertragungskontamination; Kontrollieren des Erfolgs eines Hybridisierungsschrittes oder Kombinationen dieser.

## Revendications

**1.** Procédé pour l'identification d'au moins un échantillon biologique dans le domaine de l'analyse de méthylation, comprenant les étapes (a) et (b) dans l'ordre indiqué:

(a) fourniture d'un ensemble d'échantillons d'au moins deux échantillons biologiques, tandis qu'au moins un échantillon comprend un ADN génomique méthylé de manière différentielle au moins sur une position;
(b) application d'au moins un identifiant pour chaque échantillon, tandis que le au moins un identifiant appliqué n'interfère pas avec l'analyse subséquente; et tandis que le au moins un identifiant appliqué est un acide nucléique ne formant aucune structure secondaire stable et comprend au moins un site de liaison oligonucléotidique exempt de cytosine, ou exempt de cytosine et exempt de guanine; mise en contact de l'ADN de chaque échantillon avec du bisulfite;
(c) action consistant à soumettre chaque échantillon à une réaction de détection ou de quantification spécifique pour le(s) site(s) de liaison du ou des identifiants appliqués; et
(d) action consistant à soumettre chaque échantillon à une analyse de méthylation, la méthylation étant ainsi détectée ou quantifiée

tandis que l'étape (c) est mise en oeuvre avant, en même temps que ou après l'étape (d).

2. Procédé selon la revendication 1, dans lequel la détection ou la quantification des un ou plusieurs identifiants appliqués pour chaque échantillon et l'analyse de méthylation de chaque échantillon sont réalisées simultanément.

3. Procédé selon la revendication 1, dans lequel l'identifiant constitue au moins pour parties une portion d'une molécule plus grosse; une portion d'une molécule endogène de l'échantillon respectif; une portion d'une molécule exogène ajoutée à l'échantillon respectif; une section d'ADN génomique ou un ADN génomique total dérivé d'une plante; une section d'ADN génomique ou un ADN génomique total dérivé d'une bactérie; une section d'ADN génomique ou un ADN génomique total dérivé d'un non-vertébré; une section d'ADN génomique ou un ADN génomique total dérivé d'un vertébré; une courte répétition en tandem; une variante d'un polymorphisme de délétion; une variante d'un polymorphisme de nucléotide unique; une variante d'un polymorphisme de longueur; un acide nucléique artificiel; un acide nucléique circulaire; un ADN circulaire; un plasmide; un polynucléotide; un oligonucléotide; un PNA; un oligomère de PNA; un polymère de PNA; une méthylation artificielle; ou leurs combinaisons.

4. Procédé selon la revendication 1, dans lequel différents identifiants sont affectés à différents lots d'identifiants selon leurs propriétés biologiques, chimiques ou physiques respectives.

5. Procédé selon la revendication 4, **caractérisé en ce qu'**un représentant de chacun des au moins deux ensembles d'identifiants est compris dans un plasmide.

6. Procédé selon la revendication 5, dans lequel le premier ensemble d'identifiants comprend un polymorphisme de séquences et dans lequel le deuxième ensemble d'identifiants comprend un polymorphisme de longueur.

7. Procédé selon la revendication 1, dans lequel l'identifiant comprend au moins un site de liaison d'oligonucléotides recouvrant des positions cytosine converties par du bisulfite;
est **caractérisé par** une composition de base similaire à l'ADN génomique analysé de l'échantillon procuré;
est une séquence polymorphique d'environ 5, environ 10, environ 15, environ 20, environ 25, environ 30, environ 35, environ 40, environ 50, environ 75, environ 100, ou environ 200 nucléotides;
a une teneur en nucléotides de cytosine et en nucléotides de guanosine d'environ 15%, environ 20%, environ 30%, environ 40%, environ 50%, environ 60%, environ 70%, ou environ 80%, ou leurs combinaisons.

8. Procédé selon la revendication 1, dans lequel l'identifiant est **caractérisé par** une composition de base similaire à l'ADN génomique analysé de l'échantillon procuré;
est une séquence polymorphique d'environ 5, environ 10, environ 15, environ 20, environ 25, environ 30, environ 35, environ 40, environ 50, environ 75, environ 100, ou environ 200 nucléotides;
a une teneur en nucléotides de cytosine et en nucléotides de guanosine d'environ 15%, environ 20%, environ 30%, environ 40%, environ 50%, environ 60%, environ 70%, ou environ 80%, ou
leurs combinaisons.

9. Procédé selon la revendication 1, dans lequel l'identifiant est une variante d'un polymorphisme de séquences et comprend en outre environ 1, environ 10, environ 20, environ 30, environ 40, environ 50, environ 60, environ 70, environ 80, environ 90, ou environ 100 sites nucléotidiques variables.

10. Procédé selon la revendication 1, dans lequel l'identifiant est une variante d'un polymorphisme de séquences et comprend en outre environ 5, environ 10, environ 15, environ 20, ou environ 25 sites nucléotidiques variables;
a une teneur en nucléotides de cytosine et en nucléotides de guanosine d'environ 20%, environ 30%, environ 40%, environ 50%, environ 60%, environ 70%, ou environ 80%; ou les deux.

11. Procédé selon la revendication 1, dans lequel l'identifiant est une variante d'un polymorphisme de longueur et en outre a une différence de longueur d'environ 10, environ 100, environ 200, environ 300, environ 400, environ 500, environ

600, environ 700, environ 800, environ 900, ou environ 1000 nucléotides par comparaison avec d'autres identifiants polymorphes de la longueur des acides nucléiques utilisés;
est d'environ 10, environ 100, environ 500, environ 1000, environ 1500, environ 2000, environ 2500, environ 3000, environ 3500, environ 4000, environ 4500, environ 5000, environ 5500, environ 6000, environ 6500, environ 7000, environ 7500, environ 8000, environ 8500, environ 9000, environ 9500, ou environ 10000 nucléotides de long; est dérivé d'ADN non-humain; ou des combinaisons de ceux-ci.

**12.** Procédé selon la revendication 1, dans lequel l'identifiant est une variante d'un polymorphisme de longueur et en outre a une longueur d'environ 5, environ 25, environ 50, environ 75, environ 100, environ 125, environ 150, environ 175, environ 200, environ 225, environ 250, environ 275, environ 300, environ 325, environ 350, environ 375, environ 400, environ 425, environ 450, environ 475, ou environ 500 nucléotides;
est dérivé d'ADN non-humain;
a une teneur en nucléotides de cytosine et en nucléotides de guanosine d'environ 20%, environ 30%, environ 40%, environ 50%, environ 60%, environ 70%, ou environ 80%, ou leurs combinaisons.

**13.** Procédé selon la revendication 1, dans lequel l'identifiant comprend un marqueur choisi dans le groupe comprenant colorant, colorant fluorescent, colorant chimioluminescent, Cy5, Cy3, TAMRA, FAM, marqueur, marqueur d'épitope, peptide, polypeptide, protéine, saccharide, harmon, lipide, étiquette de masse, particule, particule d'or, particule d'argent, particule de platine, code noyé dans la paraffine, ou leurs combinaisons.

**14.** Procédé selon la revendication 1, dans lequel la réaction de détection ou de quantification est mise en oeuvre par un ou plusieurs moyens choisis dans le groupe comprenant: anticorps, analyse par Western blot, chromatographie, dosage immunologique, dosage immunologique par ELISA, dosage radio-immunologique, FPLC, HPLC, lumière UV, lumière, spectromètre, MALDI-TOF, acide nucléique, ADN, PNA, oligonucléotide, oligomère de PNA, procédé d'amplification, procédé d'ACP, procédé d'amplification isotherme, procédé NASBA, procédé LCR, procédé d'amplification spécifique pour la méthylation, procédé MSP (ACP spécifique pour la méthylation), procédé MSP niché, procédé HeavyMethyl™, procédé de détection, procédé de détection spécifique pour la méthylation, procédé de séquençage au bisulfite, détection au moyen de micro-arrangements d'ADN, détection au moyen de micro-arrangements d'oligonucléotides, détection au moyen de micro-arrangements d'îlots de CpG, détection au moyen d'enzymes de restriction, procédé simultané d'amplification et de détection spécifique pour la méthylation, procédé COBRA, ACP en temps réel, procédé d'ACP en temps réel HeavyMethyl™, procédé MSP MethyLight™, procédé MethyLight™, procédé MethyLight™ Algo™, procédé OM, procédé MethyLight™ à boucle en tête, procédé HeavyMethyl™ MethyLight™, procédé HeavyMethyl™ Scorpion™, procédé MSP Scorpion™, procédé Scorpion™ à boucle en tête, extension d'amorce sensible à la méthylation, et procédé Ms-SNuPE (extension d'amorce nucléotidique unique sensible à la méthylation).

**15.** Procédé selon la revendication 1, dans lequel la réaction de détection ou de quantification comprend un acide nucléique, ADN, PNA, oligonucléotide, ou oligomère de PNA qui
a une longueur d'au moins environ 10, environ 15, environ 20, environ 25, environ 30, environ 35, environ 40, environ 50, environ 60, environ 70, environ 80, environ 90, environ 100, environ 150 ou environ 2000 nucléotides,
a une teneur en nucléotides de cytosine et en nucléotides de guanosine d'environ 15%, environ 20%, environ 30%, environ 40%, environ 50%, environ 60%, environ 70%, environ 80%, ou environ 85%;
a une température de fusion d'environ 37°C, environ 45°C, environ 50°C, environ 55°C, environ 60°C, environ 65°C, environ 70°C, environ 75°C, environ 80°C, environ 85°C, environ 90°C, environ 95°C ou environ 99°C; ou leurs combinaisons.

**16.** Procédé selon la revendication 1, dans lequel la réaction de détection ou de quantification comprend un oligonucléotide qui
a une longueur d'au moins environ 5, environ 10, environ 15, environ 20, environ 25, environ 30, environ 35, environ 40, environ 50, environ 60, environ 70, environ 80, ou environ 90 nucléotides;
a une teneur en nucléotides de cytosine et en nucléotides de guanosine d'environ 5%, environ 10%, environ 20%, environ 30%, environ 40%, environ 50%, environ 60%, environ 70%, environ 80%, environ 90%, ou environ 95%;
a une température de fusion d'environ 37°C, environ 45°C, environ 50°C, environ 55°C, environ 60°C, environ 65°C, environ 70°C, environ 75°C, environ 80°C, environ 85°C, environ 90°C, environ 95°C, ou environ 99°C; ou leurs combinaisons.

**17.** Procédé selon la revendication 1, dans lequel la réaction de détection ou de quantification comprend un oligonucléotide qui

a une longueur d'au moins environ 16, environ 20, environ 25, environ 30, environ 35, ou environ 40 nucléotides;
a une teneur en nucléotides de cytosine et en nucléotides de guanosine d'environ 20%, environ 30%, environ 40%, environ 50%, environ 60%, environ 70%, ou environ 80%; et
a une température de fusion d'environ 50°C, environ 53°C, environ 56°C, environ 59°C, ou environ 62°C.

**18.** Procédé selon la revendication 1, dans lequel la réaction de détection ou de quantification comprend un oligonucléotide qui comprend une séquence d'amorçage spécifique pour un gène et une séquence qui hybride sur une variante d'un polymorphisme de séquences.

**19.** Procédé selon la revendication 1, dans lequel la réaction de détection ou de quantification comprend un oligonucléotide qui comprend deux domaines, tandis qu'un domaine comprend une séquence d'amorçage spécifique pour une cible ayant environ 10, environ 15, environ 20, environ 25, environ 30, environ 35, ou environ 40 nucléotides, a une teneur en nucléotides de cytosine et en nucléotides de guanosine d'environ 20%, environ 30%, environ 40%, environ 50%, environ 60%, environ 70%, ou environ 80%; et a une température de fusion de domaine d'environ 50°C, environ 52°C, environ 54°C, environ 56°C, environ 58°C, environ 60°C, ou environ 62°C; et là l'autre domaine comprend une séquence particulière d'environ 5, environ 10, environ 15, environ 20, environ 25, environ 30, environ 35, environ 40, environ 45, ou environ 50 nucléotides, est exempt de cytosines, de guanine, ou des deux, et a une teneur en nucléotides de cytosine et en nucléotides de guanosine d'environ 20%, environ 30%, environ 40%, environ 50%, environ 60%, environ 70%, ou environ 80%.

**20.** Procédé selon la revendication 1, dans lequel l'analyse de méthylation comprend au moins l'un choisi dans le groupe comprenant détection de l'état de méthylation, détection du niveau de méthylation, détection du schéma de méthylation, détection du niveau du schéma de méthylation, procédé d'amplification, procédé par ACP, procédé d'amplification isotherme, procédé NASBA, procédé LCR, procédé d'amplification spécifique pour la méthylation, procédé MSP (ACP spécifique pour la méthylation), procédé MSP niché, procédé HeavyMethyl™, procédé de détection, procédé de détection spécifique pour la méthylation, procédé de séquençage au bisulfite, détection au moyen de micro-arrangements d'ADN, détection au moyen de micro-arrangements d'oligonucléotides, détection au moyen de micro-arrangements d'îlots de CpG, détection au moyen d'enzymes de restriction, procédé simultané d'amplification et de détection spécifique pour la méthylation, procédé COBRA, ACP en temps réel, procédé d'ACP en temps réel HeavyMethyl™, procédé MSP MethyLight™, procédé MethyLight™, procédé MethyLight™ Algo™, procédé OM, procédé MethyLight™ à boucle en tête, procédé HeavyMethyl™ MethyLight™, procédé HeavyMethyl™ Scorpion™, procédé MSP Scorpion™, procédé Scorpion™ à boucle en tête, extension d'amorce sensible à la méthylation, et procédé Ms-SNuPE (extension d'amorce nucléotidique unique sensible à la méthylation).

**21.** Procédé approprié pour la détection d'une échange d'échantillons, d'une contamination croisée, ou des deux dans le domaine de l'analyse de méthylation, comprenant les étapes du procédé selon la revendication 1 et comprenant en outre les étapes de
déduction de la présence d'un échange d'échantillons ou d'une contamination croisée à partir de la présence d'au moins un identifiant non appliqué à un échantillon ou de l'absence d'échange d'échantillons ou d'une contamination croisée à partir de l'absence d'au moins un identifiant non appliqué à un échantillon.

**22.** Procédé selon la revendication 21, dans lequel l'étape de déduction de la présence ou de l'absence d'un échange d'échantillons, d'une contamination croisée, ou des deux comprend
la déduction de l'ampleur d'une contamination croisée sur la base d'un échantillon unique, à partir de la quantité absolue ou relative d'au moins un identifiant présent dans ledit échantillon unique.

**23.** Procédé approprié pour l'identification d'un échantillon dans un mélange d'échantillons dans le champ de l'analyse de méthylation, comprenant les étapes du procédé selon la revendication 1 et comprenant en outre les étapes d'identification d'un échantillon dans l'ensemble d'échantillons mis en pool, par détection du au moins un identifiant respectif appliqué.

**24.** Procédé approprié pour la détection d'une inhibition d'amplification dans le domaine de l'analyse de méthylation, comprenant les étapes du procédé selon la revendication 1 et comprenant en outre les étapes de déduction d'une présence, absence ou inhibition d'amplification partielle à partir de la présence, l'absence, ou la quantité du produit d'une réaction d'amplification spécifique pour l'identifiant.

**25.** Procédé approprié pour la normalisation, le calibrage, ou les deux dans le domaine de l'analyse de méthylation, comprenant les étapes du procédé selon la revendication 1 et comprenant en outre les étapes de normalisation

d'au moins un échantillon, de calibrage d'un mode opératoire expérimental, ou les deux en fonction du ou des identifiants détectés ou quantifiés par comparaison avec la quantité totale ajoutée d'un ou plusieurs identifiants.

**26.** Procédé approprié pour l'identification d'une contamination par transfert dans le domaine de l'analyse de méthylation, comprenant les étapes du procédé selon la revendication 1 et comprenant en outre les étapes de déduction de la présence d'une contamination d'échantillon par transfert à partir de la présence d'au moins un identifiant non appliqué pour ledit échantillon, ou déduction de l'absence d'une contamination d'échantillon à partir de l'absence d'identifiants non appliquée pour ledit échantillon.

**27.** Procédé approprié pour l'évaluation du succès d'une étape d'hybridation dans le domaine de l'analyse de méthylation, dans lequel la réaction de détection ou de quantification comprend une étape d'hybridation, et le procédé comprenant en sus les étapes du procédé selon la revendication 1 et comprenant en outre les étapes de l'évaluation du succès d'une étape d'hybridation dans laquelle (a) la présence d'un signal dérivé pour le au moins un identifiant appliqué indique la présence d'une étape d'hybridation réussie, et dans laquelle (b) l'absence du signal dérivé pour le au moins un identifiant appliqué indique la présence d'une étape d'hybridation infructueuse.

**28.** Procédé selon les revendications 21, 23 à 27, comprenant en outre la mise en contact de l'ADN de chaque échantillon et le au moins un identifiant appliqué avec un réactif ou une enzyme qui distingue entre une position méthylée et une position non-méthylée.

**29.** Procédé approprié pour la maîtrise du bon déroulement d'un processus ou procédé, comprenant les étapes du procédé selon la revendication 1, dans lequel

(a) un ensemble d'échantillons d'au moins 2, 3, 4, 100, 200, 400 ou 800 échantillons biologiques est procuré;
(b) tandis que les identifiants appliqués produisent un schéma d'identification sur tout l'échantillon; et tandis que
(c) au moins un identifiant est appliqué à un échantillon de l'ensemble; et le procédé comprenant en outre

la déduction du bon déroulement dudit processus ou procédé à partir des signaux des identifiants des échantillons détectés ou quantifiés.

**30.** Procédé selon la revendication 29, dans lequel seulement un identifiant unique est appliqué à chaque échantillon de l'ensemble d'échantillons.

**31.** Procédé selon la revendication 29, dans lequel la détection du bon déroulement dudit processus ou procédé à partir des signaux des identifiants détectés ou quantifiés des échantillons, comprend
la détermination de la présence d'un processus ou procédé exempt d'erreurs, tandis que lesdits signaux produisent un schéma qui correspond au schéma d'identification tel que produit initialement par l'application des identifiants aux échantillons; ou
la détermination de l'absence d'un processus ou procédé exempt d'erreurs, tandis que lesdits signaux produisent un schéma qui ne correspond pas au schéma d'identification tel que produit initialement par l'application des identifiants aux échantillons.

**32.** Procédé selon la revendication 31, dans lequel le processus ou procédé est un processus ou procédé à haut débit.

**33.** Utilisation d'un procédé selon l'une des revendications précédentes pour au moins une utilisation choisie dans le groupe comprenant la détection d'un échange d'échantillons; la détection d'une contamination croisée; l'identification d'un échantillon dans un ensemble d'échantillons regroupés; la détection de l'inhibition d'une amplification; la détermination du rendement d'une conversion d'ADN; la normalisation d'un échantillon; l'étalonnage d'un échantillon; l'identification d'une contamination inter-échantillons; la maîtrise du succès d'une étape d'hybridation ou leurs combinaisons.

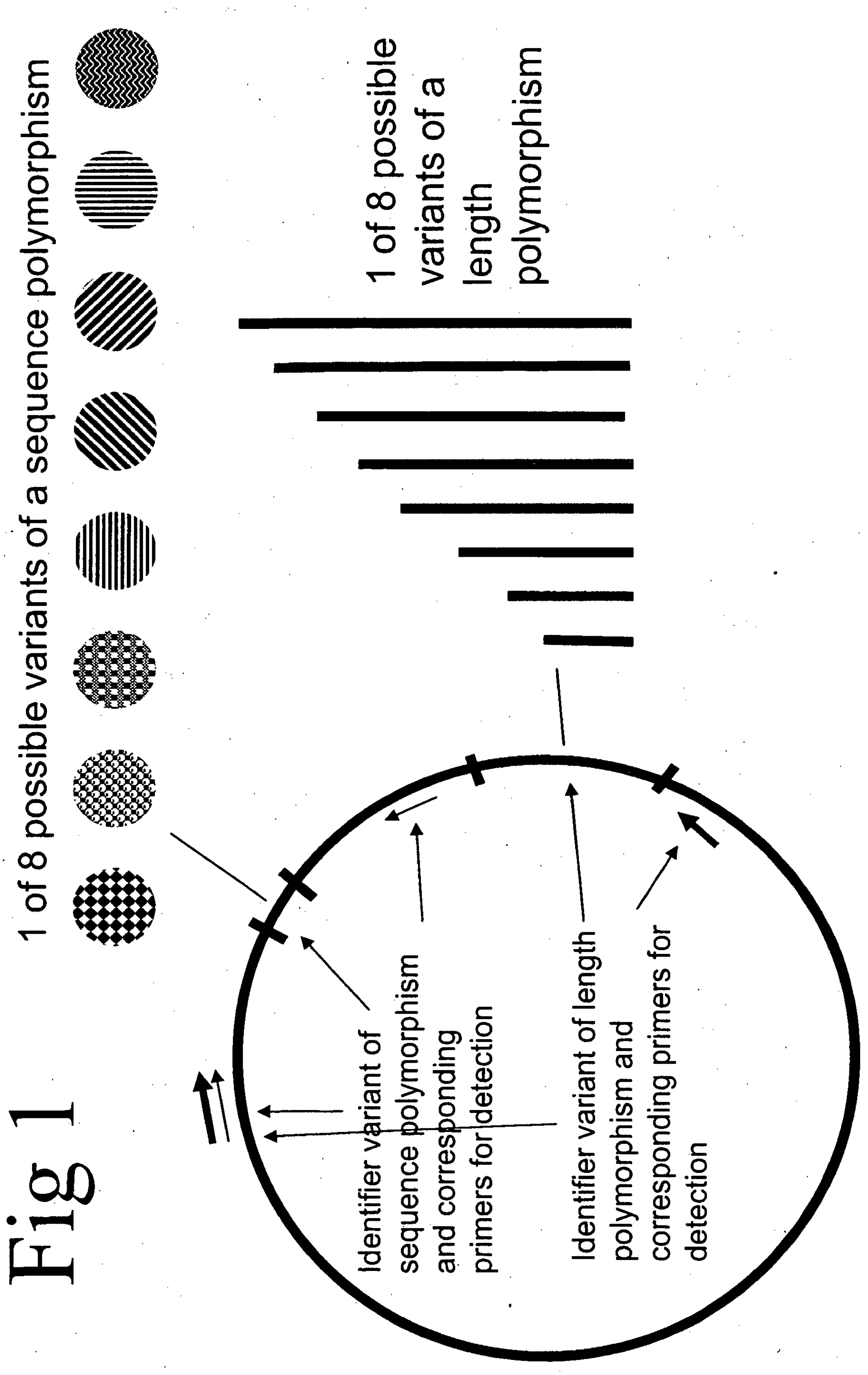
1 of 8 possible variants of a sequence polymorphism
1 of 8 possible variants of a length polymorphism
Identifier variant of sequence polymorphism and corresponding primers for detection
Identifier variant of length polymorphism and corresponding primers for detection

Fig 1

2/8

Fig 2

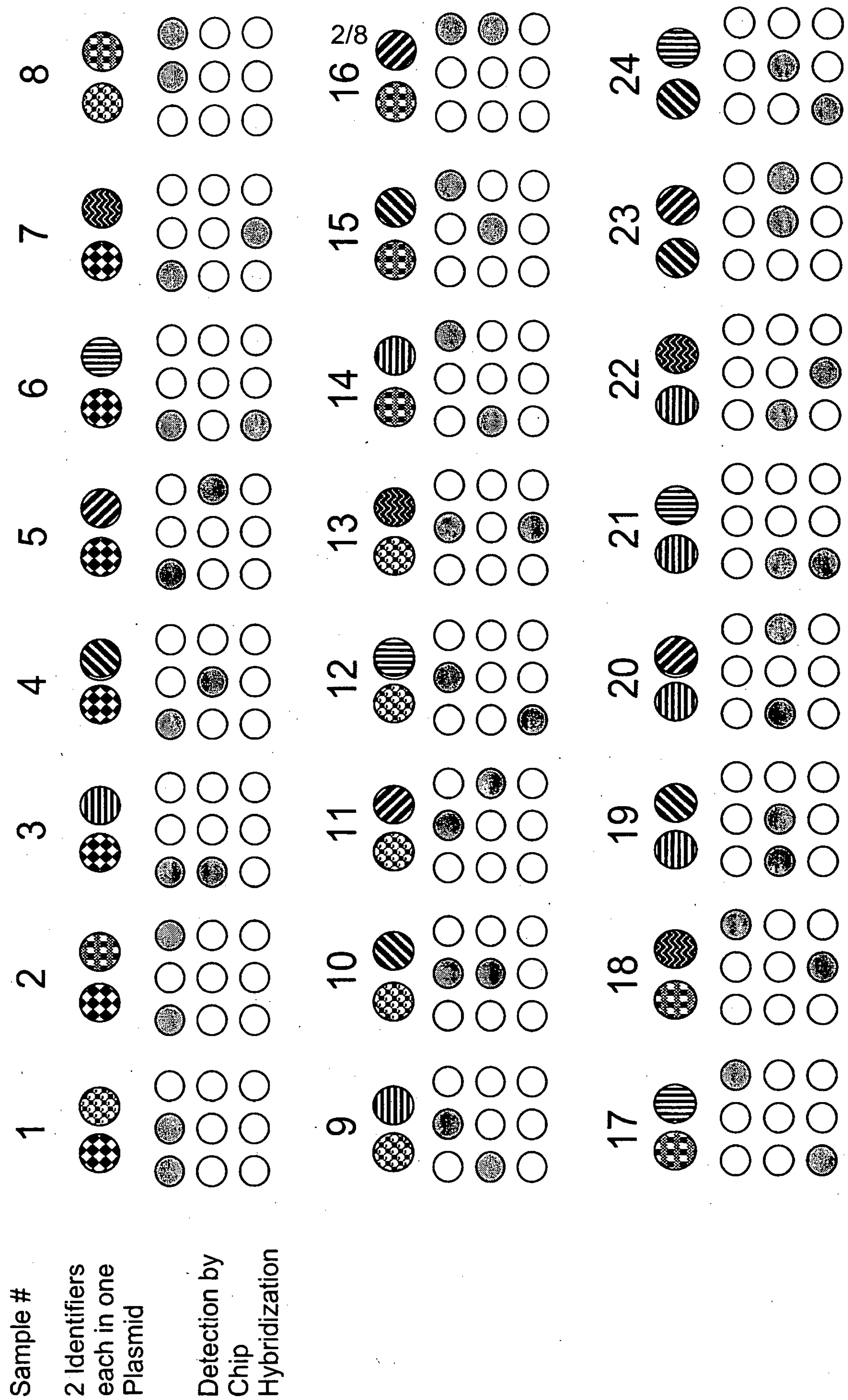
Sample #
2 Identifiers each in one Plasmid
Detection by Chip Hybridization
1
2
3
4
5
6
7
8
9
10
11
12
13
14
15
16
17
18
19
20
21
22
23
24

Fig 3

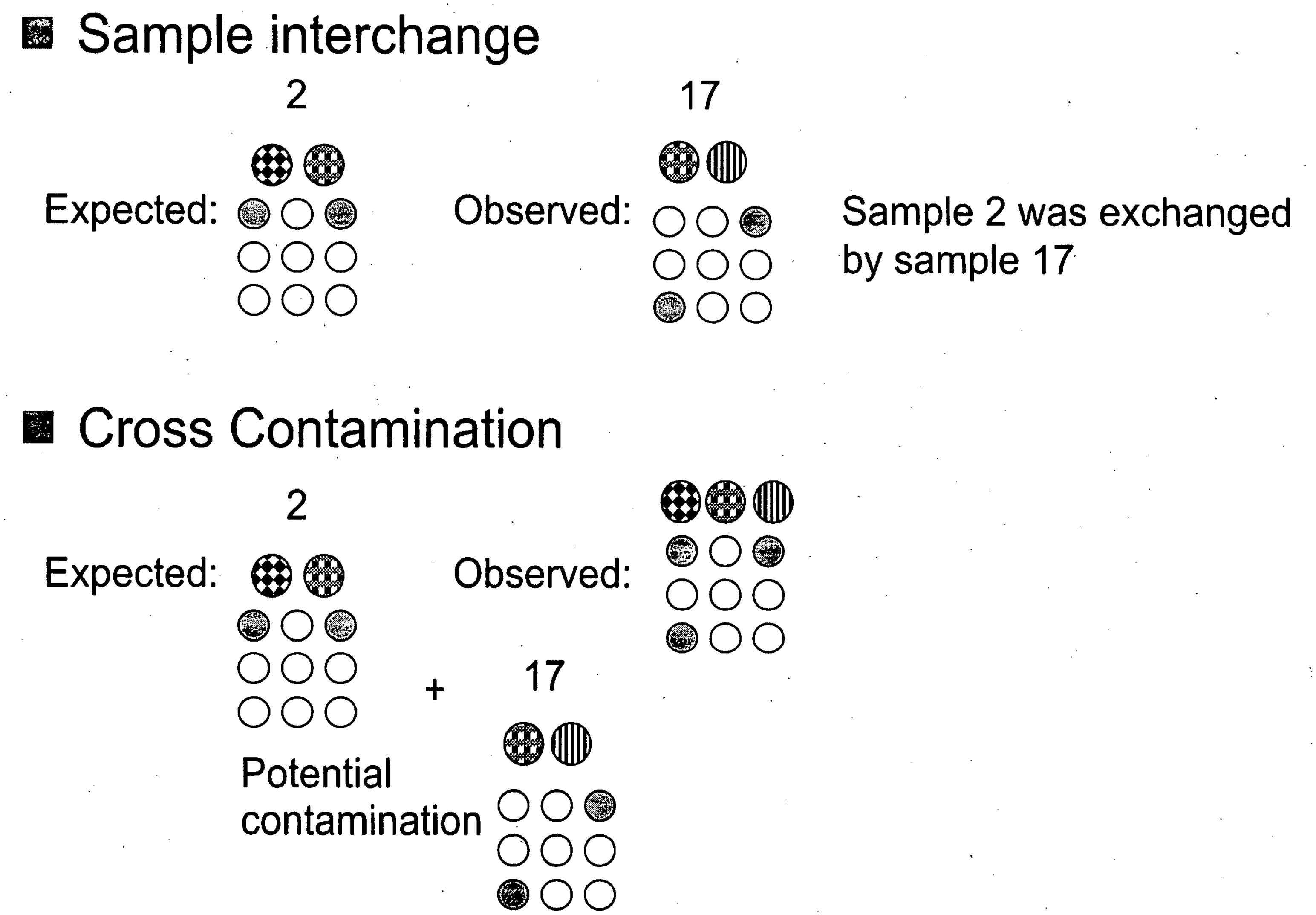
Sample interchange
2
17
Expected:
Observed:
Sample 2 was exchanged by sample 17
Cross Contamination
2
Expected:
Observed:
+
17
Potential contamination

Fig 4

A

| | | |
|---|---|---|
| 1 | 2 | 3 |
| 4 | 5 | 6 |
| 7 | 8 | 9 |

B

C

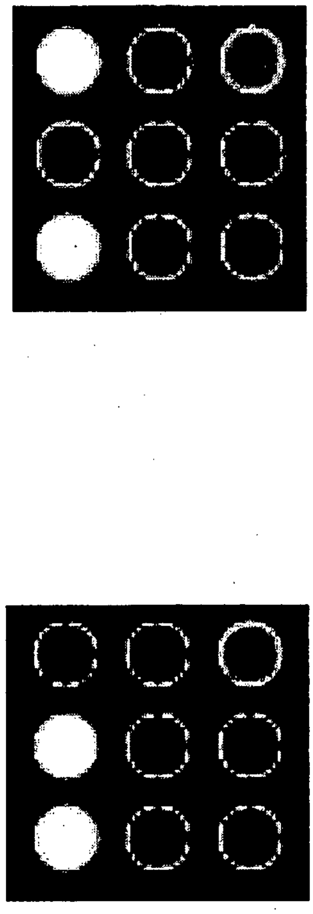

Fig 5

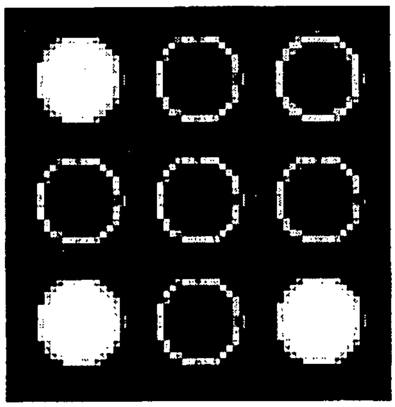

Fig 6

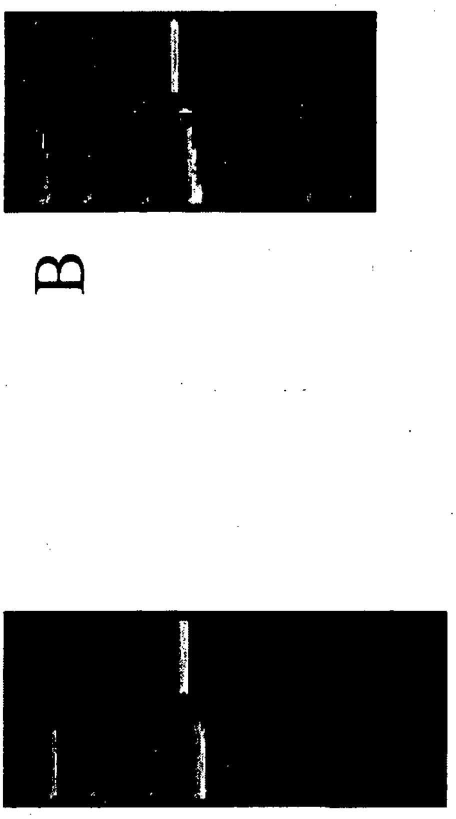

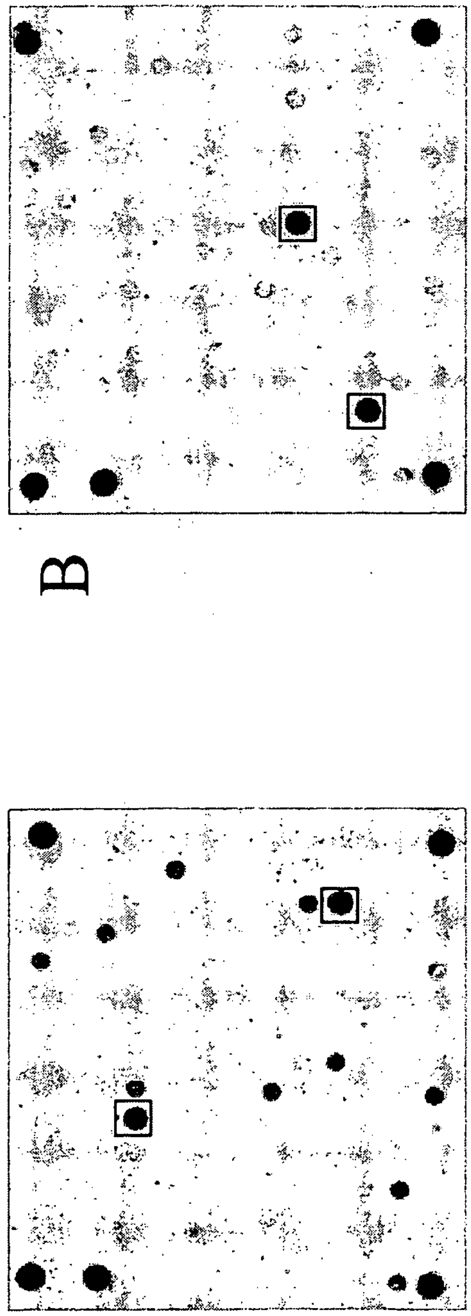


Fig 7

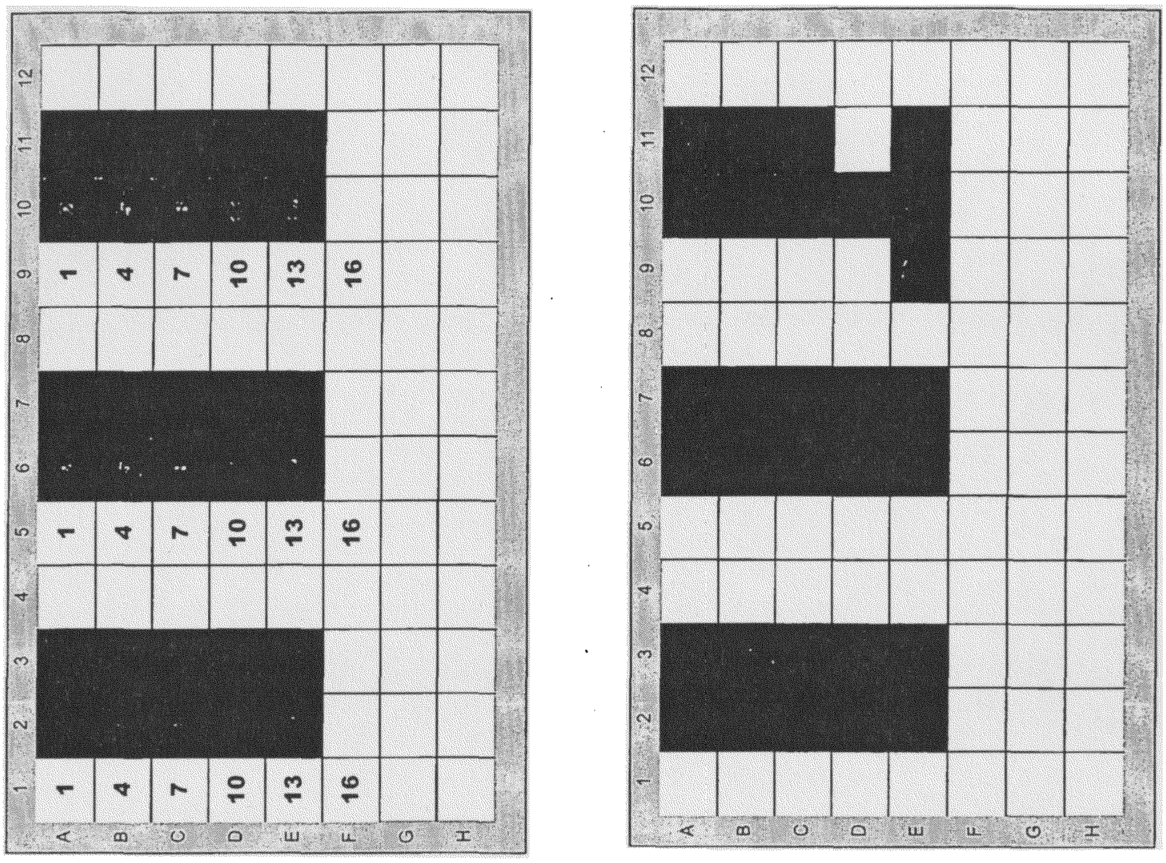

Fig 8

A

B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 994385 A
- US 6153389 A
- WO 2006113770 A
- WO 2006039563 A
- WO 05038051 A
- WO 2005038051 A
- WO 0198528 A
- DE 10029915
- US 10311661 B
- US 2006115835 A
- US 5786146 A
- WO 0218649 A
- US 20040038245 A
- WO 02072880 A
- EP 1369493 A
- WO 9928498 A
- WO 0138565 A
- WO 0218632 A
- EP 04090255 A
- US 6326145 B
- US 6365729 B
- US 20030087240 A1
- US 20030087240 A
- WO 05024056 A
- US 6251594 B
- WO 01062960 A
- WO 01062064 A
- WO 0162961 A


### Non-patent literature cited in the description


- **GARDINER-GARDEN, M. ; FROMMER, M.** *J. Mol. Biol.,* 1987, vol. 196, 261-282
- **LAIRD PW.** The power and the promise of DNA methylation markers. *Nat Rev Cancer,* April 2003, vol. 3 (4), 253-66
- **GRUNAU et al.** *Nucleic Acids Res.,* 2001, vol. 29 (13), 65-5
- **HERMAN et al.** *Proc. Natl. Acad. Sci. USA,* 1996, vol. 93, 9821-9826
- **COTTRELL SE et al.** *Nucleic Acids Res.,* 13 January 2004, vol. 32 (1), 10
- **FROMMER et al.** *Proc. Natl. Acad. Sci. USA,* 1992, vol. 89, 1827-1831
- Nature Genetics. January 1999, vol. 21
- **XIONG ; LAIRD.** *Nucleic Acids Res.,* 1997, vol. 25, 2532-2534
- **SADRI ; HOMSBY.** *Nucl. Acids Res.,* 1996, vol. 24, 5058-5059
- **EADS et al.** *Cancer Res.,* 1999, vol. 59, 2302-2306
- **TYAGI S. ; KRAMER F.R.** *Nature Biotechnology,* 1996, vol. 14, 303-308
- **WHITCOMBE et al.** *Nature and Biotechnology,* 1999, vol. 17, 804-807
- **RAND K.N. et al.** *Nucleic Acid Research,* vol. 33 (14), 127
- **WHITCOMBE et al.** Detection of PCR products using self-probing amplicons and fluorescence. *Nat Biotechnol.,* 1999, vol. 17 (8), 804-7
- **THELWELL et al.** Mode of action and application of Scorpion™ primers to mutation detection. *Nucleic Acids Res.,* 01 October 2000, vol. 28 (19), 3752-61
- **SOLINAS et al.** Duplex Scorpion™ primers in SNP analysis and FRET applications. *Nucleic Acids Res.,* 15 October 2001, vol. 29 (20), 96
- **GONZALGO et al.** *Nucleic Acids Research,* 1997, vol. 25 (12), 2529-2531

Sequence listing

<110> Epigenomics AG

<120> A METHOD OF IDENTIFYING A BIOLOGICAL SAMPLE FOR METHYLATION ANALYSIS

<160> 42

<210> 1
<211> 2309
<212> DNA
<213> Homo Sapiens

<400> 1

```
ctcttatccc tctcaccttc tcacttggca ccgttgcaga gagagaacca aacatggtaa      60
acaaagacga ccggattaga ccggttcatg aagccgacgg tgaaccgctt tttgagacta     120
ggagaagaac cggtagagtg attgcgtacc ggtttttctc agcctcggtt ttcgtgtgta     180
tctgtttgat ttggttctac agaattggtg agattggtga taaccgtacc gttttagatc     240
gattaatctg gtttgttatg tttattgtgg agatttggtt cggtttatat tgggtagtca     300
cacaatcttc ccggtggaat ccggtttggc gatttccctt ctccgataga ctctctcgga     360
gatacggaag cgaccttccg aggctcgacg tcttcgtttg cacggcggat ccggtgattg     420
agccgccgtt gttggtggta aacacagtct tatctgtgac ggctcttgac tacccaccgg     480
agaaactcgc cgtttatctc tcagatgacg gtggttctga gctgacgttc tatgctctca     540
cggaggcagc tgagtttgct aaaacttggg ttcccttctg caagaagttc aacgttgagc     600
caacatctcc cgctgcttac ttgtcttcca aggcaaactg tcttgattct gcggctgagg     660
aggtggctaa gctgtataga gaaatggcgg cgaggattga aacggcggcg agactgggac     720
gaataccgga ggaggcgcgg gtgaagtacg gtgacgggtt ttcacagtgg gatgctgacg     780
ctactcgaag aaaccatgga accattcttc aagttttggt agatggaaga gaagggaata     840
caatagcaat accaacgttg gtgtatttat caagagaaaa gagacctcaa catcatcata     900
acttcaaggc tggagcaatg aacgcattgc tgagggtttc ttcgaaaatt acttgtggga     960
aaatcatact aaacttggac tgtgatatgt acgcaaacaa ctcaaagtca acacgcgacg    1020
cgctctgcat cctcctcgat gagaaagagg gaaaagagat tgctttcgtg cagtttccgc    1080
agtgttttga caatgttaca agaaatgatt tgtatggaag catgatgcga gtaggaattg    1140
atgtggaatt tcttggattg gatggaaatg gtggtccgtt atacattgga actggatgct    1200
ttcacagaag agatgtgatc tgtggaagaa agtatggaga ggaagaagaa gaagaagaat    1260
ctgagagaat tcacgaaaat ttagagcctg agatgattaa ggctctcgcg agctgcactt    1320
atgaggaaaa cactcaatgg ggaaaggaga tgggtgtgaa atatggttgc ccggtagagg    1380
atgtaataac tggtttgacg attcagtgtc gcggatggaa atcagcctac ctgaacccgg    1440
aaaagcaagc atttctcggg gtagcgccga ccaatttgca tcaaatgcta gtgcagcaga    1500
ggagatggtc agagggagac tttcagatta tgctttcgaa gtatagtccg gtttggtatg    1560
gaaaaggaaa gatcagttta ggactgatac ttggttactg ttgctattgt ctttgggctc    1620
catcttcact acctgtgctc atttactctg ttttgacttc tctctgtctc ttcaaaggca    1680
ttcctctgtt tccaaaggtc tcgagctcgt ggtttattcc gtttggatac gtcactgttg    1740
cagctaccgc atatagccta gccgagttct tgtggtgcgg agggacgttc cgtggatggt    1800
ggaacgagca aaggatgtgg ctttatagaa gaacaagctc gtttcttttc ggatttatgg    1860
acacgattaa gaagctactt ggagtttctg agtctgcgtt tgtgatcaca gcaaaagtag    1920
cagaagaaga agcagcagag agatacaagg aagaggtaat ggagtttgga gtggagtctc    1980
ccatgtttct cgtcctcgga acactcggta tgctcaatct cttctgcttc gccgcagcgg    2040
ttgcgagact tgtttccgga gacggtggag atttgaaaac aatggggatg caatttgtga    2100
taacaggagt actagttgtc ataaactggc ctctgtataa aggtatgttg ttgaggcaag    2160
acaaaggaaa gatgccaatg agcgttacag ttaaatcagt tgttttagct ttatctgcct    2220
gtacctgttt agcgtttttg taagattgat taacaacagt caaaaaagta atcaaaataa    2280
tgaccagcag ttataatatg taattttct                                      2309
```

<210> 2
<211> 20
<212> DNA
<213> Homo Sapiens

<400> 2
ccgctgctta cttgtcttcc 20

<210> 3
<211> 20

<212> DNA
<213> Homo Sapiens

<400> 3
acagcttagc cacctcctca 20

<210> 4
<211> 19
<212> DNA
<213> Homo Sapiens

<400> 4
ctccggtatt cgtcccagt 19

<210> 5
<211> 20
<212> DNA
<213> Homo Sapiens

<400> 5
agcatcccac tgtgaaaacc 20

<210> 6
<211> 20
<212> DNA
<213> Homo Sapiens

<400> 6
atggttccat ggtttcttcg 20

<210> 7
<211> 22
<212> DNA
<213> Homo Sapiens

<400> 7
ttcccttctc ttccatctac ca 22

<210> 8
<211> 25
<212> DNA
<213> Homo Sapiens

<400> 8
ttttctcttg ataaatacac caacg 25

<210> 9
<211> 20
<212> DNA
<213> Homo Sapiens

<400> 9
cattgctcca gccttgaagt 20

<210> 10
<211> 25
<212> DNA
<213> Homo Sapiens

<400> 10
ccaagtttag tatgattttc ccaca 25

<210> 11
<211> 41
<212> DNA
<213> Homo Sapiens

<400> 11
gtgatgtgag ttaatgatgg gccgctgctt acttgtcttc c 41

<210> 12
<211> 78
<212> DNA
<213> Homo Sapiens

<400> 12

```
ccctaacctt aacatcttcc aagtactatt taaataacca tactatacca aaataatcac      60
agcttagcca cctcctca                                                    78
```

<210> 13
<211> 77
<212> DNA
<213> Homo Sapiens

<400> 13

```
aaccttactt taccatactc tagtactatt taaataacca tactatacca aaataatcct      60
ccggtattcg tcccagt                                                     77
```

<210> 14
<211> 77
<212> DNA
<213> Homo Sapiens

<400> 14

```
atataatcca ataaccccca agtactattt aaataaccat actataccaa aataatcagc      60
atcccactgt gaaaacc                                                     77
```

<210> 15
<211> 74
<212> DNA
<213> Homo Sapiens

<400> 15

```
cacaccaccc aaaaactagt actatttaaa taaccatact ataccaaaat aatcatggtt      60
ccatggtttc ttcg                                                        74
```

<210> 16
<211> 84
<212> DNA

<213> Homo Sapiens

<400> 16

```
cacaattaca catcccaata aacttagtac tatttaaata accatactat accaaaataa      60
tcttcccttc tcttccatct acca                                             84
```

<210> 17
<211> 83
<212> DNA
<213> Homo Sapiens

<400> 17

```
ccccacaatc aaacatacca tagtactatt taaataacca tactatacca aaataatctt      60
ttctcttgat aaatacacca acg                                              83
```

<210> 18
<211> 80
<212> DNA
<213> Homo Sapiens

<400> 18

```
tacaatccaa cttaaaacca ctcagtacta tttaaataac catactatac caaaataatc      60
cattgctcca gccttgaagt                                                  80
```

<210> 19
<211> 85
<212> DNA
<213> Homo Sapiens

<400> 19

```
ctcaactcaa taaaccttta cacagtacta tttaaataac catactatac caaaataatc      60
ccaagtttag tatgattttc ccaca                                            85
```

<210> 20
<211> 21
<212> DNA
<213> Homo Sapiens

<400> 20
gtgatgtgag ttaatgatgg g 21

<210> 21
<211> 23
<212> DNA
<213> Homo Sapiens

<400> 21
aaccatacta taccaaaata ate 23

<210> 22

<211> 24
<212> DNA
<213> Homo Sapiens

<400> 22
ggagagagaa gtagttgtgt aatt 24

<210> 23
<211> 24
<212> DNA
<213> Homo Sapiens

<400> 23
actacaccaa tacaaccaca tatc 24

<210> 24
<211> 20
<212> DNA
<213> Homo Sapiens

<400> 24
tgttttgatt ttgtggttga 20

<210> 25
<211> 21
<212> DNA
<213> Homo Sapiens

<400> 25
ccaacacatt aaaaactctc c 21

<210> 26
<211> 21
<212> DNA
<213> Homo Sapiens

<400> 26
tggaagatgt taaggttagg g 21

<210> 27
<211> 21
<212> DNA
<213> Homo Sapiens

<400> 27
agagtatggt aaagtaaggt t 21

<210> 28
<211> 20
<212> DNA
<213> Homo Sapiens

<400> 28
tgggggttat tggattatat 20

<210> 29
<211> 17
<212> DNA

<213> Homo Sapiens

<400> 29
agtttttggg tggtgtg 17

<210> 30
<211> 25
<212> DNA
<213> Homo Sapiens

<400> 30
aagtttattg ggatgtgtaa ttgtg 25

<210> 31
<211> 21
<212> DNA
<213> Homo Sapiens

<400> 31
atggtatgtt tgattgtggg g 21

<210> 32
<211> 23
<212> DNA
<213> Homo Sapiens

<400> 32
gagtggtttt aagttggatt gta 23

<210> 33
<211> 23
<212> DNA
<213> Homo Sapiens

<400> 33
gagtggtttt aagttggatt gta 23

<210> 34
<211> 23
<212> DNA
<213> Homo Sapiens

<400> 34
gtgtaaaggt ttattgagtt gag 23

<210> 35
<211> 19
<212> DNA
<213> Homo Sapiens

<400> 35
gatgggagag tgagtagga 19

<210> 36
<211> 43
<212> DNA
<213> Homo Sapiens

<400> 36
tggaagatgt taaggttagg gtcacttcta actctaccac tta 43

<210> 37
<211> 20
<212> DNA
<213> Homo Sapiens

<400> 37
tgatgggaga gtgagtagga 20

<210> 38
<211> 43
<212> DNA
<213> Homo Sapiens

<400> 38
agagtatggt aaagtaaggt ttcacttcta actctaccac tta 43

<210> 39
<211> 21
<212> DNA
<213> Homo Sapiens

<400> 39
ccctaacctt aacatcttcc a 21

<210> 40
<211> 21
<212> DNA
<213> Homo Sapiens

<400> 40
aaccttactt taccatactc t 21

<210> 41
<211> 23
<212> DNA
<213> Homo Sapiens

<400> 41
tagaaagttt acggtatttt aat 23

<210> 42
<211> 23
<212> DNA
<213> Homo Sapiens

<400> 42
tagaaagttt atggtatttt aat 23

<210> 43
<211> 29
<212> DNA
<213> Homo Sapiens

<400> 43
agtacttgta tttgaattgt tttttttga 29

<210> 44
<211> 29
<212> DNA
<213> Homo Sapiens

<400> 44
caaaaaaaac aattcaaata caagtacta 29

<210> 45
<211> 26
<212> DNA
<213> Homo Sapiens

<400> 45
agtactgtat ttggttggag tgggga 26

<210> 46
<211> 26
<212> DNA
<213> Homo Sapiens

<400> 46
ccccactcca accaaataca gtacta 26

<210> 47
<211> 20
<212> DNA
<213> Homo Sapiens

<400> 47
tgtggaattg tgagtggata 20

<210> 48
<211> 20
<212> DNA
<213> Homo Sapiens

<400> 48
acatactccc aaccaccata 20